# EUROPEAN PATENT APPLICATION

(11) **EP 1 281 966 A2**
(43) Date of publication of application: **05.02.2003**
(21) Application number: 02016498.4
(22) Date of filing: 23.07.2002
(51) Int. Cl.: G01N 33/543, G01N 33/566, C12Q 1/68, G01N 35/00, G01N 21/00, B01L 3/00, G01N 33/58

(54) **Method and apparatus for conducting a receptor-ligand reaction**

(30) Priority: 30.07.2001 JP 2001229058; 04.09.2001 JP 2001267154
(71) Applicant: FUJI PHOTO FILM CO., LTD., Kanagawa 250-01 (JP)
(72) Inventor: Ogura, Nobuhiko, Ashigarakami-gun, Kanagawa-ken 258 (JP); Muraishi, Katsuaki, Ashigarakami-gun, Kanagawa-ken 258 (JP); Etoh, Masahiro, Ashigarakami-gun, Kanagawa-ken 258 (JP)
(74) Representative: Klunker . Schmitt-Nilson . Hirsch

(57) **Abstract**

A method for conducting a receptor-ligand association reaction includes the step of feeding a reaction solution containing a ligand or a receptor labeled with a labeling substance to a plurality of absorptive regions which are formed in a biochemical analysis unit to be spaced from each other and in which receptors or ligands are fixed so as to cut through the plurality of absorptive regions, thereby selectively associating the ligand or the receptor contained in the reaction solution with the receptors or the ligands fixed in the plurality of absorptive regions of the biochemical analysis unit. According to the present invention, it is possible to efficiently associate a ligand or a receptor with receptors or ligands fixed in a biochemical analysis unit and produce biochemical analysis data having an excellent high quantitative characteristic with excellent repeatability.

## Description

### BACKGROUND OF THE INVENTION

The present invention relates to a method for conducting a receptor-ligand association reaction and an apparatus used therefor and, particularly, to a method for conducting a receptor-ligand association reaction and an apparatus used therefor which can efficiently associate a receptor or a ligand with ligands or receptors fixed in a biochemical analysis unit and produce biochemical analysis data having an excellent high quantitative characteristic with excellent repeatability.

### DESCRIPTION OF THE PRIOR ART

An autoradiographic analyzing system using as a detecting material for detecting radiation a stimulable phosphor which can absorb, store and record the energy of radiation when it is irradiated with radiation and which, when it is then stimulated by an electromagnetic wave having a specified wavelength, can release stimulated emission whose light amount corresponds to the amount of radiation with which it was irradiated is known, which comprises the steps of introducing a radioactively labeled substance into an organism, using the organism or a part of the tissue of the organism as a specimen, superposing the specimen and a stimulable phosphor sheet formed with a stimulable phosphor layer for a certain period of time, storing and recording radiation energy in a stimulable phosphor contained in the stimulable phosphor layer, scanning the stimulable phosphor layer with an electromagnetic wave to excite the stimulable phosphor, photoelectrically detecting the stimulated emission released from the stimulable phosphor to produce digital image signals, effecting image processing on the obtained digital image signals, and reproducing an image on displaying means such as a CRT or the like or a photographic film (see, for example, Japanese Patent Publication No. 1-60784, Japanese Patent Publication No. 1-60782, Japanese Patent Publication No. 4-3952 and the like).

There is further known chemiluminescence analysis system comprising the steps of employing, as a detecting material for light, a stimulable phosphor which can absorb and store the energy of light upon being irradiated therewith and release a stimulated emission whose amount is proportional to that of the received light upon being stimulated with an electromagnetic wave having a specific wavelength range, selectively labeling a fixed high molecular substance such as a protein or a nucleic acid sequence with a labeling substance which generates chemiluminescence emission when it contacts a chemiluminescent substance, contacting the high molecular substance selectively labeled with the labeling substance and the chemiluminescent substance, storing and recording the chemiluminescence emission in the wavelength of visible light generated by the contact of the chemiluminescent substance and the labeling substance in the stimulable phosphor contained in a stimulable phosphor layer formed on a stimulable phosphor sheet, scanning the stimulable phosphor layer with an electromagnetic wave to excite the stimulable phosphor, photoelectrically detecting the stimulated emission released from the stimulable phosphor to produce digital signals, effecting data processing on the obtained digital signals, and reproducing data on displaying means such as a CRT or a recording material such as a photographic film (see for example, U.S. Patent No. 5,028,793, UK Patent Application 2,246,197 A and the like).

Unlike the system using a photographic film, according to these systems using the stimulable phosphor as a detecting material, development, which is chemical processing, becomes unnecessary. Further, it is possible reproduce a desired image by effecting image processing on the obtained image data and effect quantitative analysis using a computer. Use of a stimulable phosphor in these processes is therefore advantageous.

On the other hand, a fluorescence analyzing system using a fluorescent substance as a labeling substance instead of a radioactive labeling substance in the autoradiographic analyzing system is known. According to this system, it is possible to study a genetic sequence, study the expression level of a gene, and to effect separation or identification of protein or estimation of the molecular weight or properties of protein or the like. For example, this system can perform a process including the steps of distributing a plurality of DNA fragments on a gel support by means of electrophoresis after a fluorescent dye was added to a solution containing a plurality of DNA fragments to be distributed, or distributing a plurality of DNA fragments on a gel support containing a fluorescent dye, or dipping a gel support on which a plurality of DNA fragments have been distributed by means of electrophoresis in a solution containing a fluorescent dye, thereby labeling the electrophoresed DNA fragments, exciting the fluorescent dye by a stimulating ray to cause it to release fluorescence emission, detecting the released fluorescence emission to produce an image and detecting the distribution of the DNA fragments on the gel support. This system can also perform a process including the steps of distributing a plurality of DNA fragments on a gel support by means of electrophoresis, denaturing the DNA fragments, transferring at least a part of the denatured DNA fragments onto a transfer support such as a nitrocellulose support by the Southern-blotting method, hybridizing a probe prepared by labeling target DNA and DNA or RNA complementary thereto with the denatured DNA fragments, thereby selectively labeling only the DNA fragments complementary to the probe DNA or probe RNA, exciting the fluorescent dye by a stimulating ray to cause it to release fluorescence emission, detecting the released fluorescence emission to produce an image and detecting the distribution of the target DNA on the transfer support. This system can further perform a process including the steps of preparing a DNA probe complementary to DNA containing a target gene labeled by a labeling substance, hybridizing it with DNA on a transfer support, combining an enzyme with the complementary DNA labeled by a labeling substance, causing the enzyme to contact a fluorescent substance, transforming the fluorescent substance to a fluorescent substance having fluorescence emission releasing property, exciting the thus produced fluorescent substance by a stimulating ray to release fluorescence emission, detecting the fluorescence emission to produce an image and detecting the distribution of the target DNA on the transfer support. This fluorescence detecting system is advantageous in that a genetic sequence or the like can be easily detected without using a radioactive substance.

Similarly, there is known a chemiluminescence detecting system comprising the steps of fixing a substance derived from a living organism such as a protein or a nucleic acid sequence on a support, selectively labeling the substance derived from a living organism with a labeling substance which generates chemiluminescence emission when it contacts a chemiluminescent substrate, contacting the substance derived from a living organism and selectively labeled with the labeling substance and the chemiluminescent substrate, photoelectrically detecting the chemiluminescence emission in the wavelength of visible light generated by the contact of the chemiluminescent substrate and the labeling substance to produce digital image signals, effecting image processing thereon, and reproducing a chemiluminescent image on a display means such as a CRT or a recording material such as a photographic film, thereby obtaining information relating to the high molecular substance such as genetic information

Further, a micro-array analyzing system has been recently developed, which comprises the steps of using a spotting device to drop at different positions on the surface of a carrier such as a slide glass plate, a membrane filter or the like specific binding substances, which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substances using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a labeling substance such as a fluorescent substance, dye or the like, thereby forming a micro-array, irradiating the micro-array with a stimulating ray, photoelectrically detecting light such as fluorescence emission released from a labeling substance such as a fluorescent substance, dye or the like, and analyzing the substance derived from a living organism. This micro-array analyzing system is advantageous in that a substance derived from a living organism can be analyzed in a short time period by forming a number of spots of specific binding substances at different positions of the surface of a carrier such as a slide glass plate at a high density and hybridizing them with a substance derived from a living organism and labeled with a labeling substance.

In addition, a macro-array analyzing system using a radioactive labeling substance as a labeling substance has been further developed, which comprises the steps of using a spotting device to drop at different positions on the surface of a carrier such as a membrane filter or the like specific binding substances, which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substance using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a radioactive labeling substance, thereby forming a macro-array, superposing the macro-array and a stimulable phosphor sheet formed with a stimulable phosphor layer, exposing the stimulable phosphor layer to a radioactive labeling substance, irradiating the stimulable phosphor layer with a stimulating ray to excite the stimulable phosphor, photoelectrically detecting the stimulated emission released from the stimulable phosphor to produce biochemical analysis data, and analyzing the substance derived from a living organism.

However, in the micro-array analyzing system and the macro-array analyzing system, it is required to produce biochemical analysis data by dropping a solution containing specific binding substances at different positions on the surface of a biochemical analysis unit such as a membrane filter or the like to form a number of spot-like regions, hybridizing a substance derived from a living organism and labeled with a labeling substance such as a radioactive labeling substance, a fluorescent substance and a labeling substance which generates chemiluminescence emission when it contacts a chemiluminescent substrate with the specific binding substances contained in the spot-like regions, thereby selectively labeling the spot-like regions, exposing a stimulable phosphor layer of a stimulable phosphor sheet to a radioactive labeling substance selectively contained in the spot-like regions, scanning the thus exposed stimulable phosphor layer with a stimulating ray, thereby exciting stimulable phosphor contained in the stimulable phosphor layer and photoelectrically detecting stimulated emission released from the stimulable phosphor, or scanning a number of the spot-like regions with a stimulating ray, thereby exciting a fluorescent substance contained in a number of the spot-like regions and photoelectrically detecting fluorescence emission released from the fluorescent substance, or bringing a labeling substance contained in a number of the spot-like regions into contact with a chemiluminescent substrate and photoelectrically detecting chemiluminescence emission released from the labeling substance.

Conventionally, hybridization of specific binding substances and a substance derived from a living organism was performed by an experimenter manually inserting a biochemical analysis unit formed with a number of the spot-like regions containing specific binding substances such as a membrane filter into a hybridization bag, pouring a hybridization solution containing a substance derived from a living organism and labeled with a labeling substance such as a radioactive labeling substance, a fluorescent substance or a labeling substance which generates chemiluminescence emission when it contacts a chemiluminescent substrate into the hybridization bag, vibrating the hybridization bag, thereby moving the substance derived from a living organism by convection or diffusion, hybridizing the substance derived from a living organism with the specific binding substances, removing the biochemical analysis unit from the hybridization bag, and inserting the biochemical analysis unit in a container filled with a cleaning solution, thereby cleaning the biochemical analysis unit.

However, in the case where specific binding substances and a substance derived from a living organism are hybridized by an experimenter manually inserting a biochemical analysis unit into a hybridization bag, pouring a hybridization solution into the hybridization bag, and vibrating the hybridization bag, it is difficult to bring the hybridization solution into uniform contact with a number of the spot-like regions containing specific binding substances and, therefore, specific binding substances and a substance derived from a living organism cannot be effectively hybridized.

Further, in the case of an experimenter manually inserting a biochemical analysis unit into a hybridization bag by, pouring a hybridization solution into the hybridization bag, vibrating the hybridization bag, hybridizing specific binding substances and a substance derived from a living organism, removing the biochemical analysis unit from the hybridization bag, and inserting the biochemical analysis unit in a container filled with a cleaning solution, thereby cleaning the biochemical analysis unit, the results of the hybridization differ among different experimenters and the repeatability of the hybridization is inevitably lowered. Moreover, even when the same experimenter performs hybridization, different results may be obtained.

Furthermore, a substance derived from a living organism should not be bonded with specific binding substances by hybridization may be bonded with the specific binding substances. In such cases, when biochemical analysis data are produced by bringing a biochemical analysis unit such as a membrane filter into close contact with a stimulable phosphor sheet formed with a stimulable phosphor layer containing stimulable phosphor, thereby exposing the stimulable phosphor layer, irradiating the stimulable phosphor layer with a stimulating ray and photoelectrically detecting stimulated emission released from the stimulable phosphor layer, or irradiating the biochemical analysis unit such as a membrane filter with a stimulating ray and photoelectrically detecting fluorescence emission released from a fluorescent substance, or photoelectrically detecting chemiluminescence emission released from a biochemical analysis unit such as a membrane filter, noise is generated in the biochemical analysis data and quantitative accuracy of quantitative analysis is lowered.

In the case where a receptor and a ligand are associated as in the case of fixing antigens or antibodies in a biochemical analysis unit such as a membrane filter and binding an antibody or an antigen to the thus fixed antigens or antibodies by an antigen-antibody reaction, the same problems occur, and in the case of hybridizing a probe DNA labeled with a hapten such as digoxigenin with a target DNA fixed in a biochemical analysis unit such as a membrane filter, binding an antibody for the hapten such as digoxigenin labeled with an enzyme which generates chemiluminescent emission when it contacts a chemiluminescent substrate or an antibody for the hapten such as digoxigenin labeled with an enzyme which generates fluorescence emission when it contacts a fluorescent substrate with the hapten labeling the probe DNA by an an antigen-antibody reaction, thereby labeling the target DNA, the same problems also occur.

### SUMMARY OF THE INVENTION

It is therefore an object of the present invention to provide a method for conducting a receptor-ligand association reaction and an apparatus used therefor which can efficiently associate a ligand or a receptor with receptors or ligands fixed in a biochemical analysis unit and produce biochemical analysis data having an excellent high quantitative characteristic with excellent repeatability.

The above other objects of the present invention can be accomplished by a method for conducting a receptor-ligand association reaction comprising the step of feeding a reaction solution containing a ligand or a receptor labeled with a labeling substance to a plurality of absorptive regions which are formed in a biochemical analysis unit to be spaced from each other and in which receptors or ligands are fixed so as to cut through the plurality of absorptive regions, thereby selectively associating the ligand or the receptor contained in the reaction solution with the receptors or the ligands fixed in the plurality of absorptive regions of the biochemical analysis unit.

In the present invention, the receptor-ligand association reaction includes a hybridization reaction and an antigen-antibody reaction.

According to the present invention, since a reaction solution containing a ligand or a receptor labeled with a labeling substance is fed to a plurality of absorptive regions which are formed in a biochemical analysis unit to be spaced from each other and in which receptors or ligands are fixed so as to cut through each of the plurality of absorptive regions, thereby selectively associating the ligand or the receptor contained in the reaction solution with the receptors or the ligands fixed in the plurality of absorptive regions of the biochemical analysis unit, it is possible to markedly increase the moving rate of the ligand or the receptor through the plurality of absorptive regions and therefore, to markedly increase the reaction rate of association of the receptors or the ligands fixed in the plurality of absorptive regions of the biochemical analysis unit and the ligand or the receptor contained in the reaction solution. It is further possible to markedly increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner.

Furthermore, according to the present invention, since a reaction solution containing a ligand or a receptor is fed to the plurality of absorptive regions which are formed in a biochemical analysis unit and in which receptors or ligands are fixed so as to cut through the plurality of absorptive regions, thereby selectively associating the ligand or the receptor contained in the reaction solution with the receptors or the ligands fixed in the plurality of absorptive regions of the biochemical analysis unit, the repeatability of the receptor-ligand association reaction can be markedly improved.

In a preferred aspect of the present invention, the reaction solution is fed to the plurality of absorptive regions of the biochemical analysis unit in both an obverse direction and a reverse direction so as to cut through the plurality of absorptive regions.

According to this preferred aspect of the present invention, since the reaction solution is fed to the plurality of absorptive regions of the biochemical analysis unit in both an obverse direction and a reverse direction so as to cut through the plurality of absorptive regions, it is possible to much more increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner and the repeatability of the receptor-ligand association reaction can be markedly improved.

In a preferred aspect of the present invention, the method for conducting a receptor-ligand association reaction comprises the steps of accommodating the biochemical analysis unit in a reaction vessel and feeding the reaction solution into the reaction vessel.

In a preferred aspect of the present invention, the method for conducting a receptor-ligand association reaction comprises the step of circulating the reaction solution into the reaction vessel accommodating the biochemical analysis unit, thereby passing the reaction solution through the plurality of absorptive regions of the biochemical analysis unit.

According to this preferred aspect of the present invention, since the reaction solution is circulated into the reaction vessel accommodating the biochemical analysis unit, thereby being passed through the plurality of absorptive regions of the biochemical analysis unit, it is possible to much more increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner and the repeatability of the receptor-ligand association reaction can be markedly improved.

In a preferred aspect of the present invention, the method for conducting a receptor-ligand association reaction comprises the steps of accommodating the biochemical analysis unit in a reaction vessel and generating ultrasound in a reaction solution containing a ligand or a receptor and accommodated in the reaction vessel in a direction cutting through the plurality of absorptive regions of the biochemical analysis unit, thereby forcibly passing the reaction solution so as to cut through the plurality of absorptive regions of the biochemical analysis unit.

According to this preferred aspect of the present invention, since the biochemical analysis unit is accommodated in a reaction vessel and ultrasound is generated in a reaction solution containing a ligand or a receptor and accommodated in the reaction vessel in a direction cutting through each of the plurality of absorptive regions of the biochemical analysis unit, thereby forcibly passing the reaction solution so as to cut through the plurality of absorptive regions of the biochemical analysis unit, it is possible to markedly increase the moving rate of the ligand or the receptor through the plurality of absorptive regions and therefore, to markedly increase the reaction rate of association of the receptors or the ligands fixed in the plurality of absorptive regions of the biochemical analysis unit and the ligand or the receptor contained in the reaction solution. It is further possible to markedly increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner.

In a further preferred aspect of the present invention, the method for conducting a receptor-ligand association reaction comprises the steps of alternately generating ultrasound in the reaction solution in an obverse direction and a reverse direction cutting through the plurality of absorptive regions of the biochemical analysis unit accommodated in the reaction vessel, thereby forcibly passing the reaction solution through the plurality of absorptive regions of the biochemical analysis unit in both the obverse direction and the reverse direction.

According to this preferred aspect of the present invention, since ultrasound is alternately generated in the reaction solution in an obverse direction and a reverse direction cutting through each of the plurality of absorptive regions of the biochemical analysis unit accommodated in the reaction vessel, thereby forcibly passing the reaction solution through the plurality of absorptive regions of the biochemical analysis unit in both the obverse direction and the reverse direction, it is possible to much more increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner and the repeatability of the receptor-ligand association reaction can be markedly improved.

In a preferred aspect of the present invention, the method for conducting a receptor-ligand association reaction comprises the step of feeding the reaction solutions whose temperature is controlled for each group consisting at least two of the absorptive regions among the plurality of absorptive regions of the biochemical analysis unit to the plurality of absorptive regions of the biochemical analysis unit, thereby selectively associating the ligand or the receptor contained in the reaction solution with the receptors or the ligands fixed in the plurality of absorptive regions of the biochemical analysis unit.

According to this preferred aspect of the present invention, since the method for conducting a receptor-ligand association reaction comprises the step of feeding the reaction solutions whose temperature is controlled for each group consisting at least two of the absorptive regions among the plurality of absorptive regions of the biochemical analysis unit to the plurality of absorptive regions of the biochemical analysis unit, thereby selectively associating the ligand or the receptor contained in the reaction solution with the receptors or the ligands fixed in the plurality of absorptive regions of the biochemical analysis unit, if the same kind of receptor or ligand is fixed in the absorptive regions constituting each of the groups, it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a preferred aspect of the present invention, the plurality of absorptive regions of the biochemical analysis unit are divided into a plurality of blocks each including two or more absorptive regions and the method for conducting a receptor-ligand association reaction comprises the step of feeding the reaction solution whose temperature is controlled for each of the blocks to the plurality of absorptive regions of the biochemical analysis unit, thereby selectively associating the ligand or the receptor contained in the reaction solution with the receptors or the ligands fixed in the plurality of absorptive regions of the biochemical analysis unit.

According to this preferred aspect of the present invention, since the plurality of absorptive regions of the biochemical analysis unit are divided into a plurality of blocks each including two or more absorptive regions and the reaction solution whose temperature is controlled for each of the blocks is fed to the plurality of absorptive regions of the biochemical analysis unit, thereby selectively associating the ligand or the receptor contained in the reaction solution with the receptors or the ligands fixed in the plurality of absorptive regions of the biochemical analysis unit, if the same kind of receptor or ligand is fixed in the absorptive regions constituting each of the blocks, it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the step of feeding the reaction solution whose temperature is controlled for each of the plurality of absorptive regions to the plurality of absorptive regions of the biochemical analysis unit, thereby selectively associating the ligand or the receptor contained in the reaction solution with the receptors or the ligands fixed in the plurality of absorptive regions of the biochemical analysis unit.

According to this preferred aspect of the present invention, since the reaction solution whose temperature is controlled for each of the plurality of absorptive regions is fed to the plurality of absorptive regions of the biochemical analysis unit, thereby selectively associating the ligand or the receptor contained in the reaction solution with the receptors or the ligands fixed in the plurality of absorptive regions of the biochemical analysis unit, it is possible to effectively prevent the ligand or the receptor associated with the receptors or the ligands fixed in the individual absorptive regions of the biochemical analysis unit, which is to be associated therewith, from being peeled off from the receptors or the ligands fixed in the individual absorptive regions of the biochemical analysis unit. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the temperature of the reaction solution is controlled in accordance with the kinds of the receptors or the ligands fixed in the plurality of absorptive regions of the biochemical analysis unit.

In a preferred aspect of the present invention, the method for conducting a receptor-ligand association reaction further comprises the step of feeding a cleaning solution to the plurality of absorptive regions of the biochemical analysis unit in which the receptors or the ligands selectively associated with the ligand or the receptor contained in the reaction solution are fixed so as to pass through the plurality of absorptive regions of the biochemical analysis unit, thereby cleaning the plurality of absorptive regions of the biochemical analysis unit.

According to this preferred aspect of the present invention, since a cleaning solution is further fed to the plurality of absorptive regions of the biochemical analysis unit in which the receptors or the ligands selectively associated with the ligand or the receptor contained in the reaction solution are fixed so as to pass through the plurality of absorptive regions of the biochemical analysis unit, thereby cleaning the plurality of absorptive regions of the biochemical analysis unit, even in the case where a ligand or a receptor which should no be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the cleaning solution is fed into the reaction vessel so as to pass through the plurality of absorptive regions of the biochemical analysis unit accommodated in the reaction vessel in both an obverse direction and a reverse direction.

According to this preferred aspect of the present invention, since the cleaning solution is fed into the reaction vessel so as to cut through the plurality of absorptive regions of the biochemical analysis unit accommodated in the reaction vessel in both an obverse direction and a reverse direction, even in the case where a ligand or the receptor which should not be associated with the receptors or the ligands fixed in the plurality of absorptive regions of the biochemical analysis unit has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the step of circulating the cleaning solution into the cartridge accommodating the biochemical analysis unit, thereby passing the cleaning solution through the plurality of absorptive regions of the biochemical analysis unit.

According to this preferred aspect of the present invention, since the cleaning solution is circulated into the cartridge accommodating the biochemical analysis unit, thereby passing the cleaning solution through the plurality of absorptive regions of the biochemical analysis unit, even in the case where a ligand or the receptor which should not be associated with the receptors or the ligands fixed in the plurality of absorptive regions of the biochemical analysis unit has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the steps of accommodating a cleaning solution in the reaction vessel and generating ultrasound in the cleaning solution in a direction cutting through the plurality of absorptive regions of the biochemical analysis unit in which the receptors or the ligands selectively associated with the ligand or the receptor contained in the reaction solution are fixed, thereby forcibly passing the cleaning solution so as to pass through the plurality of absorptive regions of the biochemical analysis unit and cleaning the plurality of absorptive regions of the biochemical analysis unit.

According to this preferred aspect of the present invention, since a cleaning solution is accommodated in the reaction vessel and ultrasound is generated in the cleaning solution in a direction cutting through each the plurality of absorptive regions of the biochemical analysis unit in which the receptors or the ligands selectively associated with the ligand or the receptor contained in the reaction solution are fixed, thereby forcibly passing the cleaning solution so as to pass through the plurality of absorptive regions of the biochemical analysis unit and cleaning the plurality of absorptive regions of the biochemical analysis unit, even in the case where a ligand or the receptor which should not be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the steps of alternately generating ultrasound in the cleaning solution in an obverse direction and a reverse direction cutting through the plurality of absorptive regions of the biochemical analysis unit accommodated in the reaction vessel, thereby forcibly passing the cleaning solution through the plurality of absorptive regions of the biochemical analysis unit in both the obverse direction and the reverse direction and cleaning the plurality of absorptive regions of the biochemical analysis unit.

According to this preferred aspect of the present invention, since ultrasound is alternately generated in the cleaning solution in an obverse direction and a reverse direction cutting through the plurality of absorptive regions of the biochemical analysis unit accommodated in the reaction vessel, thereby forcibly passing the cleaning solution through the plurality of absorptive regions of the biochemical analysis unit in both the obverse direction and the reverse direction and cleaning the plurality of absorptive regions of the biochemical analysis unit, even in the case where a ligand or the receptor which should not be associated with the receptors or the ligands fixed in the plurality of absorptive regions of the biochemical analysis unit has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the step of feeding the cleaning solutions whose temperature is controlled for each group consisting at least two of the absorptive regions among the plurality of absorptive regions of the biochemical analysis unit to the plurality of absorptive regions of the biochemical analysis unit, thereby cleaning the plurality of absorptive regions of the biochemical analysis unit.

According to this preferred aspect of the present invention, since the cleaning solutions whose temperature is controlled for each group consisting at least two of the absorptive regions among the plurality of absorptive regions of the biochemical analysis unit is fed to the plurality of absorptive regions of the biochemical analysis unit, thereby cleaning the plurality of absorptive regions of the biochemical analysis unit, if the same kind of receptor or ligand is fixed in the absorptive regions constituting each of the groups, even in the case where a ligand or the receptor which should not be associated with the receptors or the ligands fixed in the absorptive regions has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the step of feeding the cleaning solution whose temperature is controlled for each of the blocks to the plurality of absorptive regions of the biochemical analysis unit, thereby cleaning the plurality of absorptive regions of the biochemical analysis unit.

According to this preferred aspect of the present invention, since the cleaning solution whose temperature is controlled for each of the blocks is fed to the plurality of absorptive regions of the biochemical analysis unit, thereby cleaning the plurality of absorptive regions of the biochemical analysis unit, even in the case where a ligand or the receptor which should not be associated with the receptors or the ligands fixed in the absorptive regions constituting each of the blocks has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the method for conducting a receptor-ligand association reaction comprises the step of feeding the cleaning solution whose temperature is controlled for each of the plurality of absorptive regions to the plurality of absorptive regions of the biochemical analysis unit, thereby cleaning the plurality of absorptive regions of the biochemical analysis unit.

According to this preferred aspect of the present invention, since the cleaning solution whose temperature is controlled for each of the plurality of absorptive regions is fed to the plurality of absorptive regions of the biochemical analysis unit, thereby cleaning the plurality of absorptive regions of the biochemical analysis unit, even in the case where a ligand or the receptor which should not be associated with the receptors or the ligands fixed in the individual absorptive regions constituting each of the blocks has been bonded therewith in the course of the receptor-ligand association reaction, it is possible to more effectively peel off and remove the ligand or the receptor which should not be associated with the receptors or the ligands fixed in the plurality of absorptive regions of the biochemical analysis unit from the plurality of absorptive regions by cleaning the plurality of absorptive regions with the cleaning solution having an optimum temperature. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the reaction solution and/or the cleaning solution is fed to the plurality of absorptive regions of the biochemical analysis unit by independent conduits.

According to this preferred aspect of the present invention, since the reaction solution and/or the cleaning solution is fed to the plurality of absorptive regions of the biochemical analysis unit by independent conduits, it is possible to control the temperature of the reaction solution and/or the cleaning solution in a desired manner in accordance with the kinds of the receptors or the ligands fixed in the plurality of absorptive regions of the biochemical analysis unit.

In a preferred aspect of the present invention, the reaction solution contains a ligand or a receptor labeled with at least one labeling substance selected from a group consisting of a radioactive labeling substance, a fluorescent substance and a labeling substance which generates chemiluminescence emission when it contacts a chemiluminescent substrate.

In a preferred aspect of the present invention, specific binding substances whose structure or characteristics are known are fixed in the plurality of absorptive regions of the biochemical analysis unit and the method for conducting a receptor-ligand association reaction comprises the step of feeding the reaction solution containing a substance derived from a living organism and labeled with a labeling substance to the plurality of absorptive regions of the biochemical analysis unit so as to cut through them, thereby selectively hybridizing the substance derived from a living organism, labeled with a labeling substance and contained in the reaction solution with the specific binding substances fixed in the plurality of absorptive regions of the biochemical analysis unit.

In a preferred aspect of the present invention, antigens or antibodies are fixed in the plurality of absorptive regions of the biochemical analysis unit and the method for conducting a receptor-ligand association reaction comprises the step of feeding the reaction solution containing an antibody or an antigen labeled with a labeling substance to the plurality of absorptive regions of the biochemical analysis unit so as to cut through them, thereby selectively binding the an antibody or the antigen labeled with the labeling substance with the antigens or the antibodies fixed in the plurality of absorptive regions of the biochemical analysis unit.

In a preferred aspect of the present invention, specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a labeling enzyme to the plurality of absorptive regions of the biochemical analysis unit so as to cut through them, thereby binding the antibody labeled with the labeling enzyme with the hapten fixed in the plurality of absorptive regions of the biochemical analysis unit by an antigen-antibody reaction.

In a further preferred aspect of the present invention, specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a enzyme which generates chemiluminescence emission when it contacts a chemiluminescent substrate to the plurality of absorptive regions of the biochemical analysis unit so as to cut through them, thereby binding the antibody labeled with the enzyme with the hapten fixed in the plurality of absorptive regions of the biochemical analysis unit by an antigen-antibody reaction.

In another further preferred aspect of the present invention, specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a enzyme which generates a fluorescence substance when it contacts a fluorescent substrate to the plurality of absorptive regions of the biochemical analysis unit so as to cut through them, thereby binding the antibody labeled with the enzyme with the hapten fixed in the plurality of absorptive regions of the biochemical analysis unit by an antigen-antibody reaction.

In the present invention, illustrative examples of the combination of hapten and antibody include digoxigenin and anti-digoxigenin antibody, theophylline and anti-theophylline antibody, fluorosein and anti-fluorosein antibody, and the like. Further, the combination of biotin and avidin, antigen and antibody may be utilized instead of the combination of hapten and antibody.

In a preferred aspect of the present invention, the biochemical analysis unit includes an absorptive substrate formed of an absorptive material and the plurality of absorptive regions of the biochemical analysis unit are formed by absorbing a receptor or a ligand in regions spaced apart from each other of the absorptive substrate.

In a preferred aspect of the present invention, the biochemical analysis unit includes a substrate formed of a plurality of through-holes to be spaced apart from each other and the plurality of absorptive regions of the biochemical analysis unit are formed by charging an absorptive material in the plurality of through-holes formed in the substrate.

According to this preferred aspect of the present invention, since the biochemical analysis unit includes a substrate formed of a plurality of through-holes to be spaced apart from each other and the plurality of absorptive regions of the biochemical analysis unit are formed by charging an absorptive material in the plurality of through-holes formed in the substrate, the reaction solution and/or the cleaning solution fed to a particular absorptive region can be reliably prevented from penetrating the substrate from the absorptive region to neighboring absorptive regions and can be reliably fed to desired absorptive regions.

In a further preferred aspect of the present invention, the plurality of absorptive regions of the biochemical analysis unit are formed by embedding an absorptive material in the plurality of through-holes formed in the substrate.

In a further preferred aspect of the present invention, the plurality of absorptive regions of the biochemical analysis unit are formed by pressing an absorptive membrane containing an absorptive material in the plurality of through-holes formed in the substrate.

According to this preferred aspect of the present invention. since the plurality of absorptive regions of the biochemical analysis unit can be formed merely by pressing an absorptive membrane containing an absorptive material in the plurality of through-holes formed in the substrate, the biochemical analysis unit can be formed in a simple manner.

In another preferred aspect of the present invention. the biochemical analysis unit includes an absorptive substrate formed of an absorptive material and a pair of substrates formed with a plurality of through-holes in the same pattern and the pair of substrates are in close contact with opposite surfaces of the absorptive substrate, thereby forming the plurality of absorptive regions of the absorptive substrate within the plurality of through-holes of the pair of substrates.

According to this preferred aspect of the present invention. since the biochemical analysis unit includes an absorptive substrate formed of an absorptive material and a pair of substrates formed with a plurality of through-holes in the same pattern and the pair of substrates are in close contact with opposite surfaces of the absorptive substrate, thereby forming the plurality of absorptive regions of the absorptive substrate within the plurality of through-holes of the pair of substrates, the reaction solution can be fed solely to the plurality of absorptive regions in which the receptor or the ligand is fixed so as to pass therethrough and therefore, the efficiency of a receptor-ligand association reaction can be markedly improved. Further, since the cleaning solution can be fed solely to the plurality of absorptive regions in which the receptors or the ligands selectively associated with the ligand or the receptor contained in the reaction solution are fixed so as to pass therethrough, the cleaning efficiency can be markedly improved.

In a preferred aspect of the present invention, the substrate of the biochemical analysis unit is formed with 10 or more absorptive regions.

In a further preferred aspect of the present invention, the substrate of the biochemical analysis unit is formed with 50 or more absorptive regions.

In a further preferred aspect of the present invention, the substrate of the biochemical analysis unit is formed with 100 or more absorptive regions.

In a further preferred aspect of the present invention, the substrate of the biochemical analysis unit is formed with 500 or more absorptive regions.

In a further preferred aspect of the present invention, the substrate of the biochemical analysis unit is formed with 1,000 or more absorptive regions.

In a further preferred aspect of the present invention, the substrate of the biochemical analysis unit is formed with 5,000 or more absorptive regions.

In a further preferred aspect of the present invention, the substrate of the biochemical analysis unit is formed with 10,000 or more absorptive regions.

In a further preferred aspect of the present invention, the substrate of the biochemical analysis unit is formed with 50,000 or more absorptive regions.

In a further preferred aspect of the present invention, the substrate of the biochemical analysis unit is formed with 100,000 or more absorptive regions.

In a preferred aspect of the present invention, each of the plurality of absorptive regions formed in the substrate of the biochemical analysis unit has a size of less than 5 mm².

In a further preferred aspect of the present invention, each of the plurality of absorptive regions formed in the substrate of the biochemical analysis unit has a size of less than 1 mm².

In a further preferred aspect of the present invention, each of the plurality of absorptive regions formed in the substrate of the biochemical analysis unit has a size of less than 0.5 mm².

In a further preferred aspect of the present invention, each of the plurality of absorptive regions formed in the substrate of the biochemical analysis unit has a size of less than 0.1 mm².

In a further preferred aspect of the present invention, each of the plurality of absorptive regions formed in the substrate of the biochemical analysis unit has a size of less than 0.05 mm².

In a further preferred aspect of the present invention, each of the plurality of absorptive regions formed in the substrate of the biochemical analysis unit has a size of less than 0.01 mm².

In a preferred aspect of the present invention, the plurality of absorptive regions are formed in the substrate of the biochemical analysis unit at a density of 10 or more per cm².

In a further preferred aspect of the present invention, the plurality of absorptive regions are formed in the substrate of the biochemical analysis unit at a density of 50 or more per cm².

In a further preferred aspect of the present invention, the plurality of absorptive regions are formed in the substrate of the biochemical analysis unit at a density of 100 or more per cm².

In a further preferred aspect of the present invention, the plurality of absorptive regions are formed in the substrate of the biochemical analysis unit at a density of 500 or more per cm².

In a further preferred aspect of the present invention, the plurality of absorptive regions are formed in the substrate of the biochemical analysis unit at a density of 1,000 or more per cm².

In a further preferred aspect of the present invention, the plurality of absorptive regions are formed in the substrate of the biochemical analysis unit at a density of 5,000 or more per cm².

In a further preferred aspect of the present invention, the plurality of absorptive regions are formed in the substrate of the biochemical analysis unit at a density of 10,000 or more per cm².

In a further preferred aspect of the present invention, the plurality of absorptive regions are formed in the substrate of the biochemical analysis unit at a density of 50,000 or more per cm².

In a further preferred aspect of the present invention, the plurality of absorptive regions are formed in the substrate of the biochemical analysis unit at a density of 100,000 or more per cm².

In a preferred aspect of the present invention, the plurality of absorptive regions are formed in the substrate of the biochemical analysis unit in a regular pattern.

In a preferred aspect of the present invention, each of the plurality of absorptive regions is formed substantially circular in the substrate of the biochemical analysis unit in a regular pattern.

In another preferred aspect of the present invention, each of the plurality of absorptive regions is formed substantially rectangular.

In a preferred aspect of the present invention, the substrate of the biochemical analysis unit has a property of attenuating radiation energy.

According to this preferred aspect of the present invention, even in the case of forming the plurality of absorptive regions in the substrate of the biochemical analysis unit at a high density, absorbing specific binding substances which can specifically bind a substance derived from a living organism and whose sequence, base length, composition and the like are known in the plurality of absorptive regions, hybridizing a substance derived from a living organism and labeled with a radioactive labeling substance and selectively labeling the plurality of absorptive regions with the radioactive labeling substance, when the biochemical analysis unit and a stimulable phosphor sheet formed with a stimulable phosphor layer to expose the stimulable phosphor layer formed in the stimulable phosphor sheet to the radioactive labeling substance selectively contained in the plurality of absorptive regions of the biochemical analysis unit, since the substrate of the biochemical analysis unit has a property of attenuating radiation energy, electron beams (β rays) released from the radioactive labeling substance contained in the individual absorptive regions of the biochemical analysis unit can be effectively prevented from scattering in the substrate of the biochemical analysis unit. Therefore, it is possible to cause electron beams (β rays) to selectively enter a corresponding region of the stimulable phosphor layer to expose only the corresponding regions of the stimulable phosphor layer thereto, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the substrate of the biochemical analysis unit has a property of reducing the energy of radiation to 1/5 or less when the radiation travels in the substrate by a distance equal to that between neighboring absorptive regions.

In a further preferred aspect of the present invention, the substrate of the biochemical analysis unit has a property of reducing the energy of radiation to 1/10 or less when the radiation travels in the substrate by a distance equal to that between neighboring absorptive regions.

In a further preferred aspect of the present invention, the substrate of the biochemical analysis unit has a property of reducing the energy of radiation to 1/50 or less when the radiation travels in the substrate by a distance equal to that between neighboring absorptive regions.

In a further preferred aspect of the present invention, the substrate of the biochemical analysis unit has a property of reducing the energy of radiation to 1/100 or less when the radiation travels in the substrate by a distance equal to that between neighboring absorptive regions.

In a further preferred aspect of the present invention, the substrate of the biochemical analysis unit has a property of reducing the energy of radiation to 1/500 or less when the radiation travels in the substrate by a distance equal to that between neighboring absorptive regions.

In a further preferred aspect of the present invention, the substrate of the biochemical analysis unit has a property of reducing the energy of radiation to 1/1,000 or less when the radiation travels in the substrate by a distance equal to that between neighboring absorptive regions.

In a preferred aspect of the present invention, the substrate of the biochemical analysis unit has a property of attenuating radiation energy.

According to this preferred aspect of the present invention, even in the case of forming the plurality of absorptive regions in the substrate of the biochemical analysis unit at a high density, absorbing specific binding substances which can specifically bind a substance derived from a living organism and whose sequence, base length, composition and the like are known in the plurality of absorptive regions, hybridizing a substance derived from a living organism and labeled with a fluorescent substance, thereby selectively labeling the plurality of absorptive regions with the fluorescent substance, irradiating the plurality of absorptive regions of the biochemical analysis unit with a stimulating ray, thereby exciting the fluorescent substance selectively contained in the plurality of absorptive regions and photoelectrically detecting fluorescence emission released from the plurality of absorptive region to produce biochemical analysis data, since the substrate of the biochemical analysis unit has a property of attenuating radiation energy, fluorescence emission released from the individual absorptive regions can be effectively prevented from scattering in the substrate of the biochemical analysis unit and mixing with fluorescence emission released from neighboring absorptive regions. Therefore, it is possible to produce biochemical analysis data with a high quantitative characteristic by photoelectrically detecting fluorescence emission.

Further, according to this preferred aspect of the present invention, even in the case of forming the plurality of absorptive regions in the substrate of the biochemical analysis unit at a high density, absorbing specific binding substances which can specifically bind a substance derived from a living organism and whose sequence, base length, composition and the like are known in the plurality of absorptive regions, hybridizing a substance derived from a living organism and labeled with a labeling substance which generates chemiluminescence emission when it contacts a chemiluminescent substrate, thereby selectively labeling the plurality of absorptive regions with the labeling substance which generates chemiluminescence emission when it contacts a chemiluminescent substrate, bringing the plurality of absorptive regions of the biochemical analysis unit into contact with a chemiluminescent substrate, thereby causing them to selectively release chemiluminescence emission and photoelectrically detecting chemiluminescence emission released from the plurality of absorptive regions of the biochemical analysis unit to produce biochemical analysis data, since the substrate of the biochemical analysis unit has a property of attenuating radiation energy, chemiluminescence emission released from the plurality of absorptive regions of the biochemical analysis unit can be effectively prevented from scattering in the substrate of the biochemical analysis unit and mixing with chemiluminescence emission released from neighboring absorptive regions. Therefore, it is possible to produce biochemical analysis data with a high quantitative characteristic by photoelectrically detecting chemiluminescence emission.

Furthermore, according to this preferred aspect of the present invention, even in the case of forming the plurality of absorptive regions in the substrate of the biochemical analysis unit at a high density, absorbing specific binding substances which can specifically bind a substance derived from a living organism and whose sequence, base length, composition and the like are known in the plurality of absorptive regions, hybridizing a substance derived from a living organism and labeled with a labeling substance which generates chemiluminescence emission when it contacts a chemiluminescent substrate, thereby recording chemiluminescence data in the plurality of absorptive regions of the biochemical analysis unit, bringing the plurality of absorptive regions of the biochemical analysis unit into contact with a chemiluminescent substrate, thereby causing them to selectively release chemiluminescence emission, superposing the biochemical analysis unit releasing chemiluminescence emission and a stimulable phosphor sheet formed with a stimulable phosphor layer, thereby exposing the stimulable phosphor layer formed in the stimulable phosphor sheet to chemiluminescence emission selectively released from the plurality of absorptive regions of the biochemical analysis unit and transferring chemiluminescence data to the stimulable phosphor layer, since the substrate of the biochemical analysis unit has a property of attenuating radiation energy, chemiluminescence emission released from the plurality of absorptive regions of the biochemical analysis unit can be effectively prevented from scattering in the substrate of the biochemical analysis unit. Therefore, it is possible to cause chemiluminescence emission selectively released from the plurality of absorptive regions of the biochemical analysis unit to selectively enter a corresponding region of the stimulable phosphor layer to expose only the corresponding regions of the stimulable phosphor layer thereto, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the substrate of the biochemical analysis unit has a property of reducing the energy of light to 1/5 or less when the light travels in the substrate by a distance equal to that between neighboring absorptive regions.

In a further preferred aspect of the present invention, the substrate of the biochemical analysis unit has a property of reducing the energy of light to 1/10 or less when the light travels in the substrate by a distance equal to that between neighboring absorptive regions.

In a further preferred aspect of the present invention, the substrate of the biochemical analysis unit has a property of reducing the energy of light to 1/50 or less when the light travels in the substrate by a distance equal to that between neighboring absorptive regions.

In a further preferred aspect of the present invention, the substrate of the biochemical analysis unit has a property of reducing the energy of light to 1/100 or less when the light travels in the substrate by a distance equal to that between neighboring absorptive regions.

In a further preferred aspect of the present invention, the substrate of the biochemical analysis unit has a property of reducing the energy of light to 1/500 or less when the light travels in the substrate by a distance equal to that between neighboring absorptive regions.

In a further preferred aspect of the present invention, the substrate of the biochemical analysis unit has a property of reducing the energy of light to 1/1,000 or less when the light travels in the substrate by a distance equal to that between neighboring absorptive regions.

In the present invention, the material for forming the substrate of the biochemical analysis unit is preferably capable of attenuating radiation energy and/or light energy but is not particularly limited. The material for forming the substrate of the biochemical analysis unit may be any type of inorganic compound material or organic compound material and the substrate of the biochemical analysis unit can preferably be formed of a metal material, a ceramic material or a plastic material.

Illustrative examples of inorganic compound materials preferably usable for forming the substrate of the biochemical analysis unit in the present invention include metals such as gold, silver, copper, zinc, aluminum, titanium, tantalum, chromium, iron, nickel, cobalt, lead, tin, selenium and the like; alloys such as brass, stainless steel, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like.

In the present invention, a high molecular compound can preferably be used as an organic compound material preferably usable for forming the substrate of the biochemical analysis unit. Illustrative examples of high molecular compounds preferably usable for forming the substrate of the biochemical analysis unit in the present invention include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifuluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Since the capability of attenuating radiation energy generally increases as specific gravity increases, the substrate of the biochemical analysis unit is preferably formed of a compound material or a composite material having specific gravity of 1.0 g/cm³ or more and more preferably formed of a compound material or a composite material having specific gravity of 1.5 g/cm³ to 23 g/cm³.

Further, since the capability of attenuating light energy generally increases as scattering and/or absorption of light increases, the substrate of the biochemical analysis unit preferably has absorbance of 0.3 per cm (thickness) or more and more preferably has absorbance of 1 per cm (thickness) or more. The absorbance can be determined by placing an integrating sphere immediately behind a plate-like member having a thickness of T cm, measuring an amount A of transmitted light at a wavelength of probe light or emission light used for measurement by a spectrophotometer, and calculating A/T. In the present invention, a light scattering substance or a light absorbing substance may be added to the substrate of the biochemical analysis unit in order to improve the capability of attenuating light energy. Particles of a material different from a material forming the substrate of the biochemical analysis unit may be preferably used as a light scattering substance and a pigment or dye may be preferably used as a light absorbing substance.

In the present invention, a porous material or a fiber material may be preferably used as the absorptive material for forming the absorptive regions of the biochemical analysis unit. The absorptive regions may be formed by combining a porous material and a fiber material.

In the present invention, a porous material for forming the absorptive regions of the biochemical analysis unit may be any type of an organic material or an inorganic material and may be an organic/inorganic composite material.

In the present invention, an organic porous material used for forming the absorptive regions of the biochemical analysis unit is not particularly limited but a carbon porous material such as an activated carbon or a porous material capable of forming a membrane filter is preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof.

In the present invention, an inorganic porous material used for forming the absorptive regions of the biochemical analysis unit is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof.

In the present invention, a fiber material used for forming the absorptive regions of the biochemical analysis unit is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

In the present invention, the absorptive layer of the biochemical analysis unit may be formed using an oxidization process such as an electrolytic process, a plasma process, an arc discharge process or the like; a primer process using a silane coupling agent, titanium coupling agent or the like; and a surface-active agent process or the like.

The above and other objects of the present invention can be also accomplished by a receptor-ligand associating apparatus including a biochemical analysis unit holding means for holding a biochemical analysis unit formed with a plurality of absorptive regions which are spaced apart from each other and in which receptors or ligands are fixed, and a solution feeding means for feeding a solution so as to pass through the plurality of absorptive regions of the biochemical analysis unit held by the biochemical analysis unit holding means.

According to the present invention, since a receptor-ligand associating apparatus includes a biochemical analysis unit holding means for holding a biochemical analysis unit formed with a plurality of absorptive regions which are spaced apart from each other and in which receptors or ligands are fixed, and a solution feeding means for feeding a solution so as to pass through each of the plurality of absorptive regions of the biochemical analysis unit held by the biochemical analysis unit holding means, it is possible to markedly increase the moving rate of the ligand or the receptor through the plurality of absorptive regions by feeding a solution containing a ligand or a receptor to the biochemical analysis unit so as to pass through the plurality of absorptive regions of the biochemical analysis unit and therefore, to markedly increase the reaction rate of association of the receptors or the ligands fixed in the plurality of absorptive regions of the biochemical analysis unit and the ligand or the receptor contained in the reaction solution. It is further possible to markedly increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner.

Furthermore, according to the present invention, since a reaction solution containing a ligand or a receptor can be fed to the plurality of absorptive regions which are formed in a biochemical analysis unit and in which receptors or ligands are fixed so as to pass through the plurality of absorptive regions, thereby selectively associating the ligand or the receptor contained in the reaction solution with the receptors or the ligands fixed in the plurality of absorptive regions of the biochemical analysis unit, the repeatability of the receptor-ligand association reaction can be markedly improved.

In a preferred aspect of the present invention, the biochemical analysis unit holding means is constituted by a reaction vessel for accommodating the biochemical analysis unit therein and the solution feeding means includes a solution circulation passage communicating with spaces in the reaction vessel on the opposite sides of the biochemical analysis unit accommodated in the reaction vessel and a pump for circulating a solution in the reaction vessel and the solution circulation passage.

According to this preferred aspect of the present invention, since the biochemical analysis unit holding means is constituted by a reaction vessel for accommodating the biochemical analysis unit therein and the solution feeding means includes a solution circulation passage communicating with spaces in the reaction vessel on the opposite sides of the biochemical analysis unit held in the reaction vessel and a pump for circulating a solution in the reaction vessel and the solution circulation passage, a reaction solution containing a ligand or a receptor can be forcibly fed into the reaction vessel by the pump so as to pass through the plurality of absorptive regions of the biochemical analysis unit via the solution circulating passage. Therefore, since it is possible to markedly increase the moving rate of the ligand or the receptor through the plurality of absorptive regions, it is possible to markedly increase the reaction rate of association of the receptors or the ligands fixed in the plurality of absorptive regions of the biochemical analysis unit and the ligand or the receptor contained in the reaction solution. Further, since it is possible to markedly increase the possibility of association of the ligand or the receptor contained in the reaction solution with the receptors or the ligands fixed in deep portions of the plurality of absorptive regions of the biochemical analysis unit, the ligand or the receptor contained in the reaction solution can be associated with the receptors or the ligands fixed in the plurality of absorptive regions of the biochemical analysis unit in a desired manner.

Moreover, according to this preferred aspect of the present invention, since a reaction solution containing a ligand or a receptor can be forcibly fed by the pump to the plurality of absorptive regions which are formed in a biochemical analysis unit and in which receptors or ligands are fixed so as to pass through the plurality of absorptive regions, thereby selectively associating the ligand or the receptor contained in the reaction solution with the receptors or the ligands fixed in the plurality of absorptive regions of the biochemical analysis unit, the repeatability of the receptor-ligand association reaction can be markedly improved.

In a further preferred aspect of the present invention, the pump is constituted so as to be driven in both a forward direction and a reverse direction.

According to this preferred aspect of the present invention, since the pump is constituted so as to be driven in both a forward direction and a reverse direction, the reaction solution can be fed to the plurality of absorptive regions of the biochemical analysis unit in both an obverse direction and a reverse direction so as to pass through the plurality of absorptive regions. Therefore, since it is possible to much more increase the possibility of association of the ligand or the receptor contained in the reaction solution with the receptors or the ligands fixed in deep portions of the plurality of absorptive regions of the biochemical analysis unit, the ligand or the receptor contained in the reaction solution can be associated with the receptors or the ligands fixed in the plurality of absorptive regions of the biochemical analysis unit in a desired manner and the repeatability of the receptor-ligand association reaction can be markedly improved.

In a further preferred aspect of the present invention, a solution discharge passage is further connected via a change-over valve to the solution circulation passage downstream of the reaction vessel with respect a direction in which a solution flows when the pump is driven in the forward direction.

If a cleaning solution for cleaning the plurality of absorptive regions of the biochemical analysis unit is circulated into the reaction vessel, there is s risk of a ligand or a receptor which should not be bonded with a receptor or a ligand fixed to the absorptive region of the biochemical analysis unit but was bonded therewith and peeled off therefrom by the cleaning solution again binding with the receptor or the ligand fixed to the absorptive region. However, according to this preferred aspect of the present invention, since a solution discharge passage is further connected via a change-over valve to the solution circulation passage downstream of the reaction vessel with respect a direction in which a solution flows when the pump is driven in the forward direction, when the plurality of absorptive regions of the biochemical analysis unit is cleaned with the cleaning solution, the cleaning solution can be discharged through the solution discharge passage without being circulated to the reaction vessel and only reaction solution containing a ligand or a receptor can be circulated via the solution circulation passage. Therefore, it is possible to improve not only the efficiency of a receptor-ligand association reaction but also the efficiency of the cleaning operation.

In a further preferred aspect of the present invention, the apparatus for conducting a receptor-ligand association reaction further includes at least one reaction solution sources capable of communicating with the solution circulation passage for accommodating a reaction solution containing a ligand or a receptor.

In a further preferred aspect of the present invention, the apparatus for conducting a receptor-ligand association reaction further includes two or more reaction solution sources for accommodating a reaction solution containing a ligand or a receptor and the two or more reaction solution sources are constituted so as to selectively communicate with the solution circulation passage.

In a further preferred aspect of the present invention, the apparatus for conducting a receptor-ligand association reaction further includes at least one cleaning solution source capable of communicating with the solution circulation passage for accommodating a cleaning solution.

In a further preferred aspect of the present invention, the apparatus for conducting a receptor-ligand association reaction further includes two or more reaction solution sources for accommodating a reaction solution containing a ligand or a receptor and at least one cleaning solution source for accommodating a cleaning solution and the two or more reaction solution sources and at least one cleaning solution source are constituted so as to selectively communicate with the solution circulation passage.

In a preferred aspect of the present invention, the biochemical analysis holding means is constituted by a reaction vessel for accommodating the biochemical analysis unit and a solution and the solution feeding means is constituted as ultrasound generating means for generating ultrasound in the solution accommodated in the reaction vessel in a direction cutting through the plurality of absorptive regions of the biochemical analysis unit accommodated in the reaction vessel.

According to this preferred aspect of the present invention, since the biochemical analysis holding means is constituted by a reaction vessel for accommodating the biochemical analysis unit and a solution and the solution feeding means is constituted as ultrasound generating means for generating ultrasound in the solution accommodated in the reaction vessel in a direction acutting through the plurality of absorptive regions of the biochemical analysis unit accommodated in the reaction vessel, a reaction solution containing a ligand or a receptor can be forcibly moved through each of the plurality of absorptive regions of the biochemical analysis unit by generating ultrasound in the reaction solution in a direction cutting through the plurality of absorptive regions of the biochemical analysis unit accommodated in the reaction vessel by the ultrasound generating means. Therefore, since it is possible to markedly increase the moving rate of the ligand or the receptor through the plurality of absorptive regions, it is possible to markedly increase the reaction rate of association of the receptors or the ligands fixed in the plurality of absorptive regions of the biochemical analysis unit and the ligand or the receptor contained in the reaction solution. Further, it is possible to markedly increase the possibility of association of the ligand or the receptor contained in the reaction solution with the receptors or the ligands fixed in deep portions of the plurality of absorptive regions of the biochemical analysis unit. Therefore, the ligand or the receptor contained in the reaction solution can be associated with the receptors or the ligands fixed in the plurality of absorptive regions of the biochemical analysis unit in a desired manner.

Furthermore, according to this preferred aspect of the present invention, since a reaction solution containing a ligand or a receptor can be fed to the plurality of absorptive regions which are formed in a biochemical analysis unit and in which receptors or ligands are fixed so as to cut through the plurality of absorptive regions, thereby selectively associating the ligand or the receptor contained in the reaction solution with the receptors or the ligands fixed in the plurality of absorptive regions of the biochemical analysis unit, the repeatability of the receptor-ligand association reaction can be markedly improved.

In a further preferred aspect of the present invention, the ultrasound generating means includes a pair of ultrasonic oscillators, one of them being constituted so as to generate ultrasound in a solution cutting through the plurality of absorptive regions of the biochemical analysis unit accommodated in the reaction vessel in an obverse direction and the other being constituted so as to generate ultrasound in a solution cutting through the plurality of absorptive regions of the biochemical analysis unit accommodated in the reaction vessel in a reverse direction.

According to this preferred aspect of the present invention, since the ultrasound generating means includes a pair of ultrasonic oscillators, one of them being constituted so as to generate ultrasound in a solution cutting through each of the plurality of absorptive regions of the biochemical analysis unit accommodated in the reaction vessel in an obverse direction and the other being constituted so as to generate ultrasound in a solution cutting through each of the plurality of absorptive regions of the biochemical analysis unit accommodated in the reaction vessel in a reverse direction, the reaction solution can be fed to the plurality of absorptive regions of the biochemical analysis unit accommodated in the reaction vessel in both an obverse direction and a reverse direction so as to cut through the plurality of absorptive regions by alternately actuating the pair of ultrasonic oscillators. Therefore, since it is possible to much more increase the possibility of association of the ligand or the receptor contained in the reaction solution with the receptors or the ligands fixed in deep portions of the plurality of absorptive regions of the biochemical analysis unit, the ligand or the receptor contained in the reaction solution can be associated with the receptors or the ligands fixed in the plurality of absorptive regions of the biochemical analysis unit in a desired manner and the repeatability of the receptor-ligand association reaction can be markedly improved.

In a preferred aspect of the present invention, the apparatus for conducting a receptor-ligand association reaction further includes a plurality of tubular members for feeding a solution, each having an opening facing one of the plurality of absorptive regions of the biochemical analysis unit held by the biochemical analysis unit holding means, and temperature control means, each independently provided for a group consisting of two or more tubular members among the plurality of tubular members for controlling the temperature of a solution flowing through the two or more tubular members constituting the group.

According to this preferred aspect of the present invention, since the apparatus for conducting a receptor-ligand association reaction further includes a plurality of tubular members for feeding a solution, each having an opening facing one of the plurality of absorptive regions of the biochemical analysis unit held by the biochemical analysis unit holding means, and temperature control means, each independently provided for a group consisting of two or more tubular members among the plurality of tubular members for controlling the temperature of a solution flowing through the two or more tubular members constituting the group, it is possible to feed a reaction solution whose temperature is controlled for the absorptive regions of the biochemical analysis unit corresponding to the two or more tubular members constituting each of the groups to the plurality of absorptive regions of the biochemical analysis unit, thereby selectively associating the ligand or the receptor contained in the reaction solution with the receptors or the ligands fixed in the plurality of absorptive regions of the biochemical analysis unit. Therefore, if the same kind of receptor or ligand is fixed in the absorptive regions corresponding to the two or more tubular members constituting each of the groups, since it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit, the ligand or the receptor which is to be associated with the receptors or the ligands fixed in the individual absorptive regions of the biochemical analysis unit can be associated therewith in a desired manner and it is possible to effectively prevent noise from being generated in biochemical analysis data and to produce biochemical analysis data having an excellent high quantitative characteristic with excellent repeatability.

Moreover, according to this preferred aspect of the present invention, since the apparatus for conducting a receptor-ligand association reaction further includes a plurality of tubular members for feeding a solution, each having an opening facing one of the plurality of absorptive regions of the biochemical analysis unit held by the biochemical analysis unit holding means, and temperature control means, each independently provided for a group consisting of two or more tubular members among the plurality of tubular members for controlling the temperature of a solution flowing through the two or more tubular members constituting the group, it is possible to feed a cleaning solution whose temperature is controlled for the absorptive regions of the biochemical analysis unit corresponding to the two or more tubular members constituting each of groups to the plurality of absorptive regions of the biochemical analysis unit, thereby cleaning the plurality of absorptive regions of the biochemical analysis unit. Therefore, if the same kind of receptor or ligand is fixed in the absorptive regions corresponding to the two or more tubular members constituting each of the groups, even in the case where a ligand or the receptor which should not be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the plurality of tubular members are divided into a plurality of blocks each including two or more tubular members and an independent temperature control means is provided for each of the blocks.

According to this preferred aspect of the present invention, since the plurality of tubular members are divided into a plurality of blocks each including two or more tubular members and an independent temperature control means is provided for each of the blocks, it is possible to feed a reaction solution whose temperature is controlled for the absorptive regions of the biochemical analysis unit corresponding to the two or more tubular members constituting each of the blocks to the plurality of absorptive regions of the biochemical analysis unit, thereby selectively associating the ligand or the receptor contained in the reaction solution with the receptors or the ligands fixed in the plurality of absorptive regions of the biochemical analysis unit. Therefore, if the same kind of receptor or ligand is fixed in the absorptive regions corresponding to the two or more tubular members constituting each of the blocks, since it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit, the ligand or the receptor which is to be associated with the receptors or the ligands fixed in the individual absorptive regions of the biochemical analysis unit can be associated therewith in a desired manner and it is possible to effectively prevent noise from being generated in biochemical analysis data and to produce biochemical analysis data having an excellent high quantitative characteristic with excellent repeatability.

Moreover, according to this preferred aspect of the present invention, since the plurality of tubular members are divided into a plurality of blocks each including two or more tubular members and an independent temperature control means is provided for each of the blocks, it is possible to feed a cleaning solution whose temperature is controlled for the absorptive regions of the biochemical analysis unit corresponding to the two or more tubular members constituting each of blocks to the plurality of absorptive regions of the biochemical analysis unit, thereby cleaning the plurality of absorptive regions of the biochemical analysis unit. Therefore, if the same kind of receptor or ligand is fixed in the absorptive regions corresponding to the two or more tubular members constituting each of the blocks, even in the case where a ligand or the receptor which should not be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the apparatus for conducting a receptor-ligand association reaction further includes a plurality of tubular members for feeding a solution, each having an opening facing one of the plurality of absorptive regions of the biochemical analysis unit held by the biochemical analysis unit holding means, the plurality of tubular members being disposed in the manner of a matrix at least in the vicinity of the biochemical analysis unit held by the biochemical analysis unit holding means, and an independent temperature control means is provided for the plurality of tubular members constituting each line of matrix.

According to this preferred aspect of the present invention, since the plurality of tubular members are disposed in the manner of a matrix at least in the vicinity of the biochemical analysis unit held by the biochemical analysis unit holding means and an independent temperature control means is provided for the plurality of tubular members constituting each line of matrix, it is possible to feed a reaction solution whose temperature is controlled for the absorptive regions of the biochemical analysis unit corresponding to the plurality of tubular members constituting each line of matrix to the plurality of absorptive regions of the biochemical analysis unit, thereby selectively associating the ligand or the receptor contained in the reaction solution with the receptors or the ligands fixed in the plurality of absorptive regions of the biochemical analysis unit. Therefore, if the same kind of receptor or ligand is fixed in the absorptive regions corresponding to the two or more tubular members constituting each line of matrix, since it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit, the ligand or the receptor which is to be associated with the receptors or the ligands fixed in the individual absorptive regions of the biochemical analysis unit can be associated therewith in a desired manner and it is possible to effectively prevent noise from being generated in biochemical analysis data and to produce biochemical analysis data having an excellent high quantitative characteristic with excellent repeatability.

Moreover, according to this preferred aspect of the present invention, since the plurality of tubular members are disposed in the manner of a matrix at least in the vicinity of the biochemical analysis unit held by the biochemical analysis unit holding means and an independent temperature control means is provided for the plurality of tubular members constituting each line of matrix, it is possible to feed a cleaning solution whose temperature is controlled for the absorptive regions of the biochemical analysis unit corresponding to the two or more tubular members constituting each line of matrix to the plurality of absorptive regions of the biochemical analysis unit, thereby cleaning the plurality of absorptive regions of the biochemical analysis unit. Therefore, if the same kind of receptor or ligand is fixed in the absorptive regions corresponding to the plurality of tubular members constituting each line of matrix, even in the case where a ligand or the receptor which should not be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the apparatus for conducting a receptor-ligand association reaction further includes a plurality of tubular members for feeding a solution, each having an opening facing one of the plurality of absorptive regions of the biochemical analysis unit held by the biochemical analysis unit holding means, and an independent temperature control means is provided for each of the plurality of tubular members.

According to this preferred aspect of the present invention, since an independent temperature control means is provided for each of the plurality of tubular members, it is possible to a reaction solution whose temperature is controlled for the absorptive region of the biochemical analysis unit corresponding to each of the plurality of tubular members to the plurality of absorptive regions of the biochemical analysis unit, thereby selectively associating the ligand or the receptor contained in the reaction solution with the receptors or the ligands fixed in the plurality of absorptive regions of the biochemical analysis unit. Therefore, since it is possible to effectively prevent the ligand or the receptor associated with the receptors or the ligands fixed in the individual absorptive regions of the biochemical analysis unit, which is to be associated therewith from being peeled off from the receptors or the ligands fixed in the individual absorptive regions of the biochemical analysis unit, the ligand or the receptor which is to be associated with the receptors or the ligands fixed in the individual absorptive regions of the biochemical analysis unit can be associated therewith in a desired manner and it is possible to effectively prevent noise from being generated in biochemical analysis data and to produce biochemical analysis data having an excellent high quantitative characteristic with excellent repeatability.

Moreover, according to this preferred aspect of the present invention, since an independent temperature control means is provided for each of the plurality of tubular members, it is possible to feed a cleaning solution whose temperature is controlled for the absorptive region of the biochemical analysis unit corresponding to each of the two or more tubular members to the plurality of absorptive regions of the biochemical analysis unit, thereby cleaning the plurality of absorptive regions of the biochemical analysis unit. Therefore, even in the case where a ligand or the receptor which should not be associated with the receptors or the ligands fixed in the plurality of absorptive regions of the biochemical analysis unit has been bonded therewith in the course of the receptor-ligand association reaction, it is possible to effectively peel off and remove the ligand or the receptor which should not be associated with the receptors or the ligands fixed in the plurality of absorptive regions of the biochemical analysis unit from the plurality of absorptive regions. Therefore, since the ligand or the receptor which is to be associated with the receptors or the ligands fixed in the individual absorptive regions of the biochemical analysis unit can be associated therewith in a desired manner, it is possible to effectively prevent noise from being generated in biochemical analysis data and to produce biochemical analysis data having an excellent high quantitative characteristic with excellent repeatability.

In a further preferred aspect of the present invention, the apparatus for conducting a receptor-ligand association reaction further includes a plurality of solution collecting tubular members each facing one of the openings of the plurality of tubular members via one of the plurality of absorptive regions of the biochemical analysis unit held by the biochemical analysis unit holding means and being adapted for receiving a solution fed to the absorptive region of the biochemical analysis unit from the tubular member.

According to this preferred aspect of the present invention, since the apparatus for conducting a receptor-ligand association reaction further includes a plurality of solution collecting tubular members each facing one of the openings of the plurality of tubular members via one of the plurality of absorptive regions of the biochemical analysis unit held by the biochemical analysis unit holding means and being adapted for receiving a solution fed to the absorptive region of the biochemical analysis unit from the tubular member, a reaction solution containing a ligand or a receptor can be collected and repeatedly fed to the plurality of absorptive regions of the biochemical analysis unit, thereby selectively associating the ligand or the receptor contained in the reaction solution with the receptors or the ligands fixed in the plurality of absorptive regions of the biochemical analysis unit. Therefore, the ligand or the receptor which is to be associated with the receptors or the ligands fixed in the individual absorptive regions of the biochemical analysis unit can be associated therewith in a desired manner and it is possible to effectively prevent noise from being generated in biochemical analysis data and to produce biochemical analysis data having an excellent high quantitative characteristic with excellent repeatability.

In a further preferred aspect of the present invention, the apparatus for conducting a receptor-ligand association reaction further includes a thermal insulating member between the temperature control means and the biochemical analysis unit holding means and the plurality of tubular members are disposed so as to extend in the thermal insulating member.

In a further preferred aspect of the present invention, the apparatus for conducting a receptor-ligand association reaction further includes at least one reaction solution source capable of communicating with the plurality of tubular members at end portions opposite to the openings of the plurality of tubular members for accommodating a reaction solution containing a ligand or a receptor.

In a further preferred aspect of the present invention, the apparatus for conducting a receptor-ligand association reaction further includes two or more reaction solution sources for accommodating a reaction solution containing a ligand or a receptor and the two or more reaction solution sources are constituted so as to selectively communicate with the plurality of tubular members at end portions opposite to the openings of the plurality of tubular members.

In a further preferred aspect of the present invention, the apparatus for conducting a receptor-ligand association reaction further includes at least one cleaning solution source capable of communicating with the plurality of tubular members at end portions opposite to the openings of the plurality of tubular members for accommodating a cleaning solution.

In a further preferred aspect of the present invention, the apparatus for conducting a receptor-ligand association reaction further includes two or more reaction solution sources for accommodating a reaction solution containing a ligand or a receptor and at least one cleaning solution source for accommodating a cleaning solution and the two or more reaction solution sources and the at least one cleaning solution source are constituted so as to selectively communicate with the plurality of tubular members at end portions opposite to the openings of the plurality of tubular members.

The above and other objects and features of the present invention will become apparent from the following description made with reference to the accompanying drawings.

### BRIEF DESCRIPTION OF THE DRAWINGS

Figure 1 is a schematic perspective view showing a biochemical analysis unit used in a method for conducting a receptor-ligand association reaction which is a preferred embodiment of the present invention.
Figure 2 is a schematic front view showing a spotting device.
Figure 3 is a schematic longitudinal cross sectional view showing an apparatus for conducting a receptor-ligand association reaction which is a preferred embodiment of the present invention.
Figure 4 is a schematic perspective view showing a stimulable phosphor sheet onto which radiation data are to be transferred.
Figure 5 is a schematic cross-sectional view showing a method for exposing a number of the stimulable phosphor layer regions formed in the stimulable phosphor sheet to a radioactive labeling substance contained in a number of the absorptive regions formed in a biochemical analysis unit.
Figure 6 is a schematic view showing a scanner for reading radiation data recorded in a number of the stimulable phosphor layer regions formed in the support of the stimulable phosphor sheet to produce biochemical analysis data.
Figure 7 is a schematic perspective view showing details in the vicinity of a photomultiplier of a scanner shown in Figure 6.
Figure 8 is a schematic cross-sectional view taken along a line A-A in Figure 7.
Figure 9 is a schematic cross-sectional view taken along a line B-B in Figure 7.
Figure 10 is a schematic cross-sectional view taken along a line C-C in Figure 7.
Figure 11 is a schematic cross-sectional view taken along a line D-D in Figure 7.
Figure 12 is a schematic plan view showing the scanning mechanism of an optical head.
Figure 13 is a block diagram of a control system, an input system, a drive system and a detection system of the scanner shown in Figure 6.
Figure 14 is a schematic perspective view showing a stimulable phosphor sheet onto which chemiluminescence data are to be transferred.
Figure 15 is a schematic view showing a scanner for reading chemiluminescence data recorded in a number of stimulable phosphor layer regions formed in a support of a stimulable phosphor sheet 80 and producing biochemical analysis data.
Figure 16 is a schematic perspective view showing details in the vicinity of a photomultiplier of a scanner shown in Figure 15.
Figure 17 is a schematic cross-sectional view taken along a line E-E in Figure 16.
Figure 18 is a schematic front view showing a data producing system for reading chemiluminescence data recorded in a number of absorptive regions formed in a substrate of a biochemical analysis unit, and producing biochemical analysis data.
Figure 19 is a schematic longitudinal cross sectional view showing a cooled CCD camera.
Figure 20 is a schematic vertical cross sectional view showing a dark box of a data producing system.
Figure 21 is a block diagram of a personal computer of a data producing system and peripheral devices thereof.
Figure 22 is a schematic longitudinal cross sectional view showing an apparatus for conducting a receptor-ligand association reaction which is another preferred embodiment of the present invention.
Figure 23 is a schematic longitudinal cross sectional view showing an apparatus for conducting a receptor-ligand association reaction which is a further preferred embodiment of the present invention.
Figure 24 is a schematic perspective view showing another example of a biochemical analysis unit.
Figure 25 is a schematic perspective view showing an apparatus for conducting a receptor-ligand association reaction which is another preferred embodiment of the present invention.

### DESCRIPTION OF THE PREFERRED EMBODIMENTS

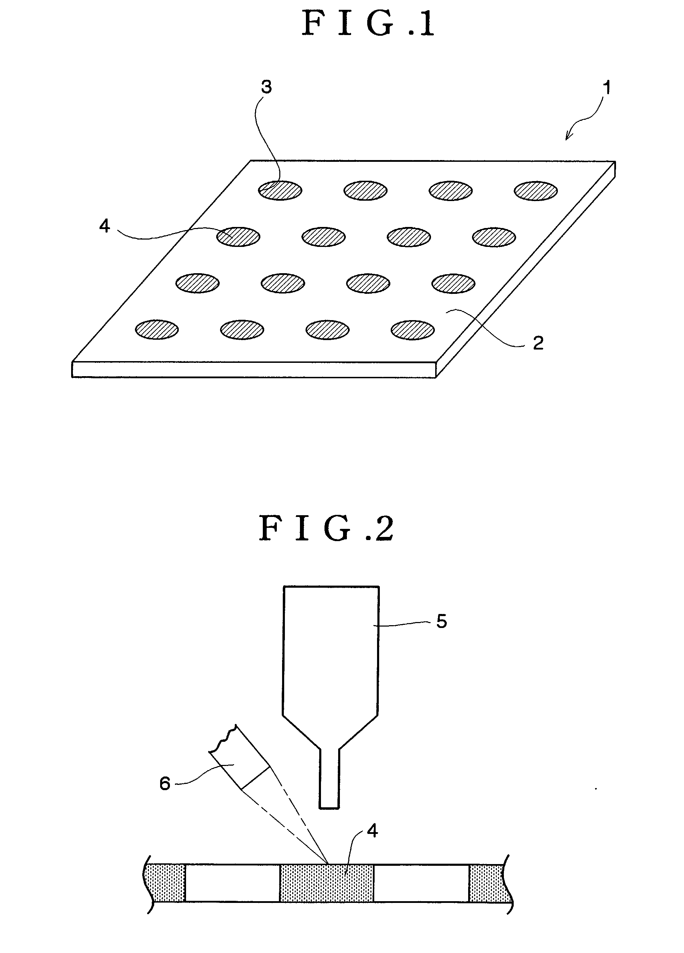

Figure 1 is a schematic perspective view showing a biochemical analysis unit used in a method for conducting a receptor-ligand association reaction which is a preferred embodiment of the present invention.

As shown in Figure 1, a biochemical analysis unit 1 according to this embodiment includes a substrate 2 formed of stainless steel and formed with a number of substantially circular through-holes 3 at a high density, and a number of absorptive regions 4 are dot-like formed by charging nylon-6 in the through-holes 3.

Although not accurately shown in Figure 1, in this embodiment, about 10,000 through-holes 3 having a size of about 0.01 mm² are regularly formed at a density of about 5,000 per cm² in the substrate 2.

A number of absorptive regions 4 are formed by charging nylon-6 in the through-holes 3 formed in the substrate in such a manner that the surfaces of the absorptive regions 4 are located at the same height level as that of the substrate 2.

Figure 2 is a schematic front view showing a spotting device.

As shown in Figure 2, when biochemical analysis is performed, a solution containing specific binding substances such as a plurality of cDNAs whose sequences are known but differ from each other are spotted using a spotting device 5 onto a number of the absorptive regions 4 of the biochemical analysis unit 1 and the specific binding substances are fixed therein.

As shown in Figure 2, the spotting device includes an injector 5 for ejecting a solution of specific binding substances toward the biochemical analysis unit 1 and a CCD camera 6 and is constituted so that the solution of specific binding substances such as cDNAs are spotted from the injector 6 when the tip end portion of the injector 5 and the center of the absorptive region 4 into which the solution containing specific binding substances is to be spotted are determined to coincide with each other as a result of viewing them using the CCD camera 6, thereby ensuring that the solution of specific binding substances can be accurately spotted into a number of the absorptive regions 4 of the biochemical analysis unit 1.

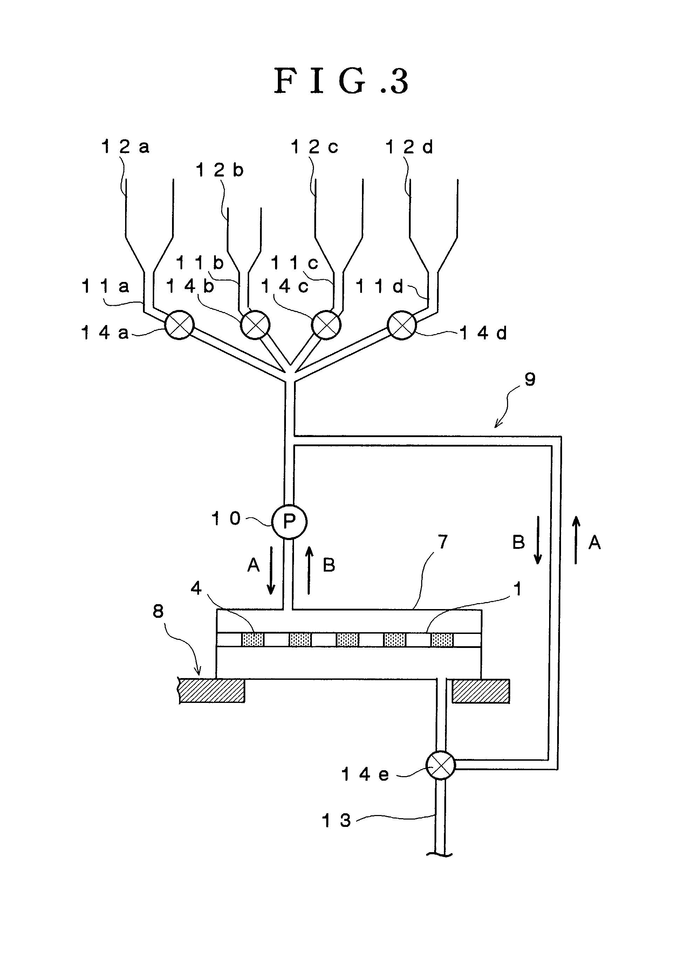

Figure 3 is a schematic longitudinal cross sectional view showing an apparatus for conducting a receptor-ligand association reaction which is a preferred embodiment of the present invention.

As shown in Figure 3, the apparatus conducting for a receptor-ligand association reaction according to this embodiment includes a cartridge 7 for accommodating the biochemical analysis unit 1, a stage 8 for holding the cartridge 7, a solution circulation pipe 9 connected to the cartridge 7 through which a hybridization buffer, a mixed solution of a hybridization buffer and a probe solution, an antibody solution or a cleaning solution flows, a pump 10 provided in the solution circulation pipe 9 for forcibly feeding a hybridization buffer, a mixed solution of a hybridization buffer and a probe solution, an antibody solution or a cleaning solution into the cartridge 7 via the solution circulation pipe 9, a hybridization buffer tank 12a connected to the solution circulation pipe 9 via a hybridization buffer feed pipe 11a for accommodating a hybridization buffer, a probe solution chip 12b connected to the solution circulation pipe 9 via a probe solution feed pipe 11b for accommodating a probe solution, an antibody solution tank connected to the solution circulation pipe 9 via an antibody solution feed pipe 11c for accommodating an antibody solution, a cleaning solution tank 12d connected to the solution circulation pipe 9 via a cleaning solution feed pipe 11d for accommodating a cleaning solution, and a solution discharge pipe 13 connected to the solution circulation pipe 9 below the cartridge 7.

As shown in Figure 3, a change-over valve 14a is provided in the hybridization buffer feed pipe 11a and a change-over valve 14b is provided in the probe solution feed pipe 11b. Further, a change-over valve 14c is provided in the antibody solution feed pipe 11c and a change-over valve 14d is provided in the cleaning solution feed pipe 11d.

A change-over valve 14e is provided at a connection between the solution circulation pipe 9 and the solution discharge pipe 13.

In this embodiment, the pump 10 is constituted as to be driven in both the forward direction and the reverse direction.

The change-over valve 14a provided in the hybridization buffer feed pipe 11a is constituted as a three-way valve so that it can selectively assume a first position where the hybridization buffer feed pipe 11a and the solution circulation pipe 9 communicate with each other, a second position where the atmosphere and the solution circulation pipe 9 communicate with each other or a third position where communication between the hybridization buffer tank 12a and the atmosphere, and the solution circulation pipe 9 is shut off, and the change-over valve 14b provided in the probe solution feed pipe 11b is constituted as a three-way valve so that it can selectively assume a first position where the probe solution feed pipe 11b and the solution circulation pipe 9 communicate with each other, a second position where the atmosphere and the solution circulation pipe 9 communicate with each other or a third position where communication between the probe solution chip 12b and the atmosphere, and the solution circulation pipe 9 is shut off.

The change-over valve 14c provided in the antibody solution feed pipe 11c is constituted as a three-way valve so that it can selectively assume a first position where the antibody solution feed pipe 11c and the solution circulation pipe 9 communicate with each other, a second position where the atmosphere and the solution circulation pipe 9 communicate with each other or a third position where communication between the antibody solution tank 12c and the atmosphere, and the solution circulation pipe 9 is shut off, and the change-over valve 14d provided in the cleaning solution feed pipe 11d is constituted as a three-way valve so that it can selectively assume a first position where the cleaning solution feed pipe 11d and the solution circulation pipe 9 communicate with each other, a second position where the atmosphere and the solution circulation pipe 9 communicate with each other or a third position where communication between the cleaning solution feed pipe 11d and the atmosphere, and the solution circulation pipe 9 is shut off.

Further, the change-over valve 14e provided at the connection between the solution circulation pipe 9 and the solution discharge pipe 13 is constituted as a two-way valve and so as to be able to assume a first position where the upstream portion of the solution circulation pipe 9 and the downstream portion of the solution circulation pipe 9 communicate with each other and a second position where the solution circulation pipe 9 and the solution discharge pipe 13 communicate with each other.

In the thus constituted apparatus for conducting receptor-legand association, a substance derived from a living body, labeled with a labeling substance and contained in a probe solution selectively hybridizes specific binding substances contained in a number of the absorptive regions 4 of the biochemical analysis unit 1 in the following manner.

The biochemical analysis unit 1 formed with a number of the absorptive regions 4 in which specific binding substances are absorbed is first accommodated in the cartridge 7 held by the stage 8 and a hybridization buffer is prepared and accommodated in the hybridization buffer tank 12a.

Then, the change-over valve 14b provided in the probe solution feed pipe 11b, the change-over valve 14c provided in the antibody solution feed pipe 11c and the change-over valve 14d provided in the cleaning solution feed pipe 11d are located at their second positions where the atmosphere and the solution circulation pipe 9 communicate with each other and the change-over valve 14e provided at the connection between the solution circulation pipe 9 and the solution discharge pipe 13 is located at its first position.

When the change-over valve 14a provided in the hybridization buffer feed pipe 11a has been located at its first position where the hybridization buffer feed pipe 11a and the solution circulation pipe 9 communicate with each other, the pump 10 is driven in the forward direction.

As a result, a hybridization buffer accommodated in the hybridization buffer tank 12a is fed into the cartridge 7 via the hybridization buffer feed pipe 11a and the solution circulation pipe 9.

When the inner space of the cartridge 7 and the solution circulation pipe 9 have been filled with the hybridization buffer, the change-over valve 14a provided in the hybridization buffer feed pipe 11a is located at its third position where communication between the hybridization buffer tank 12a and the atmosphere, and the solution circulation pipe 9 is shut off and the change-over valve 14b provided in the probe solution feed pipe 11b, the change-over valve 14c provided in the antibody solution feed pipe 11c and the change-over valve 14d provided in the cleaning solution feed pipe 11d are located at their third positions.

On the other hand, driving of the pump 10 in the forward direction is continued and as a result, the hybridization buffer filling the inner space of the cartridge 7 and the solution circulation pipe 9 is circulated through the cartridge 7 and the solution circulation pipe 9 in the direction indicated by an arrow A in Figure 3, whereby the hybridization buffer is forcibly moved through a number of the absorptive regions 4 of the biochemical analysis unit 1 accommodated in the cartridge 7.

As a result, pre-hybridization is performed.

When a first predetermined time period has passed, the pump 10 is once stopped and then driven in the reverse direction.

As a result, the hybridization buffer filling the inner space of the cartridge 7 and the solution circulation pipe 9 is circulated through the cartridge 7 and the solution circulation pipe 9 in the direction indicated by an arrow B in Figure 3, whereby the hybridization buffer is forcibly moved through a number of the absorptive regions 4 of the biochemical analysis unit 1 accommodated in the cartridge 7.

When a first predetermined time period has passed, the pump 10 is once stopped and then driven in the forward direction, thereby circulating the hybridization buffer filling the inner space of the cartridge 7 and the solution circulation pipe 9 through the cartridge 7 and the solution circulation pipe 9 in the direction indicated by the arrow A in Figure 3 and forcibly moving it through a number of the absorptive regions 4 of the biochemical analysis unit 1 accommodated in the cartridge 7.

In this manner, when the pump 10 has been driven alternately in the forward direction and the reverse direction a predetermined number of times, thereby forcibly feeding the hybridization buffer into the cartridge 7, the pump 10 is stopped and pre-hybridization is completed.

Then, a probe solution is prepared and accommodated in the probe solution chip 12b.

In the case where a specific binding substance such as cDNA is to be labeled with a radioactive labeling substance, a probe solution containing a substance derived from a living organism and labeled with a radioactive labeling substance as a probe is prepared and is accommodated in the probe solution chip 12b.

On the other hand, in the case where a specific binding substance such as cDNA is to be labeled with a fluorescent substance, a probe solution containing a substance derived from a living organism and labeled with a fluorescent substance as a probe is prepared and is accommodated in the probe solution chip 12b.

Further, in the case where a specific binding substance such as cDNA is to be labeled with a labeling substance which generates chemiluminescence emission when it contacts a chemiluminescent substrate, a probe solution containing a substance derived from a living organism and labeled with a hapten such as digoxigenin as a probe is prepared and is accommodated in the probe solution chip 12b.

It is possible to prepare a probe solution containing two or more substances derived from a living organism among a substance derived from a living organism and labeled with a radioactive labeling substance, a substance derived from a living organism and labeled with a fluorescent substance such as a fluorescent dye and a substance derived from a living organism and labeled with a hapten such as digoxigenin and accommodate it in the probe solution chip 12b. In this embodiment, a probe solution containing a substance derived from a living organism and labeled with a radioactive labeling substance, a substance derived from a living organism and labeled with a fluorescent substance such as a fluorescent dye and a substance derived from a living organism and labeled with a hapten such as digoxigenin is prepared and accommodated in the probe solution chip 12b.

When the probe solution has been accommodated in the probe solution chip 12b, the change-over valve 14a provided in the hybridization buffer feed pipe 11a, the change-over valve 14c provided in the antibody solution feed pipe 11c and the change-over valve 14d provided in the cleaning solution feed pipe 11d are located at their second positions where the atmosphere and the solution circulation pipe 9 communicate with each other and the change-over valve 14e provided at the connection between the solution circulation pipe 9 and the solution discharge pipe 13 is located at its first position.

Then, the change-over valve 14b provided in the probe solution feed pipe 11b is located at its first position where the probe solution feed pipe 11b and the solution circulation pipe 9 communicate with each other and the pump 10 is driven in the forward direction.

As a result, a probe solution accommodated in the probe solution chip 12b is fed into the cartridge 7 via the probe solution feed pipe 11b and the solution circulation pipe 9 and mixed with the hybridization buffer filling the inner space of the cartridge 7 and the solution circulation pipe 9.

When a predetermined amount of the probe solution has been fed into the cartridge 7, the change-over valve 14b provided in the probe solution feed pipe 11b is located at its third position where communication between the probe solution tank 12b and the atmosphere, and the solution circulation pipe 9 is shut off and the change-over valve 14a provided in the hybridization buffer feed pipe 11a, the change-over valve 14c provided in the antibody solution feed pipe 11c and the change-over valve 14d provided in the cleaning solution feed pipe 11d are located at their third positions.

On the other hand, the pump 10 continued to be driven in the forward direction and therefore, the mixed solution produced by mixing the probe solution with the hybridization buffer filling the inner space of the cartridge 7 and the solution circulation pipe 9 is circulated through the cartridge 7 and the solution circulation pipe 9 in the direction indicated by an arrow A in Figure 3, whereby the mixed solution of the hybridization buffer and the probe solution is forcibly moved through a number of the absorptive regions 4 of the biochemical analysis unit 1 accommodated in the cartridge 7.

As a result, a substance derived from a living organism and contained in the mixed solution of the hybridization buffer and the probe solution selectively hybridizes with specific binding substances absorbed in a number formed in the substrate 2 of the absorptive regions 4 of the biochemical analysis unit 1 accommodated in the cartridge 7.

When a second predetermined time period has passed, the pump 10 is once stopped and then driven in the reverse direction.

As a result, the mixed solution of the hybridization buffer and the probe solution filling the inner space of the cartridge 7 and the solution circulation pipe 9 is circulated through the cartridge 7 and the solution circulation pipe 9 in the direction indicated by an arrow B in Figure 3, whereby the mixed solution of the hybridization buffer the probe solution is forcibly moved through a number of the absorptive regions 4 of the biochemical analysis unit 1 accommodated in the cartridge 7.

When the second predetermined time period has passed, the pump 10 is once stopped and then driven in the forward direction, thereby circulating the mixed solution of the hybridization buffer and the probe solution filling the inner space of the cartridge 7 and the solution circulation pipe 9 through the cartridge 7 and the solution circulation pipe 9 in the direction indicated by the arrow A in Figure 3 and forcibly moving it through a number of the absorptive regions 4 of the biochemical analysis unit 1 accommodated in the cartridge 7.

In this manner, when the pump 10 has been driven alternately in the forward direction and the reverse direction a predetermined number of times, thereby forcibly feeding the mixed solution of the hybridization buffer and the probe solution into the cartridge 7, the pump 10 is stopped.

In this embodiment, since the mixed solution of the hybridization buffer and the probe solution is forcibly moved through each of a number the absorptive regions 4 formed in the substrate 2 of the biochemical analysis unit 1 accommodated in the cartridge 7 repeatedly in this manner, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in the mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Further, the change-over valve 14b provided in the probe solution feed pipe 11b is located at its second position where the atmosphere and the solution circulation pipe 9 communicate with each other and the change-over valve 14e provided at the connection between the solution circulation pipe 9 and the solution discharge pipe 13 is located at its second position where the solution circulation pipe 9 and the solution discharge pipe 13 communicate with each other. The pump 10 is then driven in the forward direction.

As a result, the mixed solution of the hybridization buffer and the probe solution filling the inner space of the cartridge 7 and the solution circulation pipe 9 is discharged through the solution discharge pipe 13.

When the mixed solution of the hybridization buffer and the probe solution filling the inner space of the cartridge 7 and the solution circulation pipe 9 has been discharged through the solution discharge pipe 13, the change-over valve 14e provided at the connection between the solution circulation pipe 9 and the solution discharge pipe 13 is located at its first position and the change-over valve 14a provided in the hybridization buffer feed pipe 11a, the change-over valve 14b provided in the probe solution feed pipe 11b and the change-over valve 14c provided in the antibody solution feed pipe 11c are located at their second positions where the atmosphere and the solution circulation pipe 9 communicate with each other.

The change-over valve 14d provided in the cleaning solution feed pipe 11d is then located at its first position where the cleaning solution feed pipe 11d and the solution circulation pipe 9 communicate with each other and the pump 10 is driven in the forward direction, thereby feeding a cleaning solution accommodated in the cleaning solution tank 12d into the cartridge 7 via the cleaning solution feed pipe 11d and the solution circulation pipe 9.

In this manner, when an inner space of the cartridge 7 and the solution circulation pipe 9 has been filled with the cleaning solution, the change-over valve 14a provided in the cleaning solution feed pipe 11d is located at its third position where communication between the cleaning solution tank 12d and the atmosphere, and the solution circulation pipe 9 is shut off and the change-over valve 14d provided in the hybridization buffer feed pipe 11a, the change-over valve 14b provided in the probe solution feed pipe 11b and the change-over valve 14c provided in the antibody solution feed pipe 11c are located at their third positions.

On the other hand, driving of the pump 10 in the forward direction is continued and as a consequence, the cleaning solution filling the inner space of the cartridge 7 and the solution circulation pipe 9 is circulated through the cartridge 7 and the solution circulation pipe 9 in the direction indicated by an arrow A in Figure 3, whereby the cleaning solution is forcibly moved through a number of the absorptive regions 4 of the biochemical analysis unit 1 accommodated in the cartridge 7.

As a result, a number of the absorptive regions 4 formed in the substrate 2 of the biochemical analysis unit 1 accommodated in the cartridge 7 are cleaned with the cleaning solution.

When a third predetermined time period has passed, the pump 10 is once stopped and then driven in the reverse direction.

As a result, the cleaning solution filling the inner space of the cartridge 7 and the solution circulation pipe 9 is circulated through the cartridge 7 and the solution circulation pipe 9 in the direction indicated by an arrow B in Figure 3, whereby the cleaning solution is forcibly moved through a number of the absorptive regions 4 of the biochemical analysis unit 1 accommodated in the cartridge 7.

When the third predetermined time period has passed, the pump 10 is once stopped and then driven in the forward direction, thereby circulating the cleaning solution filling the inner space of the cartridge 7 and the solution circulation pipe 9 through the cartridge 7 and the solution circulation pipe 9 in the direction indicated by the arrow A in Figure 3 and forcibly moving it through a number of the absorptive regions 4 of the biochemical analysis unit 1 accommodated in the cartridge 7.

In this manner, when the pump 10 has been driven alternately in the forward direction and the reverse direction a predetermined number of times, thereby forcibly feeding the cleaning solution into the cartridge 7, the pump 10 is stopped.

In this embodiment, since the cleaning solution is forcibly moved through a number the absorptive regions 4 formed in the substrate 2 of the biochemical analysis unit 1 accommodated in the cartridge 7 repeatedly in this manner, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1 and the efficiency of cleaning operation can be markedly improved.

Further, the change-over valve 14d provided in the cleaning solution feed pipe 11d is located at its second position where the atmosphere and the solution circulation pipe 9 communicate with each other and the change-over valve 14e provided at the connection between the solution circulation pipe 9 and the solution discharge pipe 13 is located at its second position where the solution circulation pipe 9 and the solution discharge pipe 13 communicate with each other. The pump 10 is then driven in the forward direction.

As a result, the cleaning solution filling the inner space of the cartridge 7 and the solution circulation pipe 9 is discharged through the solution discharge pipe 13.

In this manner, radiation data of a radioactive labeling substance and a fluorescence data of a fluorescent substance such as a fluorescent dye are recorded in a number of the absorptive regions 4 formed in the substrate 2 of the biochemical analysis unit 1.

The fluorescence data recorded in a number of the absorptive regions 4 of the biochemical analysis unit 1 are read by a scanner described later and biochemical analysis data are produced.

On the other hand, radiation data recorded in a number of the absorptive regions 4 of the biochemical analysis unit 1 are transferred onto a stimulable phosphor sheet described later and read by a scanner described later, thereby producing biochemical analysis data.

To the contrary, in order to record chemiluminescence data in a number of the absorptive regions 4 formed in the substrate 2 of the biochemical analysis unit 1, an antibody solution containing an antibody to the hapten such as digoxigenin labeled with an enzyme which generates chemiluminescence emission when it contacts a chemiluminescent substrate is further prepared and accommodated in the antibody solution tank 12c and the antibody to the hapten such as digoxigenin labeled with an enzyme which generates chemiluminescence emission when it contacts a chemiluminescent substrate is bonded with the hapten such as digoxigenin labeling a substance derived from a living organism selectively hybridized with specific binding substances absorbed in a number of the absorptive regions formed in the substrate 2 of the biochemical analysis unit 1 by the an antigen-antibody reaction.

Specifically, an antibody solution containing an antibody to the hapten such as digoxigenin labeled with an enzyme which generates chemiluminescence emission when it contacts a chemiluminescent substrate is first prepared and accommodated in the antibody solution tank 12c.

When the antibody solution has been accommodated in the antibody solution tank 12c, the change-over valve 14e provided at the connection between the solution circulation pipe 9 and the solution discharge pipe 13 is located at its first position and the change-over valve 14a provided in the hybridization buffer feed pipe 11a, the change-over valve 14b provided in the probe solution feed pipe 11b and the change-over valve 14d provided in the cleaning solution feed pipe 11d are located at their second positions where the atmosphere and the solution circulation pipe 9 communicate with each other.

The change-over valve 14c provided in the antibody solution feed pipe 11c is then located at its first position where the antibody solution feed pipe 11c and the solution circulation pipe 9 communicate with each other and the pump 10 is driven in the forward direction, thereby feeding a antibody solution accommodated in the cleaning solution tank 12d into the cartridge 7 via the antibody solution feed pipe 11c and the solution circulation pipe 9.

In this manner, when an inner space of the cartridge 7 and the solution circulation pipe 9 has been filled with the antibody solution, the change-over valve 14c provided in the antibody solution feed pipe 11c is located at its third position where communication between the antibody solution tank 12c and the atmosphere, and the solution circulation pipe 9 is shut off and the change-over valve 14a provided in the hybridization buffer feed pipe 11a, the change-over valve 14b provided in the probe solution feed pipe 11b and the change-over valve 14d provided in the cleaning solution feed pipe 11d are located at their third positions.

On the other hand, the pump 10 continued to be driven in the forward direction and therefore, the antibody solution filling the inner space of the cartridge 7 and the solution circulation pipe 9 is circulated through the cartridge 7 and the solution circulation pipe 9 in the direction indicated by the arrow A in Figure 3, whereby the antibody solution is forcibly moved through a number of the absorptive regions 4 of the biochemical analysis unit 1 accommodated in the cartridge 7.

As a result, the antibody to the hapten such as digoxigenin labeled with an enzyme which generates chemiluminescence emission when it contacts a chemiluminescent substrate is bonded with the hapten such as digoxigenin labeling a substance derived from a living organism selectively hybridized with specific binding substances absorbed in a number of the absorptive regions formed in the substrate 2 of the biochemical analysis unit 1 by the an antigen-antibody reaction.

When a fourth predetermined time period has passed, the pump 10 is once stopped and then driven in the reverse direction.

As a consequence, the antibody solution filling the inner space of the cartridge 7 and the solution circulation pipe 9 is circulated through the cartridge 7 and the solution circulation pipe 9 in the direction indicated by an arrow B in Figure 3, whereby the antibody solution is forcibly moved through a number of the absorptive regions 4 of the biochemical analysis unit 1 accommodated in the cartridge 7.

As a result, the antibody to the hapten such as digoxigenin labeled with an enzyme which generates chemiluminescence emission when it contacts a chemiluminescent substrate is bonded with the hapten such as digoxigenin labeling a substance derived from a living organism selectively hybridized with specific binding substances absorbed in a number of the absorptive regions formed in the substrate 2 of the biochemical analysis unit 1 by the an antigen-antibody reaction.

When the fourth predetermined time period has passed, the pump 10 is once stopped and then driven in the forward direction, thereby circulating the antibody solution filling the inner space of the cartridge 7 and the solution circulation pipe 9 through the cartridge 7 and the solution circulation pipe 9 in the direction indicated by the arrow A in Figure 3 and forcibly moving it through a number of the absorptive regions 4 of the biochemical analysis unit 1 accommodated in the cartridge 7.

In this manner, when the pump 10 has been driven alternately in the forward direction and the reverse direction for predetermined times, thereby forcibly feeding the antibody solution into the cartridge 7, the pump 10 is stopped.

In this embodiment, since the antibody solution is forcibly moved through a number the absorptive regions formed in the substrate 2 of the biochemical analysis unit repeatedly in this manner, it is possible to markedly increase the moving rate of an antibody through the absorptive regions 4 of the biochemical analysis unit 1 and, therefore, the reaction rate of an antigen-antibody reaction can be markedly improved. Further, since it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Further, the change-over valve 14c provided in the antibody solution feed pipe 11c is located at its second position where the atmosphere and the solution circulation pipe 9 communicate with each other and the change-over valve 14e provided at the connection between the solution circulation pipe 9 and the solution discharge pipe 13 is located at its second position where the solution circulation pipe 9 and the solution discharge pipe 13 communicate with each other. The pump 10 is then driven in the forward direction.

As a result, the antibody solution filling the inner space of the cartridge 7 and the solution circulation pipe 9 is discharged through the solution discharge pipe 13.

When the antibody solution filling the inner space of the cartridge 7 and the solution circulation pipe 9 has been discharged through the solution discharge pipe 13, the change-over valve 14e provided at the connection between the solution circulation pipe 9 and the solution discharge pipe 13 is located at its first position and the change-over valve 14a provided in the hybridization buffer feed pipe 11a, the change-over valve 14b provided in the probe solution feed pipe 11b and the change-over valve 14c provided in the antibody solution feed pipe 11c are located at their second positions where the atmosphere and the solution circulation pipe 9 communicate with each other.

The change-over valve 14d provided in the cleaning solution feed pipe 11d is then located at its first position where the cleaning solution feed pipe 11d and the solution circulation pipe 9 communicate with each other and the pump 10 is driven in the forward direction, thereby feeding a cleaning solution accommodated in the cleaning solution tank 12d into the cartridge 7 via the cleaning solution feed pipe 11d and the solution circulation pipe 9.

In this manner, when an inner space of the cartridge 7 and the solution circulation pipe 9 has been filled with the cleaning solution, the change-over valve 14a provided in the cleaning solution feed pipe 11d is located at its third position where communication between the cleaning solution tank 12d and the atmosphere, and the solution circulation pipe 9 is shut off and the change-over valve 14d provided in the hybridization buffer feed pipe 11a, the change-over valve 14b provided in the probe solution feed pipe 11b and the change-over valve 14c provided in the antibody solution feed pipe 11c are located at their third positions.

On the other hand, driving of the pump 10 in the forward direction is continued and as a consequence, the cleaning solution filling the inner space of the cartridge 7 and the solution circulation pipe 9 is circulated through the cartridge 7 and the solution circulation pipe 9 in the direction indicated by an arrow A in Figure 3, whereby the cleaning solution is forcibly moved through a number of the absorptive regions 4 of the biochemical analysis unit 1 accommodated in the cartridge 7.

As a result, a number of the absorptive regions 4 formed in the substrate 2 of the biochemical analysis unit 1 accommodated in the cartridge 7 are cleaned with the cleaning solution.

When a fifth predetermined time period has passed, the pump 10 is once stopped and then driven in the reverse direction.

As a result, the cleaning solution filling the inner space of the cartridge 7 and the solution circulation pipe 9 is circulated through the cartridge 7 and the solution circulation pipe 9 in the direction indicated by an arrow B in Figure 3, whereby the cleaning solution is forcibly moved through a number of the absorptive regions 4 of the biochemical analysis unit 1 accommodated in the cartridge 7.

When the fifth predetermined time period has passed, the pump 10 is once stopped and then driven in the forward direction, thereby circulating the cleaning solution filling the inner space of the cartridge 7 and the solution circulation pipe 9 through the cartridge 7 and the solution circulation pipe 9 in the direction indicated by the arrow A in Figure 3 and forcibly moving it through a number of the absorptive regions 4 of the biochemical analysis unit 1 accommodated in the cartridge 7.

In this manner, when the pump 10 has been driven alternately in the forward direction and the reverse direction a predetermined number of times, thereby forcibly feeding the cleaning solution into the cartridge 7, the pump 10 is stopped.

In this embodiment, since the cleaning solution is forcibly moved through a number the absorptive regions 4 formed in the substrate 2 of the biochemical analysis unit 1 accommodated in the cartridge 7 repeatedly in this manner, even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1 and the efficiency of cleaning operation can be markedly improved.

Further, the change-over valve 14d provided in the cleaning solution feed pipe 11d is located at its second position where the atmosphere and the solution circulation pipe 9 communicate with each other and the change-over valve 14e provided at the connection between the solution circulation pipe 9 and the solution discharge pipe 13 is located at its second position where the solution circulation pipe 9 and the solution discharge pipe 13 communicate with each other. The pump 10 is then driven in the forward direction.

As a result, the cleaning solution filling the inner space of the cartridge 7 and the solution circulation pipe 9 is discharged through the solution discharge pipe 13.

Chemiluminescence data are recorded in a number of the absorptive regions 4 formed in the substrate 2 of the biochemical analysis unit 1 in the foregoing manner.

Chemiluminescence data recorded in a number of the absorptive regions 4 of the biochemical analysis unit 1 are read by a cooled CCD camera of a data producing system described later or transferred onto a stimulable phosphor sheet described later and read by a scanner described later, thereby producing biochemical analysis data.

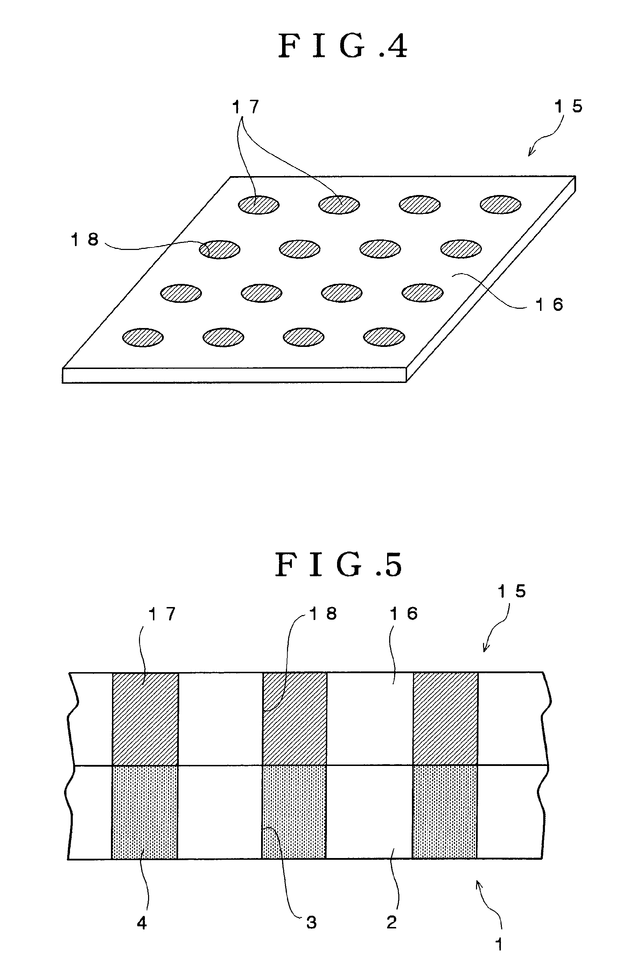

Figure 4 is a schematic perspective view showing a stimulable phosphor sheet onto which radiation data are to be transferred.

As shown in Figure 4, a stimulable phosphor sheet 15 according to this embodiment includes a support 16 made of stainless steel and regularly formed with a number of substantially circular through-holes 18 and a number of stimulable phosphor layer regions 17 are dot-like formed by charging BaFX system stimulable phosphor (where X is at least one halogen atom selected from the group consisting of Cl, Br and I) capable of absorbing and storing radiation energy in the through-holes 18.

A number of the through-holes 18 are formed in the support 16 in the same pattern as that of a number of the absorptive regions 4 formed in the substrate 2 of the biochemical analysis unit 1 and each of them has the same size as that of the absorptive region 4 formed in the substrate 2 of the biochemical analysis unit 1.

Therefore, although not accurately shown in Figure 4, in this embodiment, about 10,000 substantially circular stimulable phosphor layer regions 17 having a size of about 0.01 mm² are dot-like formed in a regular pattern at a density of about 5,000 per cm² in the support 16 of the stimulable phosphor sheet 15.

In this embodiment, stimulable phosphor is charged in a number of the through-holes 18 formed in the support 16 in such a manner that the surfaces of the stimulable phosphor layer regions 17 lie at the same height level of that of the surface of the support 16.

Figure 5 is a schematic cross-sectional view showing a method for exposing a number of the stimulable phosphor layer regions 17 formed in the stimulable phosphor sheet 15 by a radioactive labeling substance contained in a number of the absorptive regions 4 formed in the biochemical analysis unit 1.

As shown in Figure 5, when the stimulable phosphor layer regions 17 of a stimulable phosphor sheet 15 are to be exposed, the stimulable phosphor sheet 15 is superposed on the biochemical analysis unit 1 in such a manner that each of the stimulable phosphor layer regions 17 formed in the support 16 of the stimulable phosphor sheet 15 faces the corresponding absorptive regions 4 formed in the substrate 2 of the biochemical analysis unit 1.

In this embodiment, since the biochemical analysis unit 1 is formed by charging nylon-6 in a number of the through-holes 3 formed in the substrate 2 made of stainless steel, the biochemical analysis unit 1 does not stretch or shrink even when it is subjected to liquid processing such as hybridization and, therefore, it is possible to easily and accurately superpose the stimulable phosphor sheet 15 on the biochemical analysis unit 1 so that each of the stimulable phosphor layer regions 17 formed in the support 16 of the stimulable phosphor sheet 15 accurately faces the corresponding absorptive region 4 formed in the substrate 2 of the biochemical analysis unit 1, thereby exposing the stimulable phosphor layer regions 17.

In this manner, each of the stimulable phosphor layer regions 17 formed in the support 16 of the stimulable phosphor sheet 15 is kept to face the corresponding absorptive region 4 formed in the substrate 2 of the biochemical analysis unit 1 for a predetermined time period, whereby a number of the stimulable phosphor layer regions 17 formed in the support 16 of the stimulable phosphor sheet 15 are exposed to the radioactive labeling substance contained in a number of the absorptive regions 4 formed in the substrate 2 of the biochemical analysis unit 1.

During the exposure operation, electron beams (β rays) are released from the radioactive labeling substance contained in the absorptive regions 4 of the biochemical analysis unit 1. However, since a number of the absorptive regions 4 of the biochemical analysis unit 1 are formed spaced apart from each other in the substrate 2 made of stainless steel and the substrate 2 made of stainless steel capable of attenuating radiation energy is present around each of the absorptive regions 4, electron beams (β rays) released from the radioactive labeling substance contained in the absorptive regions 4 of the biochemical analysis unit 1 can be efficiently prevented from scattering in the substrate 2 of the biochemical analysis unit 1. Further, since a number of the stimulable phosphor layer regions 17 of the stimulable phosphor sheet 15 are formed by charging stimulable phosphor in a number of the through-holes 18 formed in the support 16 made of stainless steel capable of attenuating radiation energy and the support 16 made of stainless steel is present around each of the stimulable phosphor layer regions 17, electron beams (β rays) released from the radioactive labeling substance contained in the absorptive regions 4 of the biochemical analysis unit 1 can be efficiently prevented from scattering in the support 16 of the stimulable phosphor sheet 15. Therefore, it is possible to cause all electron beams (β rays) released from the radioactive labeling substance contained in the absorptive region 4 to enter the stimulable phosphor layer region 17 the absorptive region 4 faces and to effectively prevent electron beams (β rays) released from the absorptive region 4 from entering stimulable phosphor layer regions 17 to be exposed to electron beams (β rays) released from neighboring absorptive regions 4.

In this manner, a number of the stimulable phosphor layer regions 17 formed in the support 16 of the stimulable phosphor sheet 15 can be selectively exposed to a radioactive labeling substance contained in the corresponding absorptive region 4 of the biochemical analysis unit 1.

Thus, radiation data of a radioactive labeling substance are recorded in a number of the stimulable phosphor layer regions 17 formed in the support 16 of the stimulable phosphor sheet 15.

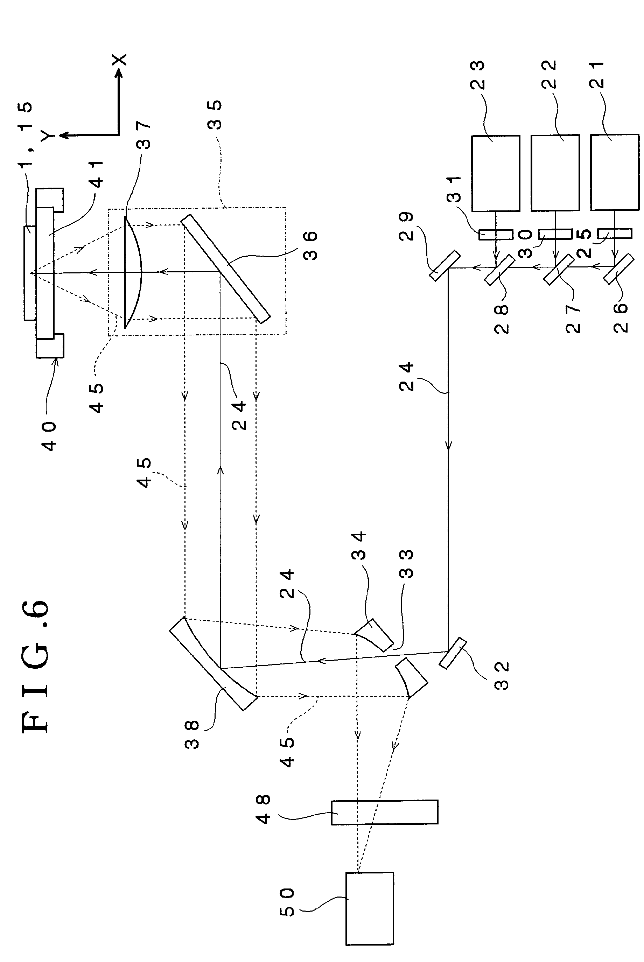

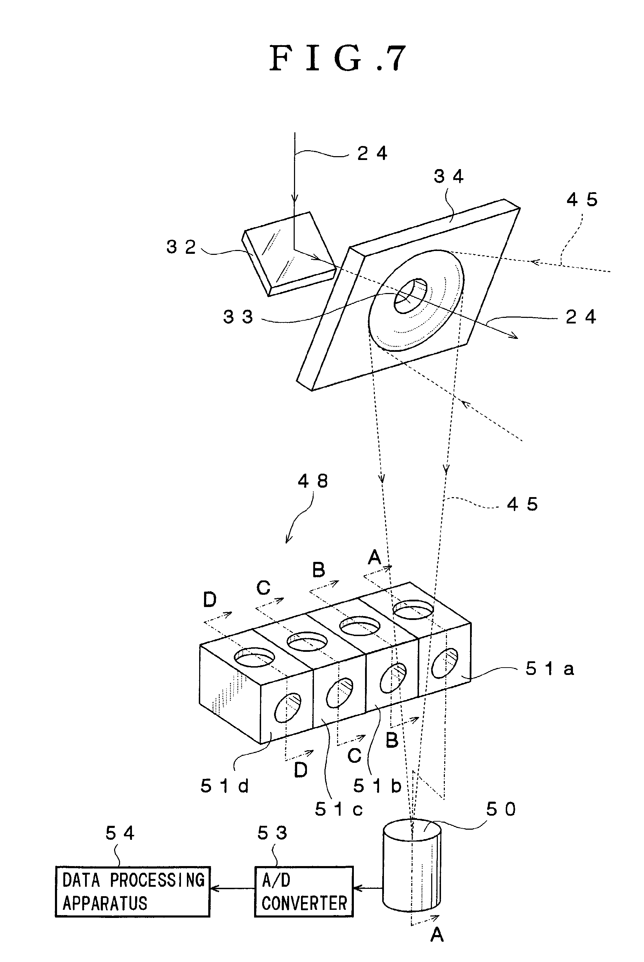

Figure 6 is a schematic view showing a scanner for reading radiation data recorded in a number of the stimulable phosphor layer regions 17 formed in the support 16 of the stimulable phosphor sheet 15 to produce biochemical analysis data and Figure 7 is a schematic perspective view showing details in the vicinity of a photomultiplier of a scanner shown in Figure 6.

The scanner shown in Figures 6 and 7 is constituted so as to read radiation data recorded in a number of the stimulable phosphor layer regions 17 formed in the support 16 of the stimulable phosphor sheet 15 and fluorescence data of a fluorescent substance such as a fluorescent dye recorded in a number of the absorptive regions 4 formed in the substrate 2 of the biochemical analysis unit 1 to produce biochemical analysis data.

As shown in Figure 6, the scanner includes a first laser stimulating ray source 21 for emitting a laser beam having a wavelength of 640 nm, a second laser stimulating ray source 22 for emitting a laser beam having a wavelength of 532 nm and a third laser stimulating ray source 23 for emitting a laser beam having a wavelength of 473 nm.

In this embodiment, the first laser stimulating ray source 21 is constituted by a semiconductor laser beam source and the second laser stimulating ray source 22 and the third laser stimulating ray source 23 are constituted by a second harmonic generation element.

A laser beam 24 emitted from the first laser stimulating source 21 passes through a collimator lens 25, thereby being made a parallel beam, and is reflected by a mirror 26. A first dichroic mirror 27 for transmitting light having a wavelength of 640 nm but reflecting light having a wavelength of 532 nm and a second dichroic mirror 28 for transmitting light having a wavelength equal to and longer than 532 nm but reflecting light having a wavelength of 473 nm are provided in the optical path of the laser beam 24 emitted from the first laser stimulating ray source 21. The laser beam 24 emitted from the first laser stimulating ray source 21 and reflected by the mirror 26 passes through the first dichroic mirror 27 and the second dichroic mirror 28 and advances to a mirror 29.

On the other hand, the laser beam 24 emitted from the second laser stimulating ray source 22 passes through a collimator lens 30, thereby being made a parallel beam, and is reflected by the first dichroic mirror 27, thereby changing its direction by 90 degrees. The laser beam 24 then passes through the second dichroic mirror 28 and advances to the mirror 29.

Further, the laser beam 24 emitted from the third laser stimulating ray source 23 passes through a collimator lens 31, thereby being made a parallel beam, and is reflected by the second dichroic mirror 28, thereby changing its direction by 90 degrees. The laser beam 24 then advances to the mirror 29.

The laser beam 24 advancing to the mirror 29 is reflected by the mirror 29 and advances to a mirror 32 to be reflected thereby.

A perforated mirror 34 formed with a hole 33 at the center portion thereof is provided in the optical path of the laser beam 24 reflected by the mirror 32. The laser beam 24 reflected by the mirror 32 passes through the hole 33 of the perforated mirror 34 and advances to a concave mirror 38.

The laser beam 24 advancing to the concave mirror 38 is reflected by the concave mirror 38 and enters an optical head 35.

The optical head 35 includes a mirror 36 and an aspherical lens 37. The laser beam 24 entering the optical head 35 is reflected by the mirror 36 and condensed by the aspherical lens 37 onto the stimulable phosphor sheet 15 or the biochemical analysis unit 1 placed on the glass plate 41 of a stage 40.

When the laser beam 24 impinges on one of the stimulable phosphor layer regions 17 formed in the support 16 of the stimulable phosphor sheet 15, stimulable phosphor contained in the stimulable phosphor layer region 17 is excited, thereby releasing stimulated emission 45. On the other hand, when the laser beam 24 impinges on one of the absorptive regions 4 formed in the substrate 2 of the biochemical analysis unit 1, a fluorescent dye or the like contained in the absorptive region 4 is excited, thereby releasing fluorescence emission 45.

The stimulated emission 45 released from the stimulable phosphor layer region 17 formed in the support 16 of the stimulable phosphor 15 or the fluorescence emission 45 released from the absorptive region 4 formed in the substrate 2 of the biochemical analysis unit 1 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 or the fluorescence emission 45 advancing to the concave mirror 38 is reflected by the concave mirror 38 and advances to the perforated mirror 34.

As shown in Figure 7, the stimulated emission 45 or the fluorescence emission 45 advancing to the perforated mirror 34 is reflected downward by the perforated mirror 34 formed as a concave mirror and advances to a filter unit 48, whereby light having a predetermined wavelength is cut. The stimulated emission 45 or the fluorescence emission 45 then impinges on a photomultiplier 50, thereby being photoelectrically detected.

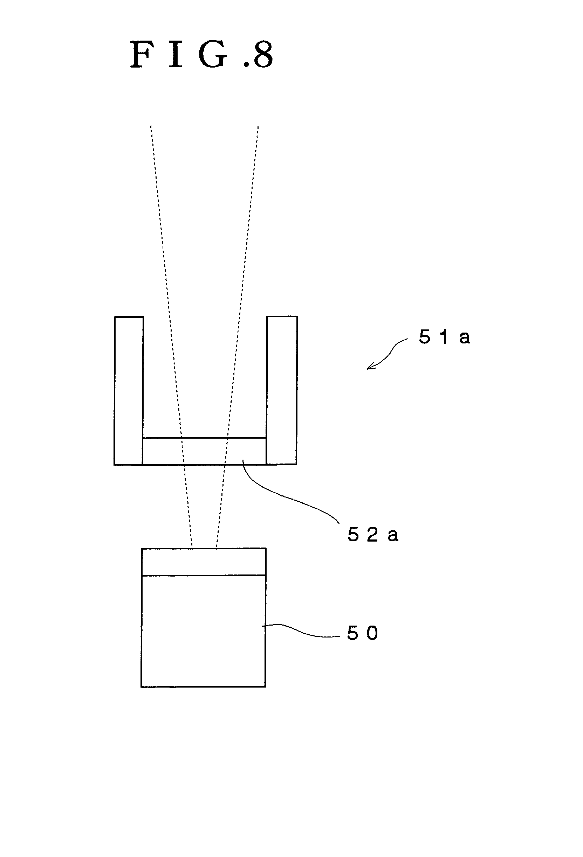

As shown in Figure 7, the filter unit 48 is provided with four filter members 51a, 51b, 51c and 51d and is constituted to be laterally movable in Figure 8 by a motor (not shown).

Figure 8 is a schematic cross-sectional view taken along a line A-A in Figure 7.

As shown in Figure 8, the filter member 51a includes a filter 52a and the filter 52a is used for reading fluorescence emission 45 by stimulating a fluorescent substance such as a fluorescent dye contained in a number of the absorptive regions 4 formed in the substrate 2 of the biochemical analysis unit 1 using the first laser stimulating ray source 21 and has a property of cutting off light having a wavelength of 640 nm but transmitting light having a wavelength longer than 640 nm.

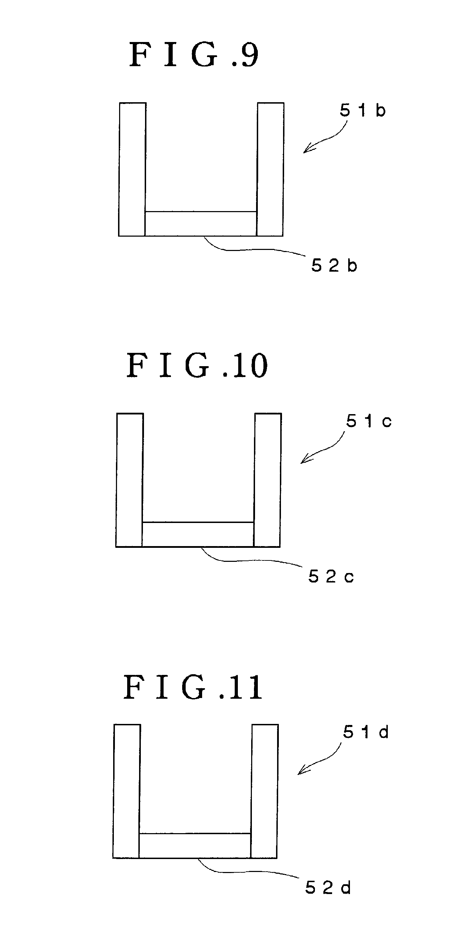

Figure 9 is a schematic cross-sectional view taken along a line B-B in Figure 7.

As shown in Figure 9, the filter member 51b includes a filter 52b and the filter 52b is used for reading fluorescence emission 45 by stimulating a fluorescent substance such as a fluorescent dye contained in a number of the absorptive regions 4 formed in the substrate 2 of the biochemical analysis unit 1 using the second laser stimulating ray source 22 and has a property of cutting off light having a wavelength of 532 nm but transmitting light having a wavelength longer than 532 nm.

Figure 10 is a schematic cross-sectional view taken along a line C-C in Figure 7.

As shown in Figure 10, the filter member 51c includes a filter 52c and the filter 52c is used for reading fluorescence emission 45 by stimulating a fluorescent substance such as a fluorescent dye contained in in a number of the absorptive regions 4 formed in the substrate 2 of the biochemical analysis unit 1 using the third laser stimulating ray source 23 and has a property of cutting off light having a wavelength of 473 nm but transmitting light having a wavelength longer than 473 nm.

Figure 11 is a schematic cross-sectional view taken along a line D-D in Figure 7.

As shown in Figure 11, the filter member 51d includes a filter 52d and the filter 52d is used for reading stimulated emission 45 released from stimulable phosphor contained in the stimulable phosphor layer 17 formed in the support 16 of the stimulable phosphor sheet 15 upon being stimulated using the first laser stimulating ray source 1 and has a property of transmitting only light having a wavelength corresponding to that of stimulated emission 45 emitted from stimulable phosphor and cutting off light having a wavelength of 640 nm.

Therefore, in accordance with the kind of stimulating ray source to be used, one of these filter members 51a, 51b, 51c, 51d is selectively positioned in front of the photomultiplier 50, thereby enabling the photomultiplier 50 to photoelectrically detect only light to be detected.

The analog data produced by photoelectrically detecting stimulated emission 45 or fluorescence emission 45 with the photomultiplier 50 are converted by an A/D converter 53 into digital data and the digital data are fed to a data processing apparatus 54.

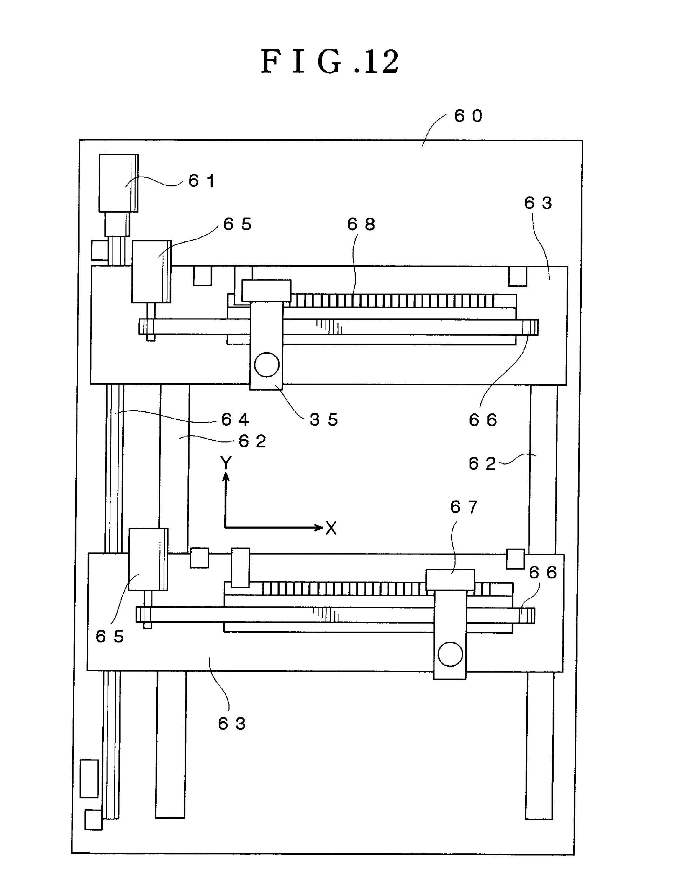

Figure 12 is a schematic plan view showing the scanning mechanism of the optical head 35.

In Figure 12, optical systems other than the optical head 35 and the paths of the laser beam 24 and stimulated emission 45 or fluorescence emission 45 are omitted for simplification.

As shown in Figure 12, the scanning mechanism of the optical head 35 includes a base plate 60, and a sub-scanning pulse motor 61 and a pair of rails 62, 62 are fixed on the base plate 60. A movable base plate 63 is further provided so as to be movable in the sub-scanning direction indicated by an arrow Y in Figure 12.

The movable base plate 63 is formed with a threaded hole (not shown) and a threaded rod 64 rotated by the sub-scanning pulse motor 61 is engaged with the inside of the hole.

A main scanning stepping motor 65 is provided on the movable base plate 63. The main scanning stepping motor 65 is adapted for intermittently driving an endless belt 66 at a pitch equal to the distance between neighboring absorptive regions 4 formed in the substrate 2 of the biochemical analysis unit 1, namely, the distance between neighboring stimulable phosphor layer regions 17 formed in the support 16 of the stimulable phosphor sheet 15.

The optical head 35 is fixed to the endless belt 66 and when the endless belt 66 is driven by the main scanning stepping motor 65, the optical head 35 is moved in the main scanning direction indicated by an arrow X in Figure 12.

In Figure 12, the reference numeral 67 designates a linear encoder for detecting the position of the optical head 35 in the main scanning direction and the reference numeral 68 designates slits of the linear encoder 67.

Therefore, the optical head 35 is moved in the main scanning direction indicated by the arrow X and the sub-scanning direction indicated by the arrow Y in Figure 12 by driving the endless belt 66 in the main scanning direction by the main scanning stepping motor 65 and intermittently moving the movable base plate 63 in the sub-scanning direction by the sub-scanning pulse motor 61, thereby scanning all of the stimulable phosphor layer regions 17 formed in the support 16 of the stimulable phosphor sheet 15 or all of the absorptive regions 4 formed in the substrate 2 of the biochemical analysis unit 1 with the laser beam 24.

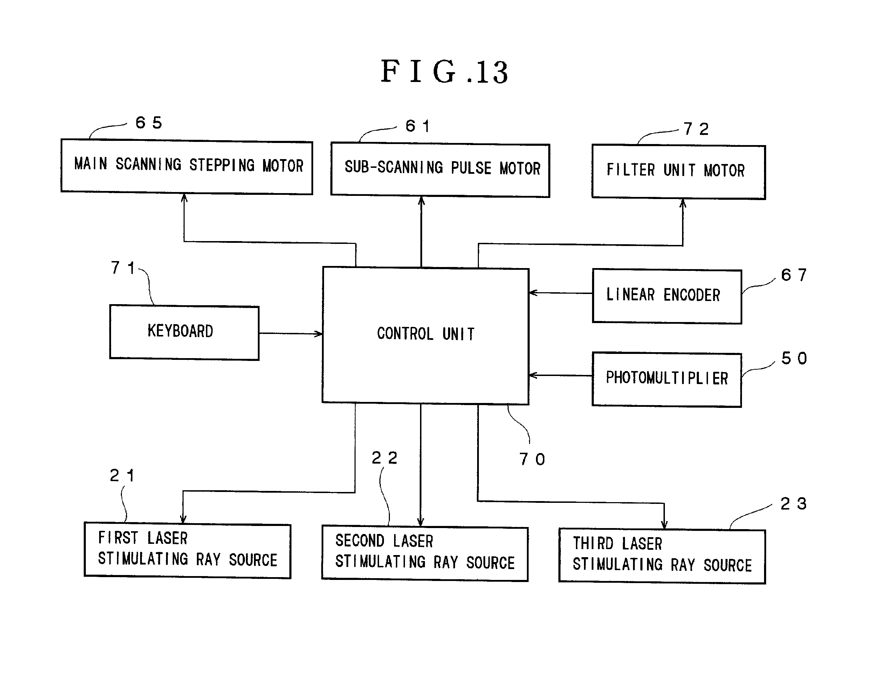

Figure 13 is a block diagram of a control system, an input system, a drive system and a detection system of the scanner shown in Figure 6.

As shown in Figure 13, the control system of the scanner includes a control unit 70 for controlling the overall operation of the scanner and the input system of the scanner includes a keyboard 71 which can be operated by a user and through which various instruction signals can be input.

As shown in Figure 13, the drive system of the scanner includes the main scanning stepping motor 65 for intermittently moving the optical head 35 in the main scanning direction, the sub-scanning pulse motor 61 for moving the optical head 35 in the sub-scanning direction and a filter unit motor 72 for moving the filter unit 48 provided with the four filter members 51a, 51b, 51c and 51d.

The control unit 70 is adapted for selectively outputting a drive signal to the first laser stimulating ray source 21, the second laser stimulating ray source 22 or the third laser stimulating ray source 23 and outputting a drive signal to the filter unit motor 72.

As shown in Figure 13, the detection system of the scanner includes the photomultiplier 50 and the linear encoder 67 for detecting the position of the optical head 35 in the main scanning direction.

In this embodiment, the control unit 70 is adapted to control the on and off operation of the first laser stimulating ray source 21, the second laser stimulating ray source 22 or the third laser stimulating ray source 23 in accordance with a detection signal indicating the position of the optical head 35 input from the linear encoder 67.

The thus constituted scanner reads radiation data recorded in a number of the stimulable phosphor layer regions 17 formed in the support 16 of the stimulable phosphor sheet 15 and produces biochemical analysis data in the following manner.

A stimulable phosphor sheet 15 is first set on the glass plate 41 of the stage 40 by a user.

An instruction signal indicating that radiation data recorded in the stimulable phosphor layer region 17 formed in the support 16 of the stimulable phosphor sheet 15 are to be read is then input through the keyboard 71.

The instruction signal input through the keyboard 71 is input to the control unit 70 and the control unit 70 outputs a drive signal to the filter unit motor 72 in accordance with the instruction signal, thereby moving the filter unit 48 so as to locate the filter member 51d provided with the filter 52d having a property of transmitting only light having a wavelength corresponding to that of stimulated emission emitted from stimulable phosphor but cutting off light having a wavelength of 640 nm in the optical path of stimulated emission 45.

The control unit 70 further outputs a drive signal to the main scanning stepping motor 65 to move the optical head 35 in the main scanning direction and when it determines based on a detection signal indicating the position of the optical head 35 input from the linear encoder 67 that the optical head 35 has reached a position where a laser beam 24 can be projected onto a first stimulable phosphor layer region 17 among a number of the stimulable phosphor layer regions 17 formed in the support 16 of the stimulable phosphor sheet 15, it outputs a drive stop signal to the main scanning stepping motor 65 and a drive signal to the first stimulating ray source 21, thereby actuating it to emit a laser beam 24 having a wavelength of 640 nm.

A laser beam 24 emitted from the first laser stimulating source 21 passes through the collimator lens 25, thereby being made a parallel beam, and is reflected by the mirror 26.

The laser beam 24 reflected by the mirror 26 passes through the first dichroic mirror 27 and the second dichroic mirror 28 and advances to the mirror 29.

The laser beam 24 advancing to the mirror 29 is reflected by the mirror 29 and advances to the mirror 32 to be reflected thereby.

The laser beam 24 reflected by the mirror 32 passes through the hole 33 of the perforated mirror 34 and advances to the concave mirror 38.

The laser beam 24 advancing to the concave mirror 38 is reflected by the concave mirror 38 and enters the optical head 35.

The laser beam 24 entering the optical head 35 is reflected by the mirror 36 and condensed by the aspherical lens 37 onto the first stimulable phosphor layer region 17 of the stimulable phosphor sheet 15 placed on the glass plate 41 of a stage 40.

In this embodiment, since the stimulable phosphor layer regions 17 are formed by charging stimulable phosphor in a number of the through-holes 18 formed in the support 16 made of stainless steel capable of attenuating light energy, it is possible to effectively prevent the laser beam 24 from scattering in each of the stimulable phosphor layer regions 17 and entering the neighboring stimulable phosphor layer regions 17 to excite stimulable phosphor contained in the neighboring stimulable phosphor layer regions 17.

When the laser beam 24 impinges onto the first stimulable phosphor layer region 17 formed in the support 16 of the stimulable phosphor sheet 15, stimulable phosphor contained in the first stimulable phosphor layer region 17 is excited by the laser beam 24, thereby releasing stimulated emission 45 from the first stimulable phosphor layer region 17.

The stimulated emission 45 released from the first stimulable phosphor layer region 17 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 advancing to the concave mirror 38 is reflected by the concave mirror 38 and advances to the perforated mirror 34.

As shown in Figure 7, the stimulated emission 45 advancing to the perforated mirror 34 is reflected downward by the perforated mirror 34 formed as a concave mirror and advances to the filter 52d of the filter unit 48.

Since the filter 52d has a property of transmitting only light having a wavelength corresponding to that of stimulated emission emitted from stimulable phosphor and cutting off light having a wavelength of 640 nm, light having a wavelength of 640 nm corresponding to that of the stimulating ray is cut off by the filter 52d and only light having a wavelength corresponding to that of stimulated emission released from the first stimulable phosphor layer region 17 passes through the filter 52d to be photoelectrically detected by the photomultiplier 50.

Analog data produced by photoelectrically detecting stimulated emission 45 with the photomultiplier 50 are converted by the A/D converter 53 into digital data and the digital data are fed to the data processing apparatus 54.

When a predetermined time, for example, several microseconds, has passed after the first stimulating ray source 21 was turned on, the control unit 70 outputs a drive stop signal to the first stimulating ray source 21, thereby turning it off and outputs a drive signal to the main scanning stepping motor 65, thereby moving the optical head 35 by one pitch equal to the distance between neighboring stimulable phosphor layer regions 17 formed in the stimulable phosphor sheet 15.

When the control unit 70 determines based on a detection signal indicating the position of the optical head 35 input from the linear encoder 67 that the optical head 35 has been moved by one pitch equal to the distance between neighboring stimulable phosphor layer regions 17 and has reached a position where a laser beam 24 can be projected onto a second stimulable phosphor layer region 17 next to the first stimulable phosphor layer region 17 formed in the support 16 of the stimulable phosphor sheet 10, it outputs a drive signal to the first stimulating ray source 21 to turn it on, thereby causing the laser beam 24 to excite stimulable phosphor contained in the second stimulable phosphor layer region 17 formed in the support 16 of the stimulable phosphor sheet 10 next to the first stimulable phosphor layer region 17.

Similarly to the above, the second stimulable phosphor layer region 17 formed in the support 16 of the stimulable phosphor sheet 15 is irradiated with the laser beam 24 emitted from the first laser stimulating ray source 21 for a predetermined time and when biochemical analysis data have been produced from radiation data recorded in the second stimulable phosphor layer region 17 by photoelectrically detecting stimulated emission 45 released from the second stimulable phosphor layer region 17 in response to the excitation of stimulable phosphor with the photomultiplier 50 to produce analog data and digitizing the analog data by the A/D converter 53, the control unit 70 outputs a drive stop signal to the first stimulating ray source 21, thereby turning it off and outputs a drive signal to the main scanning stepping motor 65, thereby moving the optical head 35 by one pitch equal to the distance between neighboring stimulable phosphor layer regions 17.

In this manner, the on and off operation of the first stimulating ray source 21 is repeated in synchronism with the intermittent movement of the optical head 35 and when the control unit 70 determines based on a detection signal indicating the position of the optical head 35 input from the linear encoder 67 that the optical head 35 has been moved by one scanning line in the main scanning direction and that the stimulable phosphor layer regions 17 included in a first line of the stimulable phosphor layer regions 17 formed in the support 16 of the stimulable phosphor sheet 15 have been scanned with the laser beam 24, it outputs a drive signal to the main scanning stepping motor 65, thereby returning the optical head 35 to its original position and outputs a drive signal to the sub-scanning pulse motor 61, thereby causing it to move the movable base plate 63 by one scanning line in the sub-scanning direction.

When the control unit 70 determines based on a detection signal indicating the position of the optical head 35 input from the linear encoder 67 that the optical head 35 has been returned to its original position and determines that the movable base plate 63 has been moved by one scanning line in the sub-scanning direction, similarly to the manner in which the stimulable phosphor layer regions 17 included in the first line of the stimulable phosphor layer regions 17 formed in the support 16 of the stimulable phosphor sheet 15 were sequentially irradiated with the laser beam 24 emitted from the first laser stimulating ray source 21, the stimulable phosphor layer regions 17 included in a second line of the stimulable phosphor layer regions 17 formed in the support 16 of the stimulable phosphor sheet 17 are sequentially irradiated with the laser beam 24 emitted from the first laser stimulating ray source 21, thereby exciting stimulable phosphor contained in the stimulable phosphor layer regions 17 included in the second line and stimulated emission 45 released from the stimulable phosphor layer regions 17 in the second line is sequentially and photoelectrically detected by the photomultiplier 50.

Analog data produced by photoelectrically detecting stimulated emission 45 with the photomultiplier 50 are converted by an A/D converter 53 into digital data, thereby producing biochemical analysis data from radiation data recorded in the stimulable phosphor layer regions 17 formed in the support 16 of the stimulable phosphor sheet 15.

When all of the stimulable phosphor layer regions 17 formed in the support 16 of the stimulable phosphor sheet 15 have been scanned with the laser beam 24 emitted from the first laser stimulating ray source 21 to excite stimulable phosphor contained in the stimulable phosphor layer regions 17 and biochemical analysis data produced from radiation data recorded in the stimulable phosphor layer regions 17 formed in the support 16 of the stimulable phosphor sheet 15 by photoelectrically detecting stimulated emission 45 released from the stimulable phosphor layer regions 17 with the photomultiplier 50 to produce analog data and digitizing the analog data by the A/D converter 53 have been forwarded to the data processing apparatus 54, the control unit 70 outputs a drive stop signal to the first laser stimulating ray source 21, thereby turning it off.

As described above, radiation data of the radioactive labeling substance recorded in a number of the stimulable phosphor layer regions 17 of the stimulable phosphor sheet 15 are read by the first scanner to produce biochemical analysis data.

On the other hand, when fluorescence data of a fluorescent substance recorded in a number of the absorptive regions 4 formed in the substrate 2 of the biochemical analysis unit 1 are to be read to produce biochemical analysis data, the biochemical analysis unit 1 is first set by the user on the glass plate 41 of the stage 40.

An instruction signal indicating that fluorescence data recorded in a number of the absorptive regions 4 formed in the substrate 2 of the biochemical analysis unit 1 are to be read is then input by the user through the keyboard 71 together with a labeling substance identifying signal for identifying the kind of fluorescent substance such as a fluorescent dye labeling a substance derived from a living organism.

When the instruction signal and the labeling substance identifying signal are input by the user through the keyboard 71, the control unit 70 selects based on the instruction signal and the labeling substance identifying signal a laser stimulating ray source for emitting a laser beam 24 of a wavelength capable of efficiently stimulating the input fluorescent substance from among the first laser stimulating ray source 21, the second laser stimulating ray source 22 and the third laser stimulating ray source 23 and selects the filter member for cutting light having a wavelength of the laser beam 24 to be used for stimulating the input fluorescent substance and transmitting light having a longer wavelength than that of the laser beam to be used for stimulation from among the three filter members 51a, 51b and 51c.

For example, when Rhodamine (registered trademark), which can be most efficiently stimulated by a laser beam having a wavelength of 532 nm, is used as a fluorescent substance for labeling a substance derived from a living organism and a signal indicating such a fact is input, the control unit 70 selects the second laser stimulating ray source 22 and the filter 52b and outputs a drive signal to the filter unit motor 72, thereby moving the filter unit 48 so that the filter member 51b inserting the filter 52b having a property of cutting off light having a wavelength of 532 nm but transmitting light having a wavelength longer than 532 nm in the optical path of the fluorescence emission 45 to be released from the biochemical analysis unit 1.

The control unit 70 further outputs a drive signal to the main scanning stepping motor 65 to move the optical head 35 in the main scanning direction and when it determines based on a detection signal indicating the position of the optical head 35 input from the linear encoder 67 that the optical head 35 has reached a position where a laser beam 24 can be projected onto a first absorptive region 4 among a number of the absorptive regions 4 formed in the substrate 2 of the biochemical analysis unit 1, it outputs a drive stop signal to the main scanning stepping motor 65 and a drive signal to the second laser stimulating ray source 22, thereby actuating it to emit a laser beam 24 having a wavelength of 532 nm.

The laser beam 24 emitted from the second laser stimulating ray source 22 is made a parallel beam by the collimator lens 30, advances to the first dichroic mirror 27 and is reflected thereby.

The laser beam 24 reflected by the first dichroic mirror 27 transmits through the second dichroic mirror 28 and advances to the mirror 29.

The laser beam 24 advancing to the mirror 29 is reflected by the mirror 29 and further advances to the mirror 32 to be reflected thereby.

The laser beam 24 reflected by the mirror 32 advances to the perforated mirror 34 and passes through the hole 33 of the perforated mirror 34. Then, the laser beam 24 advances to the concave mirror 38.

The laser beam 24 advancing to the concave mirror 38 is reflected thereby and enters the optical head 35.

The laser beam 24 entering the optical head 35 is reflected by the mirror 36 and condensed by the aspherical lens 37 onto the first absorptive region 4 of the biochemical analysis unit 1 placed on the glass plate 41 of the stage 40.

In this embodiment, since each of the absorptive regions 4 of the biochemical analysis unit 1 is formed by charging nylon-6 in the through-hole 3 formed in the substrate 2 made of stainless steel and the substrate 2 capable of attenuating light energy are present around each of the absorptive regions 4 of the biochemical analysis unit 1, it is possible to effectively prevent the laser beam 24 from scattering in each of the absorptive regions 4 and entering the neighboring absorptive regions 4 to excite a fluorescent substance contained in the neighboring absorptive regions 4.

When the laser beam 24 impinges onto the first absorptive region 4 formed in the biochemical analysis unit 1, a fluorescent substance such as a fluorescent dye, for instance, Rhodamine, contained in the absorptive region 4 formed in the biochemical analysis unit 1 is stimulated by the laser beam 24 and fluorescence emission 45 is released from Rhodamine.

In this embodiment, since each of the absorptive regions 4 of the biochemical analysis unit 1 is formed by charging nylon-6 in the through-hole 3 formed in the substrate 2 made of stainless steel and the substrate 2 capable of attenuating light energy are present around each of the absorptive regions 4 of the biochemical analysis unit 1, it is possible to effectively prevent fluorescence emission 45 released from a fluorescent substance from scattering in the biochemical analysis unit 1 and being mixed with fluorescence emission 45 released from a fluorescent substance contained in the neighboring absorptive regions 4.

The fluorescence emission 45 released from Rhodamine is condensed by the aspherical lens 37 provided in the optical head 35 and reflected by the mirror 36 on the side of an optical path of the laser beam 24, thereby being made a parallel beam to advance to the concave mirror 38.

The fluorescence emission 45 advancing to the concave mirror 38 is reflected by the concave mirror 38 and advances to the perforated mirror 34.

As shown in Figure 7, the fluorescence emission 45 advancing to the perforated mirror 34 is reflected downward by the perforated mirror 34 formed as a concave mirror and advances to the filter 52b of a filter unit 48.

Since the filter 52b has a property of cutting off light having a wavelength of 532 nm but transmitting light having a wavelength longer than 532 nm, light having the same wavelength of 532 nm as that of the stimulating ray is cut off by the filter 52b and only light in the wavelength of the fluorescence emission 45 released from Rhodamine passes through the filter 52b to be photoelectrically detected by the photomultiplier 50.

Analog data produced by photoelectrically detecting stimulated emission 45 with the photomultiplier 50 are converted by an A/D converter 53 into digital data and the digital data are fed to a data processing apparatus 54.

When a predetermined time, for example, several microseconds, has passed after the second laser stimulating ray source 22 was turned on, the control unit 70 outputs a drive stop signal to the second laser stimulating ray source 22, thereby turning it off and outputs a drive signal to the main scanning stepping motor 65, thereby moving the optical head 35 by one pitch equal to the distance between neighboring absorptive regions 4 formed in the biochemical analysis unit 1.

When the control unit 70 determines based on a detection signal indicating the position of the optical head 35 input from the linear encoder 67 that the optical head 35 has been moved by one pitch equal to the distance between neighboring absorptive regions 4 formed in the substrate 2 of the biochemical analysis unit 1 and has reached a position where a laser beam 24 can be projected onto a second absorptive region 4 next to the first absorptive region 4 formed in the substrate 2 of the biochemical analysis unit 1, it outputs a drive signal to the second laser stimulating ray source 22 to turn it on, thereby causing the laser beam 24 to excite a fluorescent substance, for example, Rhodamine, contained in the second absorptive region 4 formed in the substrate 2 of the biochemical analysis unit 1 next to the first absorptive region 4.

Similarly to the above, the second absorptive region 4 formed in the substrate 2 of the biochemical analysis unit 1 is irradiated with the laser beam 24 for a predetermined time and when fluorescence emission 45 released from the second absorptive region 4 is photoelectrically detected by the photomultiplier 50, the control unit 70 outputs a drive stop signal to the second laser stimulating ray source 21, thereby turning it off and outputs a drive signal to the main scanning stepping motor 65, thereby moving the optical head 35 by one pitch equal to the distance between neighboring absorptive regions 4 formed in the substrate 2 of the biochemical analysis unit 1.

In this manner, the on and off operation of the second laser stimulating ray source 22 is repeated in synchronism with the intermittent movement of the optical head 35 and when the control unit 70 determines based on a detection signal indicating the position of the optical head 35 input from the linear encoder 67 that the optical head 35 has been moved by one scanning line in the main scanning direction and that the absorptive regions 4 included in a first line of the absorptive regions 4 formed in the substrate 2 of the biochemical analysis unit 1 have been scanned with the laser beam 24, it outputs a drive signal to the main scanning stepping motor 65, thereby returning the optical head 35 to its original position and outputs a drive signal to the sub-scanning pulse motor 61, thereby causing it to move the movable base plate 63 by one scanning line in the sub-scanning direction.

When the control unit 70 determines based on a detection signal indicating the position of the optical head 35 input from the linear encoder 67 that the optical head 35 has been returned to its original position and determines that the movable base plate 63 has been moved by one scanning line in the sub-scanning direction, similarly to the manner in which the absorptive regions 4 included in the first line of the absorptive regions 4 formed in the substrate 2 of the biochemical analysis unit 1 were sequentially irradiated with the laser beam 24 emitted from the second laser stimulating ray source 22, the absorptive regions 4 included in a second line of the absorptive regions 4 formed in the substrate 2 of the biochemical analysis unit 1 are sequentially irradiated with the laser beam 24 emitted from the second laser stimulating ray source 22, thereby exciting Rhodamine contained in the absorptive regions 4 included in the second line and fluorescence emission 45 released from the absorptive regions 4 included in the second line is sequentially and photoelectrically detected by the photomultiplier 50.

Analog data produced by photoelectrically detecting stimulated emission 45 with the photomultiplier 50 are converted by an A/D converter 53 into digital data and the digital data are fed to a data processing apparatus 54.

When all of the absorptive regions 4 formed in the substrate 2 of the biochemical analysis unit 1 have been scanned with the laser beam 24 to excite Rhodamine contained in the absorptive regions 4 and digital data produced by photoelectrically detecting fluorescence emission 45 released from the absorptive regions 4 by the photomultiplier 50 to produce analog data and digitizing the analog data by the A/D converter 53 have been forwarded to the data processing apparatus 54, the control unit 70 outputs a drive stop signal to the second laser stimulating ray source 22, thereby turning it off.

As described above, fluorescence data recorded in a number of the absorptive regions 4 of the biochemical analysis unit 1 are read by the scanner to produce biochemical analysis data.

Chemiluminescence data of a labeling substance recorded in absorptive regions 4 formed in the biochemical analysis unit 1 are transferred onto a stimulable phosphor sheet or read by a cooled CCD camera of a data processing system described later.

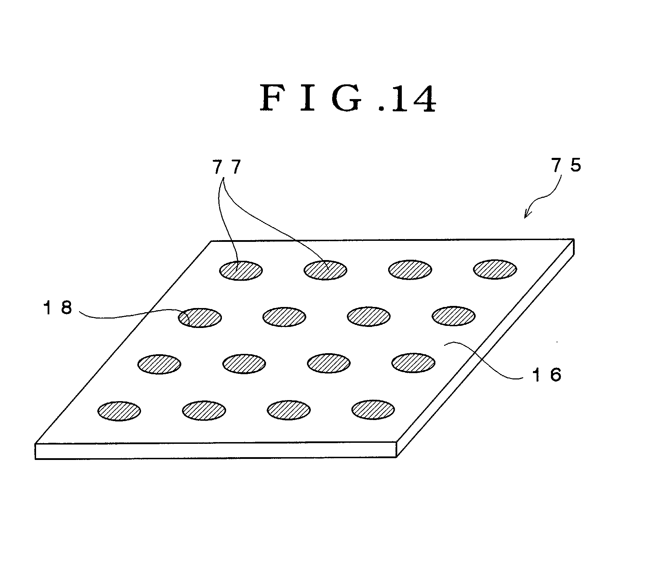

Figure 14 is a schematic perspective view showing a stimulable phosphor sheet onto which chemiluminescence data are to be transferred.

A stimulable phosphor sheet 75 shown in Figure 14 has the same configuration as that of the stimulable phosphor sheet 15 shown in Figure 4 except that a number of stimulable phosphor layer regions 77 are formed by charging SrS system stimulable phosphor capable of absorbing and storing light energy in a number of the through-holes 18 formed in the support 16.

Chemiluminescence data recorded in a number of the absorptive regions 4 of the biochemical analysis unit 1 are transferred onto a number of the stimulable phosphor layer regions 77 of the stimulable phosphor sheet 75 shown in Figure 14.

When chemiluminescence data recorded in a number of the absorptive regions 4 of the biochemical analysis unit 1 are to be transferred onto a number of the stimulable phosphor layer regions 77 of the stimulable phosphor sheet 7575, a number of the absorptive regions 4 of the biochemical analysis unit 1 are first brought into contact with a chemiluminescent substrate.

As a result, chemiluminescence emission in a wavelength of visible light is selectively released from a number of the absorptive regions 4 of the biochemical analysis unit 1.

The stimulable phosphor sheet 75 is then superposed on the biochemical analysis unit 1 formed with a number of the absorptive regions 4 selectively releasing chemiluminescence emission in such a manner that a number of the stimulable phosphor layer regions 77 formed in the stimulable phosphor sheet 75 face the corresponding absorptive regions 4 formed in the biochemical analysis unit 1.

In this manner, each of the stimulable phosphor layer regions 77 formed in the support 16 of the stimulable phosphor sheet 75 is kept to face the corresponding absorptive region 4 formed in the substrate 2 of the biochemical analysis unit 1 for a predetermined time period, whereby a number of the stimulable phosphor layer regions 77 formed in the support 16 of the stimulable phosphor sheet 75 are exposed to chemiluminescence emission released from a number of the absorptive regions 4 formed in the substrate 2 of the biochemical analysis unit 1.

In this embodiment, since the substrate 2 made of stainless steel capable of attenuating light energy are present around each of the absorptive regions 4 formed in the biochemical analysis unit 1, chemiluminescence emission released from the absorptive regions 4 of the biochemical analysis unit 1 during the exposure operation can be efficiently prevented from scattering in the biochemical analysis unit 1. Further, since the support 16 of the stimulable phosphor sheet 75 is made of stainless steel capable of attenuating light energy, chemiluminescence emission released from the absorptive regions 4 of the biochemical analysis unit 1 can be efficiently prevented from scattering in the support 16 of the stimulable phosphor sheet 75 and impinging on the stimulable phosphor layer regions 77 neighboring absorptive regions 4 face.

In this manner, chemiluminescence data are recorded in a number of the stimulable phosphor layer regions 77 formed in the support 16 of the stimulable phosphor sheet 75.

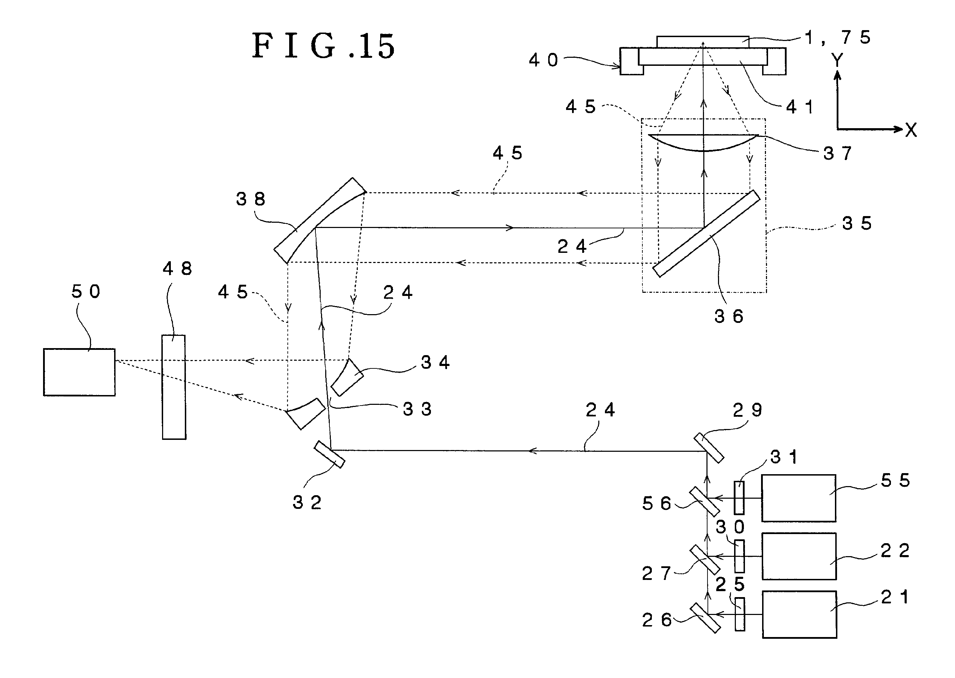

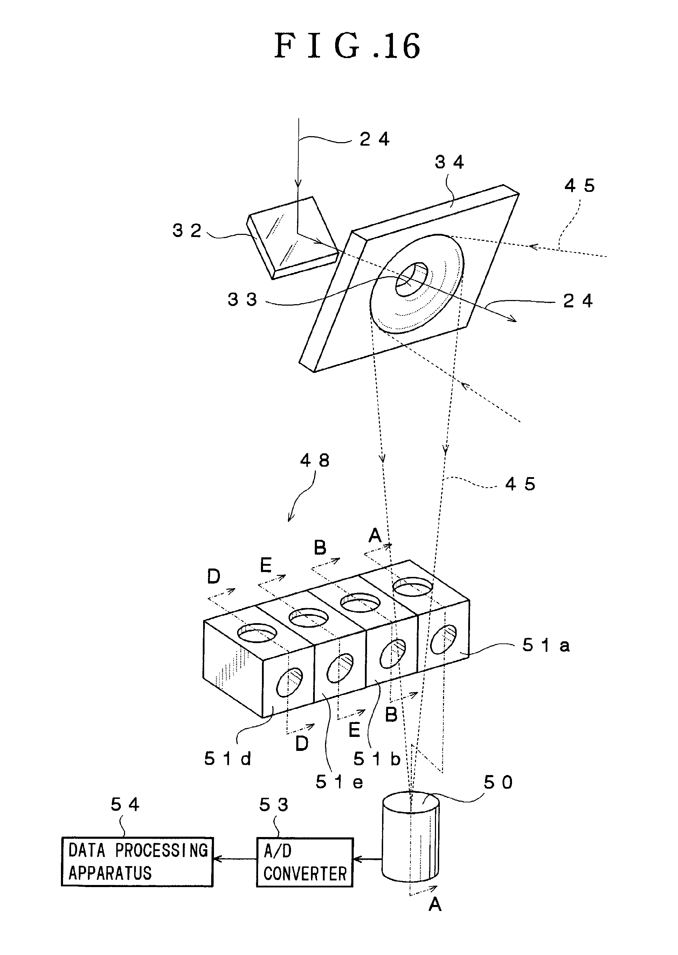

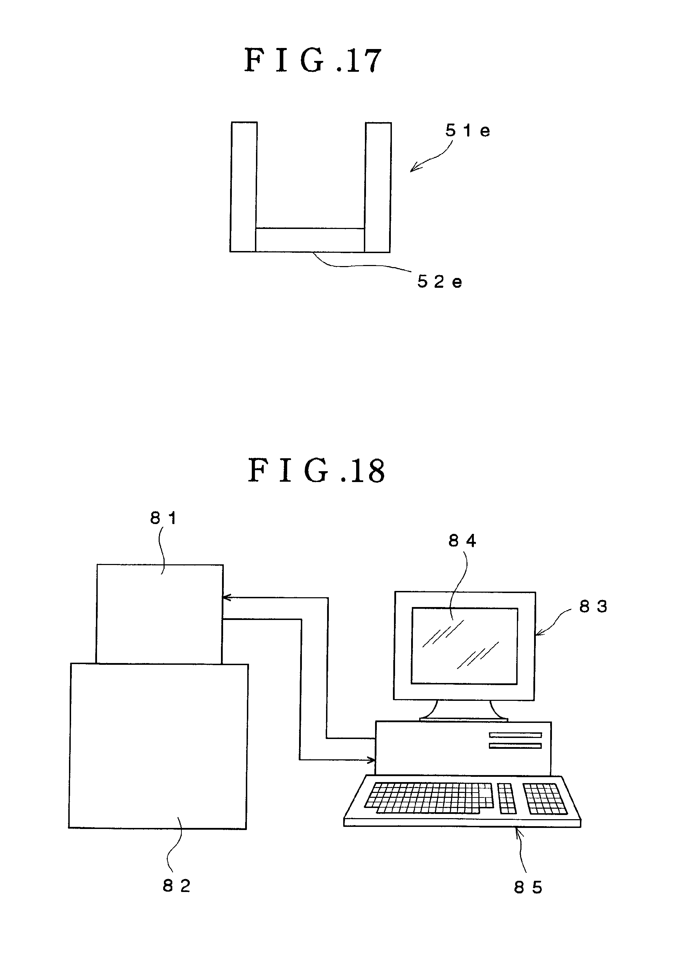

Figure 15 is a schematic view showing a scanner for reading chemiluminescence data recorded in a number of the stimulable phosphor layer regions 17 formed in the support 16 of the stimulable phosphor sheet 75 and producing biochemical analysis data. Figure 16 is a schematic perspective view showing details in the vicinity of a photomultiplier of a scanner shown in Figure 15 and Figure 17 is a schematic cross-sectional view taken along a line E-E in Figure 16.

The scanner shown in Figures 15 to 17 has the same configuration as that of the first scanner shown in Figures 6 to 13 except that it includes a fourth laser stimulating ray source 55 for emitting a laser beam 24 having a wavelength of 980 nm which can effectively stimulate SrS system stimulable phosphor instead of the third laser stimulating ray source 23 for emitting a laser beam 24 having a wavelength of 473 nm, includes a filter member 51e provided with a filter having a property of transmitting only light having a wavelength corresponding to that of stimulated emission emitted from stimulable phosphor and cutting off light having a wavelength of 980 nm, and includes a third dichroic mirror 56 for transmitting light having a wavelength equal to and shorter than 640 nm but reflecting light having a wavelength of 980 nm instead of the second dichroic mirror 28 for transmitting light having a wavelength equal to and longer than 532 nm but reflecting light having a wavelength of 473 nm.

The thus constituted scanner reads chemiluminescence data recorded in a number of the stimulable phosphor layer regions 77 of the stimulable phosphor sheet 75 and produces biochemical analysis data in the following manner.

A stimulable phosphor sheet 75 is first set on the glass plate 41 of the stage 40 by a user.

An instruction signal indicating that chemiluminescence data recorded in the stimulable phosphor layer 82 formed in the stimulable phosphor sheet 75 are to be read is then input through the keyboard 71.

The instruction signal input through the keyboard 71 is input to the control unit 70 and the control unit 70 outputs a drive signal to the filter unit motor 72 in accordance with the instruction signal, thereby moving the filter unit 48 so as to locate the filter member 51e provided with the filter 52e having a property of transmitting only light having a wavelength corresponding to that of stimulated emission emitted from the stimulable phosphor layer regions 77 and cutting off light having a wavelength of 980 nm in the optical path of stimulated emission 45.

The control unit 70 further outputs a drive signal to the main scanning stepping motor 65 to move the optical head 35 in the main scanning direction and when it determines based on a detection signal indicating the position of the optical head 35 input from the linear encoder 67 that the optical head 35 has reached a position where a laser beam 24 can be projected onto a first stimulable phosphor layer region 77 among a number of the stimulable phosphor layer regions 77 formed in the support 16 of the stimulable phosphor sheet 75, it outputs a drive stop signal to the main scanning stepping motor 65 and a drive signal to the fourth stimulating ray source 55, thereby actuating it to emit a laser beam 24 having a wavelength of 980 nm.

A laser beam 24 emitted from the fourth laser stimulating ray source 55 passes through a collimator lens 31, thereby being made a parallel beam, and is reflected by the third dichroic mirror 56, thereby changing its direction by 90 degrees. The laser beam 24 then advances to the mirror 29.

The laser beam 24 advancing to the mirror 29 is reflected by the mirror 29 and advances to the mirror 32 to be reflected thereby.

The laser beam 24 reflected by the mirror 32 passes through the hole 33 of the perforated mirror 34 and advances to the concave mirror 38.

The laser beam 24 advancing to the concave mirror 38 is reflected by the concave mirror 38 and enters the optical head 35.

The laser beam 24 entering the optical head 35 is reflected by the mirror 36 and condensed by the aspherical lens 37 onto the first stimulable phosphor layer region 77 of the stimulable phosphor sheet 75 placed on the glass plate 41 of a stage 40.

In this embodiment, since each of the stimulable phosphor layer regions 77 of the stimulable phosphor sheet 75 is formed by charging stimulable phosphor in the through-hole formed in the support 16 made of stainless steel capable of attenuating light energy, it is possible to effectively prevent the laser beam 24 from scattering in each of the stimulable phosphor layer regions 77 and entering the neighboring stimulable phosphor layer regions 77 to excite stimulable phosphor contained in the neighboring stimulable phosphor layer regions 77.

When the laser beam 24 impinges onto the first stimulable phosphor layer region 77 formed in the support 16 of the stimulable phosphor sheet 77, stimulable phosphor contained in the first stimulable phosphor layer region 77 formed in the support 16 of the stimulable phosphor sheet 75 is excited by the laser beam 24, thereby releasing stimulated emission 45 from the first stimulable phosphor layer region 77.

The stimulated emission 45 released from the first stimulable phosphor layer region 77 of the stimulable phosphor sheet 75 is condensed onto the mirror 36 by the aspherical lens 37 provided in the optical head 35 and reflected by the mirror 36 on the side of the optical path of the laser beam 24, thereby being made a parallel beam to advance to the concave mirror 38.

The stimulated emission 45 advancing to the concave mirror 38 is reflected by the concave mirror 38 and advances to the perforated mirror 34.

As shown in Figure 15, the stimulated emission 45 advancing to the perforated mirror 34 is reflected downward by the perforated mirror 34 formed as a concave mirror and advances to the filter 52e of the filter unit 48.

Since the filter 52e (Figure 16) has a property of transmitting only light having a wavelength corresponding to that of stimulated emission emitted from stimulable phosphor and cutting off light having a wavelength of 980 nm, light having a wavelength of 980 nm corresponding to that of the stimulating ray is cut off by the filter 52e and only light having a wavelength corresponding to that of stimulated emission passes through the filter 52e to be photoelectrically detected by the photomultiplier 50.

Analog data produced by photoelectrically detecting stimulated emission 45 with the photomultiplier 50 are converted by an A/D converter 53 into digital data and the digital data are fed to a data processing apparatus 54.

When a predetermined time, for example, several microseconds, has passed after the fourth stimulating ray source 55 was turned on, the control unit 70 outputs a drive stop signal to the fourth stimulating ray source 55, thereby turning it off and outputs a drive signal to the main scanning stepping motor 65, thereby moving the optical head 35 by one pitch equal to the distance between neighboring stimulable phosphor layer regions 77 formed in the support 16 of the stimulable phosphor sheet 75.

When the control unit 70 determines based on a detection signal indicating the position of the optical head 35 input from the linear encoder 67 that the optical head 35 has been moved by one pitch equal to the distance between neighboring stimulable phosphor layer regions 77, it outputs a drive signal to the fourth stimulating ray source 55 to turn it on, thereby causing the laser beam 24 to excite stimulable phosphor contained in a second stimulable phosphor layer region 77 formed in the support 16 of the stimulable phosphor sheet 75 next to the first stimulable phosphor layer region 77.

Similarly to the above, the second stimulable phosphor layer region 77 formed in the support 16 of the stimulable phosphor sheet 75 is irradiated with the laser beam 24 for a predetermined time and when stimulated emission 45 released from the second stimulable phosphor layer region 77 is photoelectrically detected by the photomultiplier 50, the control unit 70 outputs a drive stop signal to the fourth stimulating ray source 55, thereby turning it off and outputs a drive signal to the main scanning stepping motor 65, thereby moving the optical head 35 by one pitch equal to the distance between neighboring stimulable phosphor layer regions 77.

In this manner, the on and off operation of the fourth stimulating ray source 55 is repeated in synchronism with the intermittent movement of the optical head 35 and when the control unit 70 determines based on a detection signal indicating the position of the optical head 35 input from the linear encoder 67 that the optical head 35 has been moved by one scanning line in the main scanning direction and that the stimulable phosphor layer regions 77 included in a first line of the stimulable phosphor layer regions 77 formed in the support 16 of the stimulable phosphor sheet 75 have been scanned with the laser beam 24, it outputs a drive signal to the main scanning stepping motor 65, thereby returning the optical head 35 to its original position and outputs a drive signal to the sub-scanning pulse motor 61, thereby causing it to move the movable base plate 63 by one scanning line in the sub-scanning direction.

When the control unit 70 determines based on a detection signal indicating the position of the optical head 35 input from the linear encoder 67 that the optical head 35 has been returned to its original position and determines that the movable base plate 63 has been moved by one scanning line in the sub-scanning direction, similarly to the manner in which the stimulable phosphor layer regions 77 included in the first line of the stimulable phosphor layer regions 77 formed in the support 16 of the stimulable phosphor sheet 75 were sequentially irradiated with the laser beam 24 emitted from the fourth laser stimulating ray source 55, the stimulable phosphor layer regions 77 included in a second line of the stimulable phosphor layer regions 77 formed in the support 16 of the stimulable phosphor sheet 75 are sequentially irradiated with the laser beam 24 emitted from the fourth laser stimulating ray source 55, thereby exciting stimulable phosphor contained in the stimulable phosphor layer regions 77 included in the second line and stimulated emission 45 released from the stimulable phosphor layer regions 77 included in the second line is sequentially and photoelectrically detected by the photomultiplier 50.

Analog data produced by photoelectrically detecting stimulated emission 45 with the photomultiplier 50 are converted by an A/D converter 53 into digital data and the digital data are fed to a data processing apparatus 54.

When all of the stimulable phosphor layer regions 77 formed in the support 16 of the stimulable phosphor sheet 75 have been scanned with the laser beam 24 to excite stimulable phosphor contained in the stimulable phosphor layer regions 77 and digital data produced by photoelectrically detecting stimulated emission 45 released from the stimulable phosphor layer regions 77 by the photomultiplier 50 to produce analog data and digitizing the analog data by the A/D converter 53 have been forwarded to the data processing apparatus 54, the control unit 70 outputs a drive stop signal to the fourth laser stimulating ray source 55, thereby turning it off.

As described above, chemiluminescence data recorded in a number of the stimulable phosphor layer regions 77 of the stimulable phosphor sheet 75 are read by the scanner to produce biochemical analysis data.

Chemiluminescence data of a labeling substance recorded in a number of the absorptive regions 4 formed in the substrate 2 of the biochemical analysis unit 1 can instead be read by a cooled CCD camera of a data producing system to produce biochemical analysis data without transferring them onto a number of the stimulable phosphor layer regions 82 of the stimulable phosphor sheet 80.

Figure 18 is a schematic front view showing a data producing system for reading chemiluminescence data recorded in a number of the absorptive regions 4 formed in the substrate 2 of the biochemical analysis unit 1, and producing biochemical analysis data.

The data producing system shown in Figure 18 is constituted to be able to also read fluorescence data of a fluorescent substance such as a fluorescent dye recorded in a number of the absorptive regions 4 formed in the biochemical analysis unit 1.

As shown in Figure 18, the data producing system includes a cooled CCD camera 81, a dark box 82 and a personal computer 83. As shown in Figure 14, the personal computer 83 is equipped with a CRT 84 and a keyboard 85.

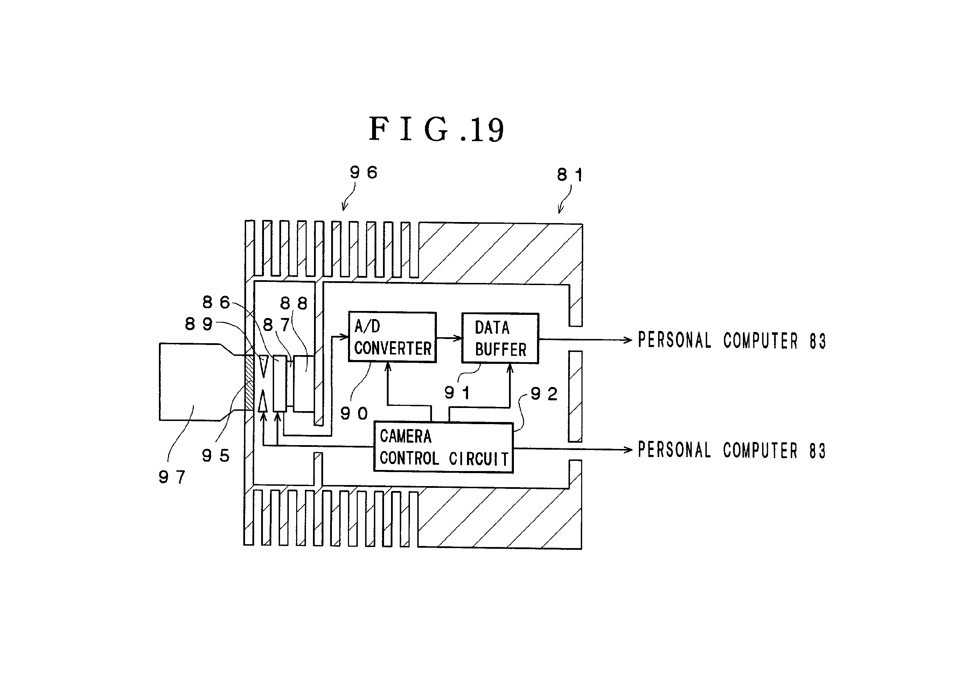

Figure 19 is a schematic longitudinal cross sectional view showing the cooled CCD camera 81.

As shown in Figure 19, the cooled CCD camera 81 includes a CCD 86, a heat transfer plate 87 made of metal such as aluminum, a Peltier element 88 for cooling the CCD 86, a shutter 89 disposed in front of the CCD 86, an A/D converter 90 for converting analog data produced by the CCD 86 to digital data, a data buffer 91 for temporarily storing the data digitized by the A/D converter 90, and a camera control circuit 92 for controlling the operation of the cooled CCD camera 81. An opening formed between the dark box 82 and the cooled CCD camera 81 is closed by a glass plate 95 and the periphery of the cooled CCD camera 81 is formed with heat dispersion fins 96 over substantially its entire length for dispersing heat.

A camera lens 97 disposed in the dark box 82 is mounted on the front surface of the glass plate 95 disposed in the cooled CCD camera 81.

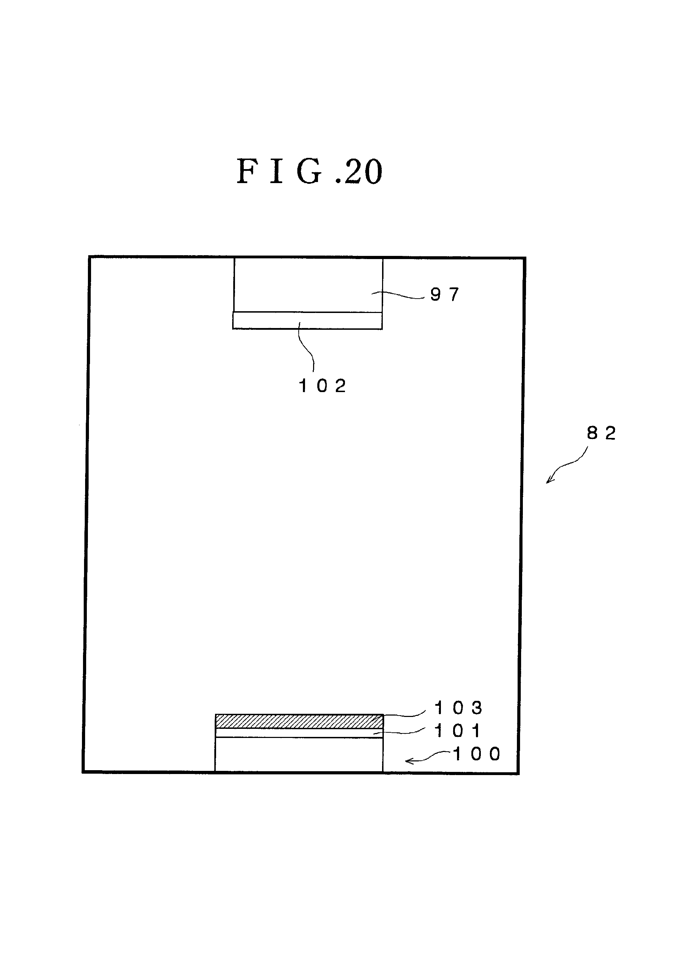

Figure 20 is a schematic vertical cross sectional view showing the dark box 82 of the data producing system.

As shown in Figure 20, the dark box 82 is equipped with a light emitting diode stimulating ray source 100 for emitting a stimulating ray. The light emitting diode stimulating ray source 100 is provided with a filter 101 detachably mounted thereon and a diffusion plate 103 mounted on the upper surface of the filter 101. The stimulating ray is emitted via the diffusion plate 103 toward a biochemical analysis unit (not shown) placed on the diffusion plate 103 so as to ensure that the biochemical analysis unit can be uniformly irradiated with the stimulating ray. The filter 101 has a property of cutting light components having a wavelength not close to that of the stimulating ray and harmful to the stimulation of a fluorescent substance and transmitting through only light components having a wavelength in the vicinity of that of the stimulating ray. A filter 102 for cutting light components having a wavelength in the vicinity of that of the stimulating ray is detachably provided on the front surface of the camera lens 97.

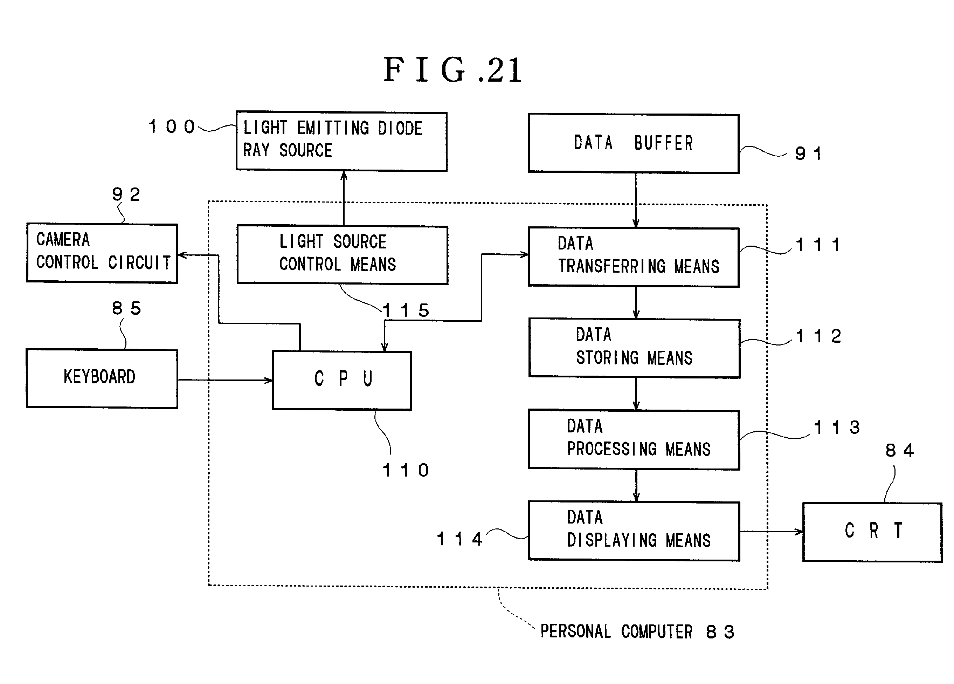

Figure 21 is a block diagram of the personal computer 83 of the data producing system and peripheral devices thereof.

As shown in Figure 21, the personal computer 83 includes a CPU 110 for controlling the exposure of the cooled CCD camera 81, a data transferring means 111 for reading the data produced by the cooled CCD camera 81 from the data buffer 91, a storing means 112 for storing data, a data processing means 113 for effecting data processing on the digital data stored in the data storing means 112, and a data displaying means 114 for displaying visual data on the screen of the CRT 84 based on the digital data stored in the data storing means 112. The light emitting diode stimulating ray source 100 is controlled by a light source control means 115 and an instruction signal can be input via the CPU 110 to the light source control means 115 through the keyboard 85. The CPU 110 is constituted so as to output various signals to the camera controlling circuit 93 of the cooled CCD camera 81.

The data producing system shown in Figures 18 to 21 is constituted so as to detect chemiluminescence emission generated by the contact of a labeling substance contained in a number of the absorptive regions 4 formed in the biochemical analysis unit 1 and a chemiluminescent substrate, with the CCD 86 of the cooled CCD camera 81 through the camera lens 97, thereby reading chemiluminescence data to produce biochemical analysis data, and irradiate the biochemical analysis unit 1 with a stimulating ray emitted from the light emitting diode stimulating ray source 100 and detect fluorescence emission released from a fluorescent substance such as a fluorescent dye contained in a number of the absorptive regions 4 formed in the biochemical analysis unit 1 upon being stimulated, with the CCD 86 of the cooled CCD camera 81 through a camera lens 97, thereby reading fluorescence data to produce biochemical analysis data.

When biochemical analysis data are to be produced by reading chemiluminescence data, the filter 102 is removed and while the light emitting diode stimulating ray source 100 is kept off, the biochemical analysis unit 1 is placed on the diffusion plate 103. At this time, the biochemical analysis unit 1 is releasing chemiluminescence emission as a result of contact of a labeling substance contained in a number of the absorptive regions 4 formed in the biochemical analysis unit 1 and a chemiluminescent substrate.

The lens focus is then adjusted by the user using the camera lens 97 and the dark box 82 is closed.

When an exposure start signal is input by the user through the keyboard 85, the exposure start signal is input through the CPU 110 to the camera control circuit 92 of the cooled CCD camera 81 so that the shutter 88 is opened by the camera control circuit 92, whereby the exposure of the CCD 86 is started.

Chemiluminescence emission released from a number of the absorptive regions 4 of the biochemical analysis unit 1 impinges on the light receiving surface of the CCD 86 of the cooled CCD camera 81 via the camera lens 97, thereby forming an image on the light receiving surface. The CCD 86 receives light of the thus formed image and accumulates it in the form of electric charges therein.

In this embodiment, since the substrate 2 made of stainless steel and capable of attenuating light energy are present around each of the absorptive regions 4 formed in the biochemical analysis unit 1, it is possible to reliably prevent chemiluminescence emission released from the labeling substance contained in each of the absorptive regions 4 from being mixed with chemiluminescence emission released from a labeling substance contained in the neighboring absorptive regions 4.

When a predetermined exposure time has passed, the CPU 110 outputs an exposure completion signal to the camera control circuit 92 of the cooled CCD camera 81.

When the camera controlling circuit 92 receives the exposure completion signal from the CPU 110, it transfers analog data accumulated in the CCD 86 in the form of electric charge to the A/D converter 90 to cause the A/D converter 90 to digitize the data and to temporarily store the thus digitized data in the data buffer 91.

At the same time, the CPU 110 outputs a data transfer signal to the data transferring means 111 to cause it to read out the digital data from the data buffer 91 of the cooled CCD camera 81 and to input them to the data storing means 112.

When the user inputs a data producing signal through the keyboard 85, the CPU 110 outputs the digital data stored in the data storing means 112 to the data processing means 113 and causes the data processing means 113 to effect data processing on the digital data in accordance with the user's instructions. The CPU 110 then outputs a data display signal to the displaying means 114 and causes the displaying means 114 to display biochemical analysis data on the screen of the CRT 84 based on the thus processed digital data.

On the other hand, when biochemical analysis data are to be produced by reading fluorescence data, the biochemical analysis unit 1 is first placed on the diffusion plate 103.

The light emitting diode stimulating ray source 100 is then turned on by the user and the lens focus is adjusted using the camera lens 97. The dark box 92 is then closed.

When the user inputs an exposure start signal through the keyboard 85, the light emitting diode stimulating ray source 100 is again turned on by the light source control means 115, thereby emitting a stimulating ray toward the biochemical analysis unit 1.

At the same time, the exposure start signal is input via the CPU 110 to the camera control circuit 92 of the cooled CCD camera 81 and the shutter 89 is opened by the camera control circuit 92, whereby the exposure of the CCD 86 is started.

The stimulating ray emitted from the light emitting diode stimulating ray source 100 passes through the filter 101, whereby light components of wavelengths not in the vicinity of that of the stimulating ray are cut. The stimulating ray then passes through the diffusion plate 103 to be made uniform light and the biochemical analysis unit 1 is irradiated with the uniform stimulating ray.

When the biochemical analysis unit 1 is irradiated with the stimulating ray, a fluorescent substance such as a fluorescent dye contained in a number of the absorptive regions 4 of the biochemical analysis unit 1 is stimulated by the stimulating ray, thereby releasing fluorescence emission from a number of the absorptive regions 4 of the biochemical analysis unit 1.

The fluorescence emission released from a number of the absorptive regions 4 of the biochemical analysis unit 1 impinges on the light receiving surface of the CCD 86 of the cooled CCD camera 81 through the filter 102 and the camera lens 97 and forms an image thereon. The CCD 86 receives light of the thus formed image and accumulates it in the form of electric charges therein. Since light components of wavelength equal to the stimulating ray wavelength are cut by the filter 102, only fluorescence emission released from the fluorescent substance such as a fluorescent dye contained in a number of the absorptive regions 4 of the biochemical analysis unit 1 is received by the CCD 86.

In this embodiment, since the substrate 2 made of stainless steel and capable of attenuating light energy are present around each of the absorptive regions 4 formed in the biochemical analysis unit 1, it is possible to reliably prevent fluorescence emission released from a fluorescent substance contained in each of the absorptive regions 4 from being mixed with fluorescence emission released from a fluorescent substance contained in the neighboring absorptive regions 4.

When a predetermined exposure time has passed, the CPU 110 outputs an exposure completion signal to the camera control circuit 92 of the cooled CCD camera 81.

When the camera controlling circuit 92 receives the exposure completion signal from the CPU 110, it transfers analog data accumulated in the CCD 86 in the form of electric charge to the A/D converter 90 to cause the A/D converter 90 to digitize the data and to temporarily store the thus digitized data in the data buffer 91.

At the same time, the CPU 110 outputs a data transfer signal to the data transferring means 111 to cause it to read out the digital data from the data buffer 91 of the cooled CCD camera 81 and to input them to the data storing means 112.

When the user inputs a data producing signal through the keyboard 85, the CPU 110 outputs the digital data stored in the data storing means 112 to the data processing apparatus 113 and causes the data processing apparatus 113 to effect data processing on the digital data in accordance with the user's instructions. The CPU 110 then outputs a data display signal to the displaying means 114 and causes the displaying means 114 to display biochemical analysis data on the screen of the CRT 84 based on the thus processed digital data.

When the production of biochemical analysis data has been completed in this manner, the biochemical analysis unit 1 is washed.

According to this embodiment, since the mixed solution of the hybridization buffer and the probe solution is forcibly fed into the cartridge 7 by the pump 10 through a number the absorptive regions 4 formed in the substrate 2 of the biochemical analysis unit 1 accommodated in the cartridge 7, thereby performing hybridization, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Further, according to this embodiment, since the mixed solution of the hybridization buffer and the probe solution is circulated by the pump 10 into the cartridge 7 via the solution circulation pipe 9 and forcibly fed into the cartridge 7 through a number the absorptive regions 4 formed in the substrate 2 of the biochemical analysis unit 1 accommodated in the cartridge 7 repeatedly, it is possible to much more improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1 and therefore, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Furthermore, according to this embodiment, since the mixed solution of the hybridization buffer and the probe solution is forcibly fed into the cartridge 7 in both the obverse direction and the reverse direction by the pump 10 through a number the absorptive regions 4 formed in the substrate 2 of the biochemical analysis unit 1 accommodated in the cartridge 7, it is possible to much more improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1 and therefore, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Moreover, according to this embodiment, since the antibody solution is forcibly fed into the cartridge 7 by the pump 10 to pass through a number the absorptive regions 4 formed in the substrate 2 of the biochemical analysis unit 1 accommodated in the cartridge 7, thereby performing an antigen-antibody reaction, it is possible to markedly increase the moving rate of an antibody through the absorptive regions 4 of the biochemical analysis unit 1 and, therefore, the reaction rate of an antigen-antibody reaction can be markedly improved. Further, since it is possible to markedly improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Further, according to this embodiment, since the antibody solution is circulated by the pump 10 into the cartridge 7 via the solution circulation pipe 9 and forcibly fed into the cartridge 7 to pass through a number the absorptive regions 4 formed in the substrate 2 of the biochemical analysis unit 1 accommodated in the cartridge 7 repeatedly, it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1 and therefore, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Furthermore, according to this embodiment, since the antibody solution is forcibly fed into the cartridge 7 in both the obverse direction and the reverse direction by the pump 10 to pass through a number the absorptive regions 4 formed in the substrate 2 of the biochemical analysis unit 1 accommodated in the cartridge 7, it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1 and therefore, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Moreover, according to this embodiment, since the cleaning solution is forcibly fed into the cartridge 7 by the pump 10 to pass through a number the absorptive regions 4 formed in the substrate 2 of the biochemical analysis unit 1 accommodated in the cartridge 7, thereby performing the cleaning of the absorptive regions 4 of the biochemical analysis unit 1, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1 and even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1. Therefore, the efficiency of cleaning operation can be markedly improved.

Further, according to this embodiment, since the cleaning solution is forcibly fed into the cartridge 7 in both the obverse direction and the reverse direction by the pump 10 to pass through a number the absorptive regions 4 formed in the substrate 2 of the biochemical analysis unit 1 accommodated in the cartridge 7, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to much more efficiently peel off and remove the substance derived from a living organism which should not be hybridized with specific binding substances absorbed in the absorptive regions 4 from a number of the absorptive regions 4 of the biochemical analysis unit 1 and even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1. Therefore, the efficiency of cleaning operation can be markedly improved.

Furthermore, according to this embodiment, since the mixed solution of the hybridization buffer and the probe solution is forcibly fed into the cartridge 7 by the pump 10 to pass through a number the absorptive regions 4 formed in the substrate 2 of the biochemical analysis unit 1 accommodated in the cartridge 7, thereby performing hybridization and the cleaning solution is forcibly fed into the cartridge 7 by the pump 10 through a number the absorptive regions 4 formed in the substrate 2 of the biochemical analysis unit 1 accommodated in the cartridge 7, thereby performing the cleaning of the absorptive regions 4 of the biochemical analysis unit 1, even if a different experimenter performs hybridization, it is possible to reliably prevent different results from being obtained and the repeatability of hybridization can be markedly improved.

Moreover, according to this embodiment, since the antibody solution is forcibly fed into the cartridge 7 by the pump 10 to pass through a number the absorptive regions 4 formed in the substrate 2 of the biochemical analysis unit 1 accommodated in the cartridge 7, thereby performing an antigen-antibody reaction and the cleaning solution is forcibly fed into the cartridge 7 by the pump 10 to pass through a number the absorptive regions 4 formed in the substrate 2 of the biochemical analysis unit 1 accommodated in the cartridge 7, thereby performing the cleaning of the absorptive regions 4 of the biochemical analysis unit 1, even if a different experimenter performs an antigen-antibody reaction, it is possible to reliably prevent different results from being obtained and the repeatability of an antigen-antibody reaction can be markedly improved.

Further, according to this embodiment, since a number of the absorptive regions 4 of the biochemical analysis unit 1 are formed by charging nylon-6 in a number of the through-holes formed in the substrate 2 made of stainless steel, the hybridization buffer, the mixed solution of the hybridization buffer and the probe solution and the cleaning solution are forcibly fed to the biochemical analysis unit 1 so as to pass through only the number of the absorptive regions 4 formed in the substrate 2 of the biochemical analysis unit 1 and, therefore, the efficiency of hybridization, the efficiency of an antigen-antibody reaction and the efficiency of the cleaning operation can be markedly improved.

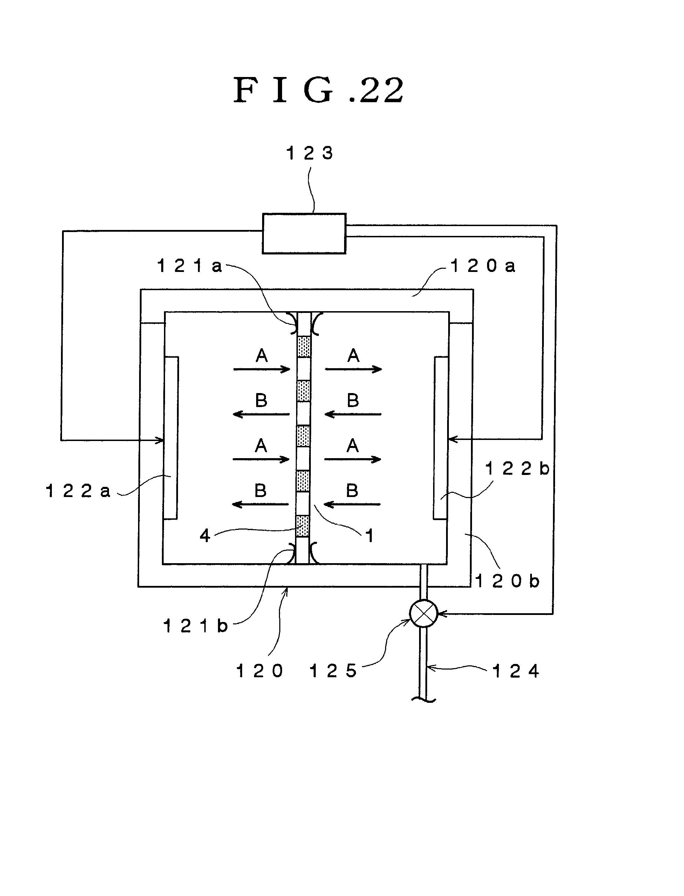

Figure 22 is a schematic longitudinal cross sectional view showing an apparatus for conducting a receptor-ligand association reaction which is another preferred embodiment of the present invention.

As shown in Figure 22, the apparatus for conducting a receptor-ligand association reaction according to this embodiment includes a reaction vessel 120 provided with a lid member 120a and a casing 120b for accommodating a hybridization buffer, a mixed solution of a hybridization buffer and a probe solution, an antibody solution or a cleaning solution.

As shown in Figure 22, a biochemical analysis unit holding member 121a is formed at a substantially center portion on the surface of the lid member 120a forming an upper inner wall surface of the reaction vessel 120, the biochemical analysis unit holding member 121a including a pair of leaf springs (not shown) and adapted for holding the upper end portion of the biochemical analysis unit 1 and a biochemical analysis unit holding member 121b is formed at a substantially center portion on the lower inner surface of the casing 120b correspondingly to the biochemical analysis unit holding member 121a, the biochemical analysis unit holding member 121b including a pair of leaf springs (not shown) and adapted for holding the lower end portion of the biochemical analysis unit 1.

As shown in Figure 22, a pair of ultrasonic oscillators 122a, 122b are provided on facing inner surfaces of the casing 120b so as to face the opposite surfaces of the biochemical analysis unit 1 when it is held in the reaction vessel 120 by the biochemical analysis unit holding member 121a and the biochemical analysis unit holding member 121b.

The pair of ultrasonic oscillators 122a, 122b are selectively oscillated by a controller 123 to generate ultrasound in a hybridization buffer, a mixed solution of a hybridization buffer and a probe solution, an antibody solution or a cleaning solution accommodated in the reaction vessel 120.

As shown in Figure 22, a solution discharge passage 124 is formed at the bottom portion of the reaction vessel 120 and a valve 125 is provided in the solution discharge passage 124 for opening and closing the solution discharge passage 124 under the control of the controller 123.

In the thus constituted apparatus for conducting receptor-legand association, a substance derived from a living body, labeled with a labeling substance and contained in a probe solution selectively hybridizes specific binding substances contained in a number of the absorptive regions 4 of the biochemical analysis unit 1 in the following manner.

A hybridization buffer is first prepared and accommodated in the reaction vessel 120.

The lower end portion of the biochemical analysis unit 1 is inserted into the pair of leaf springs constituting the biochemical analysis unit holding member 121b formed at a substantially center portion on the surface of the bottom inner wall of the casing 120b, whereby the lower end portion of the biochemical analysis unit 1 is held between the pair of leaf springs constituting the biochemical analysis unit holding member 121b.

As a result, the whole biochemical analysis unit 1 other than the upper end portion thereof is immersed in the hybridization buffer.

The lid member 120a is then closed in such a manner that the upper end portion of the biochemical analysis unit 1 is held between the pair of leaf springs constituting the biochemical analysis unit holding member 121a formed at a substantially center portion on the lower surface of the lid member 120a and that the biochemical analysis unit 1 is held at a a substantially center portion in the reaction vessel 120.

When the biochemical analysis unit 1 has been set in the reaction vessel 120 and the lid member 120a has been closed, the ultrasonic oscillator 122a is oscillated by the controller 123.

As a result, ultrasound is generated in the hybridization buffer accommodated in the reaction vessel 120, thereby generating a flow of the hybridization buffer in the direction indicated by an arrow A in Figure 22.

In this embodiment, since a number of the absorptive regions 4 are formed in the biochemical analysis unit 1 by charging nylon-6 in a number of the through-holes 3 formed in the substrate 2 made of stainless steel, the hybridization buffer is forcibly moved in the direction indicated by the arrow A in Figure 22 to pass through only the number of the absorptive regions 4 formed in the substrate 2 of the biochemical analysis unit 1.

As a result, pre-hybridization is performed.

It is preferable in order to pass the hybridization buffer through only the number of the absorptive regions 4 formed in the substrate 2 of the biochemical analysis unit 1 to generate ultrasound having a frequency of 40 kHz or more and more preferably, ultrasound having a frequency of 400 kHz or more.

When a first predetermined time period has passed, the controller 123 stops the oscillation of the ultrasonic oscillator 122a and oscillates the ultrasonic oscillator 122b.

As a result, ultrasound is generated in the hybridization buffer accommodated in the reaction vessel 120, thereby generating a flow of the hybridization buffer is generated in the direction indicated by the arrow B in Figure 22.

Therefore, the hybridization buffer is forcibly moved in the direction indicated by the arrow B in Figure 22 to pass through only the number of the absorptive regions 4 formed in the substrate 2 of the biochemical analysis unit 1.

When the first predetermined time period has passed, the controller 123 stops the oscillation of the ultrasonic oscillator 122b and oscillates the ultrasonic oscillator 122a.

As a result, ultrasound is generated in the hybridization buffer accommodated in the reaction vessel 120, thereby generating a flow of the hybridization buffer is generated in the direction indicated by the arrow A in Figure 22 and the hybridization buffer is forcibly moved in the direction indicated by the arrow A in Figure 22 to pass through only the number of the absorptive regions 4 formed in the substrate 2 of the biochemical analysis unit 1.

When the pair of ultrasonic oscillators 122a, 122b has been oscillated by the controller 123 in this mannerr a predetermined number of times, thereby forcibly moving the hybridization buffer accommodated in the reaction vessel 120 in the directions indicated by the arrows A and B in Figure 22 to pass repeatedly through only the number of the absorptive regions 4 formed in the substrate 2 of the biochemical analysis unit 1, the oscillation of the pair of ultrasonic oscillators 122a, 122b is stopped by the controller 123 and pre-hybridization is completed.

Then, a probe solution is prepared and the lid member 120a is opened to mix the probe solution with the hybridization buffer accommodated in the reaction vessel 120.

In this embodiment, a probe solution containing a substance derived from a living organism and labeled with a radioactive labeling substance, a substance derived from a living organism and labeled with a fluorescent substance such as a fluorescent dye and a substance derived from a living organism and labeled with a hapten such as digoxigenin is prepared and mixed with the hybridization buffer accommodated in the reaction vessel 120.

When the probe solution has been mixed with the hybridization buffer accommodated in the reaction vessel 120, the lid member 120a is closed and the ultrasonic oscillator 122a is oscillated by the controller 123.

As a result, ultrasound is generated in the mixed solution of the hybridization buffer and the probe solution accommodated in the reaction vessel 120, thereby generating a flow of the mixed solution of the hybridization buffer and the probe solution in the direction indicated by the arrow A in Figure 22.

Therefore, the mixed solution of the hybridization buffer and the probe solution is forcibly moved in the direction indicated by the arrow A in Figure 22 to pass through only the number of the absorptive regions 4 formed in the substrate 2 of the biochemical analysis unit 1.

As a result, a substance derived from a living organism and contained in the mixed solution of the hybridization buffer and the probe solution selectively hybridizes with specific binding substances absorbed in a number of the absorptive regions 4 formed in the substrate 2 of the biochemical analysis unit 1.

It is preferable in order to pass the mixed solution of the hybridization buffer and the probe solution through only the number of the absorptive regions 4 formed in the substrate 2 of the biochemical analysis unit 1 to generate ultrasound having a frequency of 40 kHz or more and more preferably, ultrasound having a frequency of 400 kHz or more.

When a second predetermined time period has passed, the controller 123 stops the oscillation of the ultrasonic oscillator 122a and oscillates the ultrasonic oscillator 122b.

As a result, ultrasound is generated in the mixed solution of the hybridization buffer and the probe solution accommodated in the reaction vessel 120, thereby generating a flow of the mixed solution of the hybridization buffer and the probe solution in the direction indicated by the arrow B in Figure 22.

Therefore, the mixed solution of the hybridization buffer and the probe solution is forcibly moved in the direction indicated by the arrow B in Figure 22 to pass through only the number of the absorptive regions 4 formed in the substrate 2 of the biochemical analysis unit 1.

When the first predetermined time period has passed, the controller 123 stops the oscillation of the ultrasonic oscillator 122b and oscillates the ultrasonic oscillator 122a.

As a result, ultrasound is generated in the mixed solution of the hybridization buffer and the probe solution accommodated in the reaction vessel 120, thereby generating a flow of the mixed solution of the hybridization buffer and the probe solution in the direction indicated by the arrow A in Figure 22 and the mixed solution of the hybridization buffer and the probe solution is forcibly moved in the direction indicated by the arrow A in Figure 22 through only the number of the absorptive regions 4 formed in the substrate 2 of the biochemical analysis unit 1.

When the pair of ultrasonic oscillators 122a, 122b has been oscillated by the controller 123 in this manner a predetermined number of times, thereby forcibly moving the mixed solution of the hybridization buffer and the probe solution accommodated in the reaction vessel 120 in the directions indicated by the arrows A and B in Figure 22 to pass repeatedly through only the number of the absorptive regions 4 formed in the substrate 2 of the biochemical analysis unit 1, the oscillation of the pair of ultrasonic oscillators 122a, 122b is stopped by the controller 123.

In this embodiment, since the mixed solution of the hybridization buffer and the probe solution is forcibly moved through the number the absorptive regions 4 formed in the substrate 2 of the biochemical analysis unit 1 accommodated in the reaction vessel 120 repeatedly in this manner, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in the mixed solution of the hybridization buffer and the probe solution can be hybridized with specific binding substances absorbed in a number of the absorptive regions 4 formed in the substrate 2 of the biochemical analysis unit 1 in a desired manner.

The valve 125 is then opened and the mixed solution of the hybridization buffer and the probe solution accommodated in the reaction vessel 120 is discharged through the solution discharge passage 124 to be collected in a solution collecting tank (not shown),

When the mixed solution of the hybridization buffer and the probe solution has been discharged from the reaction vessel 120, the valve 125 is closed.

Then, the lid member 120a is opened and a cleaning solution is poured into the reaction vessel 120.

When the cleaning solution has been accommodated in the reaction vessel 120, the lid member 120a is closed and the ultrasonic oscillator 122a is oscillated by the controller 123.

As a result, ultrasound is generated in the cleaning solution accommodated in the reaction vessel 120, thereby generating a flow of the cleaning solution is generated in the direction indicated by the arrow A in Figure 22.

Therefore, the cleaning solution is forcibly moved in the direction indicated by the arrow A in Figure 22 to pass through only the number of the absorptive regions 4 formed in the substrate 2 of the biochemical analysis unit 1.

As a result, the number of the absorptive regions 4 formed in the substrate 2 of the biochemical analysis unit 1 is cleaned with the cleaning solution.

It is preferable in order to pass the cleaning solution through only the number of the absorptive regions 4 formed in the substrate 2 of the biochemical analysis unit 1 to generate ultrasound having a frequency of 40 kHz or more and more preferably, ultrasound having a frequency of 400 kHz or more.

When a third predetermined time period shorter than the second predetermined time period has passed, the controller 123 stops the oscillation of the ultrasonic oscillator 122a and oscillates the ultrasonic oscillator 122b.

Since there is a risk of a substance derived from a living organism, which should not be hybridized with specific binding substances and has been peeled off from the absorptive regions 4 by the cleaning operation bonding with the absorptive regions 4 again, if the cleaning solution is repeatedly fed to the absorptive regions 4 of the biochemical analysis unit 1 for a long time, the ultrasonic oscillator 122a is stopped when the third predetermined time period shorter than the second predetermined time period has passed.

As a result, ultrasound is generated in the cleaning solution accommodated in the reaction vessel 120, thereby generating a flow of the cleaning solution is generated in a direction indicated by the arrow B in Figure 22.

Therefore, the cleaning solution is forcibly moved in the direction indicated by the arrow B in Figure 22 through only the number of the absorptive regions 4 formed in the substrate 2 of the biochemical analysis unit 1.

When the third predetermined time period has passed, the controller 123 stops the oscillation of the ultrasonic oscillator 122b and oscillates the ultrasonic oscillator 122a.

As a result, ultrasound is generated in the cleaning solution accommodated in the reaction vessel 120, thereby generating a flow of the cleaning solution in the direction indicated by the arrow A in Figure 22 and the cleaning solution is forcibly moved in the direction indicated by the arrow A in Figure 22 through only the number of the absorptive regions 4 formed in the substrate 2 of the biochemical analysis unit 1.

When the pair of ultrasonic oscillators 122a, 122b has been oscillated by the controller 123 in this manner a predetermined number of times, thereby forcibly moving the cleaning solution accommodated in the reaction vessel 120 in the directions indicated by the arrows A and B in Figure 22 to pass repeatedly through only the number of the absorptive regions 4 formed in the substrate 2 of the biochemical analysis unit 1, the oscillation of the pair of ultrasonic oscillators 122a, 122b is stopped by the controller 123.

In this embodiment, since the cleaning solution is forcibly moved through the number the absorptive regions 4 formed in the substrate 2 of the biochemical analysis unit 1 accommodated in the reaction vessel 120 repeatedly in this manner, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1 and the efficiency of cleaning operation can be markedly improved.

The valve 125 is then opened and the cleaning solution accommodated in the reaction vessel 120 is discharged through the solution discharge passage 124 to be collected in a solution collecting tank (not shown),

When the cleaning solution has been discharged from the reaction vessel 120, the valve 125 is closed.

In this manner, radiation data of a radioactive labeling substance and a fluorescence data of a fluorescent substance such as a fluorescent dye are recorded in a number of the absorptive regions 4 formed in the substrate 2 of the biochemical analysis unit 1.

Similarly to the previously described embodiment, the fluorescence data recorded in a number of the absorptive regions 4 of the biochemical analysis unit 1 are read by the scanner shown in Figures 6 to 13 and biochemical analysis data are produced.

On the other hand, similarly to the previously described embodiment, radiation data recorded in a number of the absorptive regions 4 of the biochemical analysis unit 1 are transferred onto a number of the stimulable phosphor layer regions 17 of the stimulable phosphor sheet 15 shown in Figure 4 and read by the scanner shown in Figures 6 to 13, thereby producing biochemical analysis data.

To the contrary, in order to record chemiluminescence data in a number of the absorptive regions 4 formed in the substrate 2 of the biochemical analysis unit 1, an antibody solution containing an antibody to the hapten such as digoxigenin labeled with an enzyme which generates chemiluminescence emission when it contacts a chemiluminescent substrate is further prepared and poured into the reaction vessel 120 and the antibody to the hapten such as digoxigenin labeled with an enzyme which generates chemiluminescence emission when it contacts a chemiluminescent substrate is bonded with the hapten such as digoxigenin labeling a substance derived from a living organism selectively hybridized with specific binding substances absorbed in a number of the absorptive regions formed in the substrate 2 of the biochemical analysis unit 1 by the an antigen-antibody reaction.

Specifically, an antibody solution containing an antibody to the hapten such as digoxigenin labeled with an enzyme which generates chemiluminescence emission when it contacts a chemiluminescent substrate is first prepared and the lid member 120a is opened to pour the antibody solution into the reaction vessel 120.

When the antibody solution has been accommodated in the reaction vessel 120, the lid member 120a is closed and the ultrasonic oscillator 122a is oscillated by the controller 123.

As a result, ultrasound is generated in the antibody solution accommodated in the reaction vessel 120, thereby generating a flow of the antibody solution is generated in the direction indicated by the arrow A in Figure 22.

Therefore, the cleaning solution is forcibly moved in the direction indicated by the arrow A in Figure 22 through only the number of the absorptive regions 4 formed in the substrate 2 of the biochemical analysis unit 1.

As a result, the antibody to the hapten such as digoxigenin labeled with an enzyme which generates chemiluminescence emission when it contacts a chemiluminescent substrate is bonded with the hapten such as digoxigenin labeling a substance derived from a living organism selectively bonded with specific binding substances absorbed in a number of the absorptive regions formed in the substrate 2 of the biochemical analysis unit 1 by the an antigen-antibody reaction.

It is preferable in order to pass the antibody solution through only the number of the absorptive regions 4 formed in the substrate 2 of the biochemical analysis unit 1 to generate ultrasound having a frequency of 40 kHz or more and more preferably, ultrasound having a frequency of 400 kHz or more is generated.

When a fourth predetermined time period has passed, the controller 123 stops the oscillation of the ultrasonic oscillator 122a and oscillates the ultrasonic oscillator 122b.

As a result, ultrasound is generated in the antibody solution accommodated in the reaction vessel 120, thereby generating a flow of the antibody solution is generated in the direction indicated by the arrow B in Figure 22.

Therefore, the antibody solution is forcibly moved in the direction indicated by the arrow B in Figure 22 through only the number of the absorptive regions 4 formed in the substrate 2 of the biochemical analysis unit 1.

When the fourth predetermined time period has passed, the controller 123 stops the oscillation of the ultrasonic oscillator 122b and oscillates the ultrasonic oscillator 122a.

As a result, ultrasound is generated in the antibody solution accommodated in the reaction vessel 120, thereby generating a flow of the antibody solution is generated in the direction indicated by the arrow A in Figure 22 and the antibody solution is forcibly moved in the direction indicated by the arrow A in Figure 22 through only the number of the absorptive regions 4 formed in the substrate 2 of the biochemical analysis unit 1.

When the pair of ultrasonic oscillators 122a, 122b has been oscillated by the controller 123 in this manner a predetermined number of times, thereby forcibly moving the antibody solution accommodated in the reaction vessel 120 in the directions indicated by the arrows A and B in Figure 22 to pass repeatedly through only the number of the absorptive regions 4 formed in the substrate 2 of the biochemical analysis unit 1, the oscillation of the pair of ultrasonic oscillators 122a, 122b is stopped by the controller 123.

In this embodiment, since the antibody solution is forcibly moved through the number the absorptive regions formed in the substrate 2 of the biochemical analysis unit repeatedly in this manner, it is possible to markedly increase the moving rate of an antibody through the absorptive regions 4 of the biochemical analysis unit 1 and, therefore, the reaction rate of an antigen-antibody reaction can be markedly improved. Further, since it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

The valve 125 is then opened and the antibody solution accommodated in the reaction vessel 120 is discharged through the solution discharge passage 124 to be collected in a solution collecting tank (not shown),

When the antibody solution has been discharged from the reaction vessel 120, the valve 125 is closed.

Then, the lid member 120a is opened and a cleaning solution is poured into the reaction vessel 120.

When the cleaning solution has been accommodated in the reaction vessel 120, the lid member 120a is closed and the ultrasonic oscillator 122a is oscillated by the controller 123.

As a result, ultrasound is generated in the cleaning solution accommodated in the reaction vessel 120, thereby generating a flow of the cleaning solution is generated in the direction indicated by the arrow A in Figure 22.

Therefore, the cleaning solution is forcibly moved in the direction indicated by the arrow A in Figure 22 through only the number of the absorptive regions 4 formed in the substrate 2 of the biochemical analysis unit 1.

As a result, a number of the absorptive regions 4 formed in the substrate 2 of the biochemical analysis unit 1 is cleaned with the cleaning solution.

When a fifth predetermined time period shorter than the fourth predetermined time period has passed, the controller 123 stops the oscillation of the ultrasonic oscillator 122a and oscillates the ultrasonic oscillator 122b.

Since there is a risk of an antibody which should not be bonded with the hapten labeling a substance derived from a living organism selectively hybridized with specific binding substances absorbed in the absorptive regions 4 and has been peeled off from the absorptive regions 4 by the cleaning operation bonding with the absorptive regions 4 again, if the cleaning solution is repeatedly fed to the absorptive regions 4 of the biochemical analysis unit 1 for a long time, the ultrasonic oscillator 122a is stopped when the fifth predetermined time period shorter than the fourth predetermined time period has passed.

As a result, ultrasound is generated in the cleaning solution accommodated in the reaction vessel 120, thereby generating a flow of the cleaning solution is generated in the direction indicated by the arrow B in Figure 22.

Therefore, the cleaning solution is forcibly moved in the direction indicated by the arrow B in Figure 22 through only the number of the absorptive regions 4 formed in the substrate 2 of the biochemical analysis unit 1.

When the fifth predetermined time period has passed, the controller 123 stops the oscillation of the ultrasonic oscillator 122b and oscillates the ultrasonic oscillator 122a.

As a result, ultrasound is generated in the cleaning solution accommodated in the reaction vessel 120, thereby generating a flow of the cleaning solution is generated in the direction indicated by the arrow A in Figure 22 and the cleaning solution is forcibly moved in the direction indicated by the arrow A in Figure 22 through only the number of the absorptive regions 4 formed in the substrate 2 of the biochemical analysis unit 1.

When the pair of ultrasonic oscillators 122a, 122b has been oscillated by the controller 123 in this manner for a predetermined times, thereby forcibly moving the cleaning solution accommodated in the reaction vessel 120 in the directions indicated by the arrows A and B in Figure 22 to pass repeatedly through only the number of the absorptive regions 4 formed in the substrate 2 of the biochemical analysis unit 1, the oscillation of the pair of ultrasonic oscillators 122a, 122b is stopped by the controller 123.

In this embodiment, since the cleaning solution is forcibly moved through a number the absorptive regions 4 formed in the substrate 2 of the biochemical analysis unit 1 accommodated in the reaction vessel 120 repeatedly in this manner, even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1 and the efficiency of cleaning operation can be markedly improved.

The valve 125 is then opened and the cleaning solution accommodated in the reaction vessel 120 is discharged through the solution discharge passage 124 to be collected in a solution collecting tank (not shown),

When the cleaning solution has been discharged from the reaction vessel 120, the valve 125 is closed.

In this manner, chemiluminescence data are recorded in a number of the absorptive regions 4 formed in the substrate 2 of the biochemical analysis unit 1.

Chemiluminescence data recorded in a number of the absorptive regions 4 of the biochemical analysis unit 1 are transferred onto a number of the stimulable phosphor layer regions 77 of the biochemical analysis unit 75 shown in Figure 14 and read by the scanner shown in Figures 15 to 17 or read by the cooled CCD camera 81 of the data producing system shown in Figures 18 to 21, thereby producing biochemical analysis data.

According to this embodiment, since the ultrasonic oscillators 122a, 122b are alternately oscillated, thereby generating ultrasound in the mixed solution of the hybridization buffer and the probe solution and the mixed solution of the hybridization buffer and the probe solution is forcibly moved by the thus generated ultrasound to pass through the number of the absorptive regions 4 of the biochemical analysis unit 1 set in the reaction vessel 120, thereby performing hybridization, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Further, according to this embodiment, since the ultrasonic oscillators 122a, 122b are alternately oscillated, thereby alternately generating ultrasounds whose orientations are different from each other in the mixed solution of the hybridization buffer and the probe solution and the mixed solution of the hybridization buffer and the probe solution is forcibly moved alternately in different directions by the thus generated ultrasounds to pass through a number of the absorptive regions 4 of the biochemical analysis unit 1 set in the reaction vessel 120 repeatedly, it is possible to much more improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1 and therefore, a substance derived from a living organism and contained in mixed solution of the hybridization buffer and the probe solution can be hybridized with specific binding substances absorbed in a number of the absorptive regions 4 formed in the substrate 2 of the biochemical analysis unit 1 in a desired manner.

Furthermore, according to this embodiment, since the ultrasonic oscillators 122a, 122b are alternately oscillated, thereby generating ultrasound in the antibody solution and the antibody solution is forcibly moved by the thus generated ultrasound to pass through the number of the absorptive regions 4 of the biochemical analysis unit 1 set in the reaction vessel 120, thereby performing a receptor-ligand association reaction, it is possible to markedly increase the moving rate of an antibody through the absorptive regions 4 of the biochemical analysis unit 1 and, therefore, the reaction rate of an antigen-antibody reaction can be markedly improved. Further, since it is possible to markedly improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Moreover, according to this embodiment, since the ultrasonic oscillators 122a, 122b are alternately oscillated, thereby alternately generating ultrasounds whose orientations are different from each other in the antibody solution and the antibody solution is forcibly moved alternately in different directions by the thus generated ultrasounds to pass through the number of the absorptive regions 4 of the biochemical analysis unit 1 set in the reaction vessel 120 repeatedly, it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1 and therefore, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Furthermore, according to this embodiment, since the ultrasonic oscillators 122a, 122b are alternately oscillated, thereby generating ultrasound in the cleaning solution and the cleaning solution is forcibly moved by the thus generated ultrasound to pass through the number of the absorptive regions 4 of the biochemical analysis unit 1 set in the reaction vessel 120, thereby performing the cleaning operation, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1 and even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1. Therefore, the efficiency of cleaning operation can be markedly improved

Moreover, according to this embodiment, since the ultrasonic oscillators 122a, 122b are alternately oscillated, thereby alternately generating ultrasounds whose orientations are different from each other in the cleaning solution and the cleaning solution is forcibly moved alternately in different directions by the thus generated ultrasounds to pass through the number of the absorptive regions 4 of the biochemical analysis unit 1 set in the reaction vessel 120 repeatedly, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to much more efficiently peel off and remove the substance derived from a living organism which should not be hybridized with specific binding substances absorbed in the absorptive regions 4 from a number of the absorptive regions 4 of the biochemical analysis unit 1 and even if an antibody which should not be bonded with the hapten labeling a substance derived from a living body and selectively hybridized with specific binding substances absorbed in the absorptive regions 4 of the biochemical analysis unit 1 has been bonded with the absorptive regions 4 formed in the substrate 2 of the biochemical analysis unit 1, it is possible to very efficiently peel off and remove the antibody which should not be bonded with the hapten from a number of the absorptive regions 4 of the biochemical analysis unit 1. Therefore, the efficiency of cleaning operation can be markedly improved.

Furthermore, according to this embodiment, since the ultrasonic oscillators 122a, 122b are alternately oscillated, thereby generating ultrasound in the mixed solution of the hybridization buffer and the probe solution, the mixed solution of the hybridization buffer and the probe solution is forcibly moved by the thus generated ultrasound to pass through the number of the absorptive regions 4 of the biochemical analysis unit 1 set in the reaction vessel 120, thereby performing hybridization, the ultrasonic oscillators 122a, 122b are alternately oscillated, thereby generating ultrasound in the cleaning solution and the cleaning solution is forcibly moved by the thus generated ultrasound to pass through the number of the absorptive regions 4 of the biochemical analysis unit 1 set in the reaction vessel 120, thereby performing the cleaning operation, even if a different experimenter performs hybridization, it is possible to reliably prevent different results from being obtained and the repeatability of hybridization can be markedly improved.

Moreover, according to this embodiment, since the ultrasonic oscillators 122a, 122b are alternately oscillated, thereby generating ultrasound in the antibody solution, the antibody solution is forcibly moved by the thus generated ultrasound to pass through the number of the absorptive regions 4 of the biochemical analysis unit 1 set in the reaction vessel 120, thereby performing an antigen-antibody reaction, the ultrasonic oscillators 122a, 122b are alternately oscillated, thereby generating ultrasound in the cleaning solution and the cleaning solution is forcibly moved by the thus generated ultrasound to pass through the number of the absorptive regions 4 of the biochemical analysis unit 1 set in the reaction vessel 120, thereby performing the cleaning operation, even if a different experimenter performs an antigen-antibody reaction, it is possible to reliably prevent different results from being obtained and the repeatability of an antigen-antibody reaction can be markedly improved.

Furthermore, according to this embodiment, since a number of the absorptive regions 4 of the biochemical analysis unit 1 are formed by charging nylon-6 in a number of the through-holes formed in the substrate 2 made of stainless steel, the hybridization buffer, the mixed solution of the hybridization buffer and the probe solution and the cleaning solution are forcibly fed to the biochemical analysis unit 1 so as to pass through only the number of the absorptive regions 4 formed in the substrate 2 of the biochemical analysis unit 1 and, therefore, the efficiency of hybridization, the efficiency of an antigen-antibody reaction and the efficiency of the cleaning operation can be markedly improved.

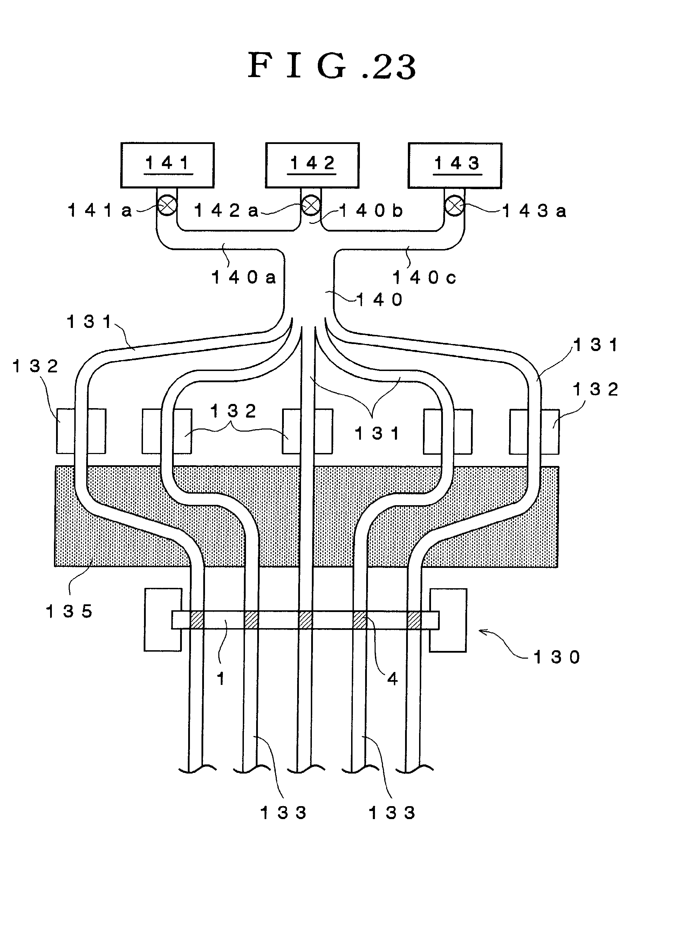

Figure 23 is a schematic longitudinal cross sectional view showing an apparatus for conducting a receptor-ligand association reaction which is a further preferred embodiment of the present invention.

As shown in Figure 23, the apparatus for conducting a receptor-ligand association reaction according to this embodiment includes a stage 130 for holding the biochemical analysis unit 1, a number of capillaries 131 disposed so that the opening end portion of each of them faces one of a number of the absorptive regions 4 formed in the substrate 2 of the biochemical analysis unit 1 held by the stage 130, temperature control units 132 provided for one for each of the capillaries 131 and adapted for controlling the temperature of a solution flowing in the corresponding capillary 132, solution collecting capillaries 133 disposed so that the opening end portion of each of them faces one of the opening end portions of a number of the capillaries 131 via the corresponding absorptive region 4 of the biochemical analysis unit 1 held by the stage 130, and a thermal isolation unit 135 formed of a thermal insulating material.

Although not shown in Figure 23, each of the temperature control units 132 includes a heater and a Peltier element.

As shown in Figure 23, each of a number of the capillaries 131 between the temperature control units 132 and the stage 130 is disposed pass through the interior of the thermal isolation unit 135.

As shown in Figure 23, an end portion of each of the capillaries 131 opposite to the opening end portion is connected to a solution feeding conduit 140 and the solution feeding conduit 140 is bifurcated to a first branch conduit 140a, a second branch conduit 140b and a third branch conduit 140c.

The first branch conduit 140a is connected to a hybridization buffer tank 141 for accommodating a hybridization buffer and the second branch conduit 140b is connected to an antibody solution tank 142 for accommodating an antibody solution.

Further, the third branch conduit 140c is connected to a cleaning solution tank 143 for accommodating a cleaning solution.

The first branch conduit 140a, the second branch conduit 140b and the third branch conduit 140c are provided with valves 141a, 142a and 143a, respectively.

In the thus constituted apparatus for conducting receptor-legand association, a substance derived from a living body, labeled with a labeling substance and contained in a probe solution selectively hybridizes specific binding substances contained in the number of the absorptive regions 4 of the biochemical analysis unit 1 in the following manner.

The biochemical analysis unit 1 formed with the number of the absorptive regions 4 in which specific binding substances are absorbed is first set on the stage 130 and a hybridization buffer is prepared and accommodated in the hybridization buffer tank 141.

The control temperature for each of the temperature control units 132 is set.

In this embodiment, since the kinds of specific binding substances absorbed in the individual absorptive regions 4 of the biochemical analysis unit 1 are known, the control temperature for each of the temperature control units 132 is set in accordance with the kind of specific binding substances absorbed in each of the absorptive regions 4.

Although a target DNA having the corresponding chain length to that of a probe DNA absorbed in the absorptive region 4 of the biochemical analysis unit 1 is hybridized with the probe DNA by hybridization, in the case where the chain length of a probe DNA absorbed in the absorptive region 4 is long, a target DNA once hybridized with the probe DNA is apt to be peel off from the probe DNA unless the reaction temperature of hybridization is controlled to be high. On the other hand, in the case where the chain length of a probe DNA absorbed in the absorptive region 4 is short, a target DNA once hybridized with the probe DNA is apt to be peel off from the probe DNA unless the reaction temperature of hybridization is controlled to be low. Therefore, in this embodiment, in the case where the chain length of a probe DNA absorbed in the absorptive region 4 of the biochemical analysis unit 1 is long, the control temperature of the temperature control unit 132 for corresponding capillary 131 is set high and in the case where the chain length of a probe DNA absorbed in the absorptive region 4 of the biochemical analysis unit 1 is short, the control temperature of the temperature control unit 132 for corresponding capillary 131 is set low.

The valve 141a is then opened, while the valve 142a and the valve 143 a are held closed.

As a result, a hybridization buffer accommodated in the hybridization buffer tank 141 is fed to each of the capillaries 131 via the first branch conduit 140a and the solution feeding conduit 140.

The temperature of the hybridization buffer fed to each of the capillaries 131 is controlled by the corresponding temperature control unit 132 to a predetermined temperature and the hybridization buffer is fed to the corresponding absorptive region 4 of the biochemical analysis unit 1 held by the stage 130.

As a result, pre-hybridization is performed.

The hybridization buffer passes through the corresponding absorptive region 4 of the biochemical analysis unit 1 and flows into the corresponding solution collecting capillary 133, thereby being collected.

The thus collected hybridization buffer is recycled to the hybridization buffer tank 141 and similarly to the above, pre-hybridization is performed for a predetermined time period.

When pre-hybridization has been completed in this manner, a probe solution is prepared and mixed with the hybridization buffer accommodated in the hybridization buffer tank 141.

In this embodiment, a probe solution containing a substance derived from a living organism and labeled with a radioactive labeling substance, a substance derived from a living organism and labeled with a fluorescent substance such as a fluorescent dye and a substance derived from a living organism and labeled with a hapten such as digoxigenin is prepared and mixed with the hybridization buffer accommodated in the hybridization buffer tank 141.

As a result, the mixed solution of the hybridization buffer and the probe solution is fed to each of the capillaries 131 via the first branch conduit 140a and the solution feeding conduit 140.

The temperature of the mixed solution of the hybridization buffer and the probe solution fed to each of the capillaries 131 is controlled by the corresponding temperature control unit 132 to a predetermined temperature and the mixed solution of the hybridization buffer and the probe solution is fed to the corresponding absorptive region 4 of the biochemical analysis unit 1 held by the stage 130.

As a result, a substance derived from a living organism and contained in the mixed solution of the hybridization buffer and the probe solution selectively hybridizes with specific binding substances absorbed in a number of the absorptive regions 4 formed in the substrate 2 of the biochemical analysis unit 1.

In this embodiment, since the temperature of the mixed solution of the hybridization buffer and the probe solution fed via the capillaries 131 is controlled by the corresponding temperature control unit 132 in accordance with the kind of specific binding substance absorbed in the corresponding absorptive region 4 of the biochemical analysis unit 1, it is possible to effectively prevent a substance derived from a living organism and once hybridized with the specific binding substance from being peeled off from the specific binding substance and, therefore, the accuracy of biochemical analysis can be improved.

The mixed solution of the hybridization buffer and the probe solution passes through the corresponding absorptive region 4 of the biochemical analysis unit 1 and flows into the corresponding solution collecting capillary 133, thereby being collected.

The thus collected mixed solution of the hybridization buffer and the probe solution is recycled to the hybridization buffer tank 141 and similarly to the above, pre-hybridization is performed for a predetermined time period.

In this embodiment, since the mixed solution of the hybridization buffer and the probe solution is fed through a number the absorptive regions 4 formed in the substrate 2 of the biochemical analysis unit 1 repeatedly in this manner, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in the mixed solution of the hybridization buffer and the probe solution can be hybridized with specific binding substances absorbed in a number of the absorptive regions 4 formed in the substrate 2 of the biochemical analysis unit 1 in a desired manner.

When a predetermined time period has been passed in this manner and hybridization has been completed, the valve 141a is closed and the cleaning operation is started in order to peel off and remove a substance derived from a living organism which has been bonded with any specific binding substance absorbed in the absorptive regions 4 of the biochemical analysis unit 1 although it should not be hybridized with the specific binding substance from the absorptive region 4 of the biochemical analysis unit 1.

When the cleaning operation is to be performed, a cleaning solution is prepared and accommodated in the cleaning solution tank 143.

The valve 143a is then opened and the cleaning solution accommodated in the cleaning solution tank 143 is fed to each of the capillaries 131 via the first branch conduit 143a and the solution feeding conduit 140.

When the cleaning operation is performed, similarly to the case where hybridization was performed, the control temperature for each of the temperature control units 132 is set in accordance with the kind of specific binding substance absorbed in each of the absorptive regions 4 and the temperature of the cleaning solution flowing in each of the capillaries 131 is controlled.

In this embodiment, since the temperatures of the hybridization buffer, the mixed solution of the hybridization buffer and the probe solution and the cleaning solution to be fed via each of the capillaries 131 are controlled by the corresponding temperature control unit 132 in accordance with the kind of specific binding substance absorbed in the corresponding absorptive region 4 of the biochemical analysis unit 1, it is possible to effectively prevent a substance derived from a living organism and hybridized with a specific binding substance absorbed in each of the absorptive regions 4 of the biochemical analysis unit 1, which is to be hybridized with the specific binding substance, from being peeled off from the specific binding substance and, on the other hand, even if a substance derived from a living organism which should not be hybridized with a specific binding substance absorbed in each of the absorptive regions 4 of the biochemical analysis unit 1 has been bonded with the absorptive regions 4 during the process of hybridization, it is possible to effectively peel off and remove the substance derived from a living organism and bonded with the specific binding substance therefrom by cleaning each of the absorptive regions 4 of the biochemical analysis unit 1 with the cleaning solution whose temperature is controlled to the temperature optimum for each of the absorptive regions 4. Therefore, it is possible to hybridize a substance derived from a living organism with specific binding substances absorbed in the individual absorptive regions of the biochemical analysis unit 1 with which the substance derived from a living organism is to be hybridized in a desired manner.

The cleaning solution passes through each of the absorptive regions 4 of the biochemical analysis unit 1 and flows into the corresponding solution collecting capillaries 133.

In this embodiment, since the cleaning solution is fed through a number the absorptive regions 4 formed in the substrate 2 of the biochemical analysis unit 1, thereby performing the cleaning of the absorptive regions 4 of the biochemical analysis unit 1, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1.

The cleaning solution is collected without being recycled to the cleaning solution tank 143 for preventing a substance derived from a living organism, which was once bonded with a specific binding substance absorbed in the absorptive region 4 of the biochemical analysis unit 1 although it should not be hybridized therewith and has been peeled off therefrom by the cleaning solution from again bonding with the specific binding substance absorbed in the absorptive region 4 of the biochemical analysis unit 1.

When a predetermined time period has passes, the valve 143a is closed and the cleaning operation is completed.

In this manner, radiation data of a radioactive labeling substance and a fluorescence data of a fluorescent substance such as a fluorescent dye are recorded in a number of the absorptive regions 4 formed in the substrate 2 of the biochemical analysis unit 1.

Similarly to the previously described embodiments, the fluorescence data recorded in a number of the absorptive regions 4 of the biochemical analysis unit 1 are read by the scanner shown in Figures 6 to 13 and biochemical analysis data are produced.

On the other hand, similarly to the previously described embodiments, radiation data recorded in a number of the absorptive regions 4 of the biochemical analysis unit 1 are transferred onto a number of the stimulable phosphor layer regions 17 of the stimulable phosphor sheet 15 shown in Figure 4 and read by the scanner shown in Figures 6 to 13, thereby producing biochemical analysis data.

To the contrary, in order to record chemiluminescence data in a number of the absorptive regions 4 formed in the substrate 2 of the biochemical analysis unit 1, an antibody solution containing an antibody to the hapten such as digoxigenin labeled with an enzyme which generates chemiluminescence emission when it contacts a chemiluminescent substrate is further prepared and fed to each of the absorptive regions 4 of the biochemical analysis unit 1 held by the stage 130 via the corresponding capillary 131 and the antibody to the hapten such as digoxigenin labeled with an enzyme which generates chemiluminescence emission when it contacts a chemiluminescent substrate is bonded with the hapten such as digoxigenin labeling a substance derived from a living organism selectively hybridized with specific binding substances absorbed in a number of the absorptive regions formed in the substrate 2 of the biochemical analysis unit 1 by the an antigen-antibody reaction.

Specifically, an antibody solution containing an antibody to the hapten such as digoxigenin labeled with an enzyme which generates chemiluminescence emission when it contacts a chemiluminescent substrate is first prepared and accommodated in the antibody solution tank 142.

The valve 142a is then opened while the valve 141a and the valve 142 a are held to be closed.

As a result, the antibody solution is fed to each of the capillaries 131 via the first branch conduit 142a and the solution feeding conduit 140.

The temperature of the antibody solution fed to each of the capillaries 131 is controlled by the corresponding temperature control unit 132 to a predetermined temperature and the antibody solution is fed to the corresponding absorptive region 4 of the biochemical analysis unit 1 held by the stage 130. In this embodiment, the control temperature of each of the temperature control units 132 is determined in accordance with the kind of hapten labeling a substance derived from a living organism and selectively hybridized with a specific binding substance absorbed in the corresponding absorptive region 4 of the biochemical analysis unit 1.

As a result, the antibody to the hapten such as digoxigenin labeled with an enzyme which generates chemiluminescence emission when it contacts a chemiluminescent substrate is bonded with the hapten such as digoxigenin labeling a substance derived from a living organism selectively bonded with specific binding substances absorbed in a number of the absorptive regions formed in the substrate 2 of the biochemical analysis unit 1 by the an antigen-antibody reaction.

In this embodiment, since the temperatures of the antibody solution to be fed via each of the capillaries 131 are controlled by the corresponding temperature control unit 132 in accordance with the kind of hapten labeling a substance derived from a living organism and selectively hybridized with a specific binding substance absorbed in the corresponding absorptive region 4 of the biochemical analysis unit 1, it is possible to effectively prevent an antibody for the hapten labeled with an enzyme which generates chemiluminescence emission when it contacts a chemiluminescent substrate and once bonded with the hapten labeling a substance derived from a living organism and selectively hybridized with a specific binding substance absorbed in the corresponding absorptive region 4 of the biochemical analysis unit 1 by an antigen-antibody reaction from being peeled off from the hapten and, therefore, the accuracy of biochemical analysis can be improved.

The antibody solution passes through the corresponding absorptive region 4 of the biochemical analysis unit 1 and flows into the corresponding solution collecting capillary 133, thereby being collected.

The thus collected antibody solution is recycled to the antibody solution tank 142 and similarly to the above, an antigen-antibody reaction is performed for a predetermined time period.

In this embodiment, since the antibody solution is moved through a number the absorptive regions 4 formed in the substrate 2 of the biochemical analysis unit repeatedly in this manner, it is possible to markedly increase the moving rate of an antibody through the absorptive regions 4 of the biochemical analysis unit 1 and, therefore, the reaction rate of an antigen-antibody reaction can be markedly improved. Further, since it is possible to much more improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

When a predetermined time period has passed in this manner and an antigen-antibody reaction has been completed, the valve 142a is closed and the cleaning operation is started for peeling off and removing from the hapten antibody for the hapten which has been bonded with the hapten labeling a substance derived from a living organism selectively hybridized with specific binding substances absorbed in a number of the absorptive regions 4 of the biochemical analysis unit 1 although it should not be bonded with the hapten.

When the cleaning operation is to be performed, the valve 143a is then opened and the cleaning solution accommodated in the cleaning solution tank 143 is fed to each of the capillaries 131 via the first branch conduit 143a and the solution feeding conduit 140.

When the cleaning operation is performed, similarly to the case where an antigen-antibody reaction was performed, the control temperature for each of the temperature control units 132 is set in accordance with the kind of the hapten labeling a substance derived from a living organism selectively hybridized with a specific binding substance absorbed in each of the absorptive regions 4 of the biochemical analysis unit 1.

In this embodiment, since the temperatures of the cleaning solution to be fed via each of the capillaries 131 are controlled by the corresponding temperature control unit 132 in accordance with the kind of the hapten labeling a substance derived from a living organism selectively hybridized with a specific binding substance absorbed in each of the absorptive regions 4 of the biochemical analysis unit 1, it is possible to effectively prevent an antibody for the hapten bonded with the hapten labeling a substance derived from a living organism selectively hybridized with a specific binding substance absorbed in each of the absorptive regions 4 of the biochemical analysis unit 1, which is to be bonded with the hapten by an antigen-antibody reaction, from being peeled off and, on the other hand, even if an antibody which should not be bonded with the hapten labeling a substance derived from a living body and selectively hybridized with a specific binding substance absorbed in each of the absorptive regions 4 of the biochemical analysis unit 1 has been bonded with the absorptive region of the biochemical analysis unit, it is possible to effectively peel off and remove an antibody for the hapten from the absorptive region 4 of the biochemical analysis unit 1 by cleaning each of the absorptive regions 4 of the biochemical analysis unit 1 with the cleaning solution whose temperature is controlled to the temperature optimum for each of the absorptive regions 4. Therefore, it is possible to bind an antibody for the hapten to be bonded with the hapten labeling a substance derived from a living body and selectively hybridized with a specific binding substance absorbed in each of the absorptive regions 4 of the biochemical analysis unit 1 with the hapten by an antigen-antibody reaction in a desired manner.

The cleaning solution passes through the corresponding absorptive region 4 of the biochemical analysis unit 1 and flows into the solution collecting conduit 133.

The cleaning solution is collected without being recycled to the cleaning solution tank 143 so as to prevent an antibody for the hapten, which was once bonded with the hapten labeling a substance derived from a living body and selectively hybridized with a specific binding substance absorbed in each of the absorptive regions 4 of the biochemical analysis unit 1 although it should not be bonded with the hapten and has been peeled off therefrom by the cleaning solution, from again bonding with the hapten labeling a substance derived from a living body and selectively hybridized with a specific binding substance absorbed in each of the absorptive regions 4 of the biochemical analysis unit 1.

When a predetermined time period has passes, the valve 143a is closed and the cleaning operation is completed.

In this manner, chemiluminescence data are recorded in a number of the absorptive regions 4 formed in the substrate 2 of the biochemical analysis unit 1.

Chemiluminescence data recorded in a number of the absorptive regions 4 of the biochemical analysis unit 1 are transferred onto a number of the stimulable phosphor layer regions 77 of the biochemical analysis unit 75 shown in Figure 14 and read by the scanner shown in Figures 15 to 17 or read by the cooled CCD camera 81 of the data producing system shown in Figures 18 to 21, thereby producing biochemical analysis data.

According to this embodiment, since the mixed solution of the hybridization buffer and the probe solution is forcibly moved through a number the absorptive regions 4 formed in the substrate 2 of the biochemical analysis unit 1 repeatedly in this manner, it is possible to markedly increase the moving rate of a substance derived from a living organism through the absorptive regions 4 of the biochemical analysis unit 1 in comparison with the case where a substance derived from a living organism and contained in the mixed solution of the hybridization buffer and the probe solution is moved only by convection or diffusion to be hybridized with specific binding substances absorbed in a number of the absorptive regions 4 of the biochemical analysis unit 1 and, therefore, the reaction rate of hybridization can be markedly improved. Further, since it is possible to markedly improve the possibility of a substance derived from a living organism and contained in mixed solution of the hybridization buffer and the probe solution associating with specific binding substances absorbed in deep portions of a number of the absorptive regions 4 formed in the substrate 2 of the biochemical analysis unit 1, a substance derived from a living organism and contained in the mixed solution of the hybridization buffer and the probe solution can be hybridized with specific binding substances absorbed in a number of the absorptive regions 4 formed in the substrate 2 of the biochemical analysis unit 1 in a desired manner.

Moreover, according to this embodiment, since the temperatures of the hybridization buffer, the mixed solution of the hybridization buffer and the probe solution and the cleaning solution to be fed via each of the capillaries 131 are controlled by the corresponding temperature control unit 132 in accordance with the kind of specific binding substance absorbed in the corresponding absorptive region 4 of the biochemical analysis unit 1, it is possible to effectively prevent a substance derived from a living organism and hybridized with a specific binding substance absorbed in each of the absorptive regions 4 of the biochemical analysis unit 1, which is to be hybridized with the specific binding substance, from being peeled off from the specific binding substance and, on the other hand, even if a substance derived from a living organism which should not be hybridized with a specific binding substance absorbed in each of the absorptive regions 4 of the biochemical analysis unit 1 has been bonded with the absorptive regions 4 during the process of hybridization, it is possible to effectively peel off and remove the substance derived from a living organism and bonded with the specific binding substance therefrom by cleaning each of the absorptive regions 4 of the biochemical analysis unit 1 with the cleaning solution whose temperature is controlled to temperature optimum to each of the absorptive regions 4. Therefore, since it is possible to hybridize a substance derived from a living organism with a specific binding substances absorbed in the individual absorptive regions of the biochemical analysis unit 1 with which the substance derived from a living organism is to be hybridized in a desired manner, it is possible to effectively prevent noise from being generated in biochemical analysis data and to produce biochemical analysis data having an excellent quantitative characteristic.

Furthermore, according to this embodiment, since the cleaning solution is forcibly fed through the number the absorptive regions 4 formed in the substrate 2 of the biochemical analysis unit 1, thereby performing the cleaning of the absorptive regions 4 of the biochemical analysis unit 1, even if a substance derived from a living organism which should not be hybridized with specific binding substances absorbed in the absorptive regions 4 formed in the substrate 2 of the biochemical analysis unit 1 has been bonded with the absorptive regions 4 during the process of hybridization, it is possible to very efficiently peel off and remove the substance derived from a living organism which should not be hybridized with specific binding substances absorbed in the absorptive regions 4 from a number of the absorptive regions 4 of the biochemical analysis unit 1.

Moreover, according to this embodiment, since the antibody solution is fed through a number the absorptive regions 4 formed in the substrate 2 of the biochemical analysis unit 1, thereby performing an antigen-antibody reaction, it is possible to markedly increase the moving rate of an antibody through the absorptive regions 4 of the biochemical analysis unit 1 and, therefore, the reaction rate of an antigen-antibody reaction can be markedly improved. Further, since it is possible to markedly improve the possibility of an antibody for the hapten contained in the antibody solution associating with the hapten labeling a substance derived from a living organism selectively hybridized with specific binding substances absorbed in deep portions of a number of the absorptive regions 4 formed in the substrate 2 of the biochemical analysis unit 1, an antibody for the hapten contained in the antibody solution can be associated with the hapten labeling a substance derived from a living organism selectively hybridized with specific binding substances absorbed in a number of the absorptive regions 4 formed in the substrate 2 of the biochemical analysis unit 1 in a desired manner.

Furthermore, according to this embodiment, since the temperatures of the antibody solution to be fed via each of the capillaries 131 are controlled by the corresponding temperature control unit 132 in accordance with the kind of hapten labeling a substance derived from a living organism and selectively hybridized with a specific binding substance absorbed in the corresponding absorptive region 4 of the biochemical analysis unit 1, it is possible to effectively prevent an antibody for the hapten labeled with an enzyme which generates chemiluminescence emission when it contacts a chemiluminescent substrate and once bonded with the hapten labeling a substance derived from a living organism and selectively hybridized with a specific binding substance absorbed in the corresponding absorptive region 4 of the biochemical analysis unit 1 by an antigen-antibody reaction from being peeled off from the hapten and, therefore, the accuracy of biochemical analysis can be improved.

Moreover, according to this embodiment, since the temperature of the cleaning solution to be fed via each of the capillaries 131 are controlled by the corresponding temperature control unit 132 in accordance with the kind of the hapten labeling a substance derived from a living organism selectively hybridized with a specific binding substance absorbed in each of the absorptive regions 4 of the biochemical analysis unit 1, it is possible to effectively prevent an antibody for the hapten bonded with the hapten labeling a substance derived from a living organism selectively hybridized with a specific binding substance absorbed in each of the absorptive regions 4 of the biochemical analysis unit 1, which is to be bonded with the hapten by an antigen-antibody reaction, from being peeled off and, on the other hand, even if an antibody which should not be bonded with the hapten labeling a substance derived from a living body and selectively hybridized with a specific binding substance absorbed in each of the absorptive regions 4 of the biochemical analysis unit 1 has been bonded with the absorptive region of the biochemical analysis unit, it is possible to effectively peel off and remove an antibody for the hapten from the absorptive region 4 of the biochemical analysis unit 1 by cleaning each of the absorptive regions 4 of the biochemical analysis unit 1 with the cleaning solution whose temperature is controlled to temperature optimum to each of the absorptive regions 4. Therefore, it is possible to bind an antibody for the hapten to be bonded with the hapten labeling a substance derived from a living body and selectively hybridized with a specific binding substance absorbed in each of the absorptive regions 4 of the biochemical analysis unit 1 with the hapten by an antigen-antibody reaction in a desired manner.

Furthermore, according to this embodiment, since a number of the absorptive regions 4 of the biochemical analysis unit 1 are formed by charging nylon-6 in a number of the through-holes formed in the substrate 2 made of stainless steel, the hybridization buffer, the mixed solution of the hybridization buffer and the probe solution and the cleaning solution are forcibly fed to the biochemical analysis unit 1 so as to pass through only the number of the absorptive regions 4 formed in the substrate 2 of the biochemical analysis unit 1 and, therefore, the efficiency of hybridization, the efficiency of an antigen-antibody reaction and the efficiency of the cleaning operation can be markedly improved.

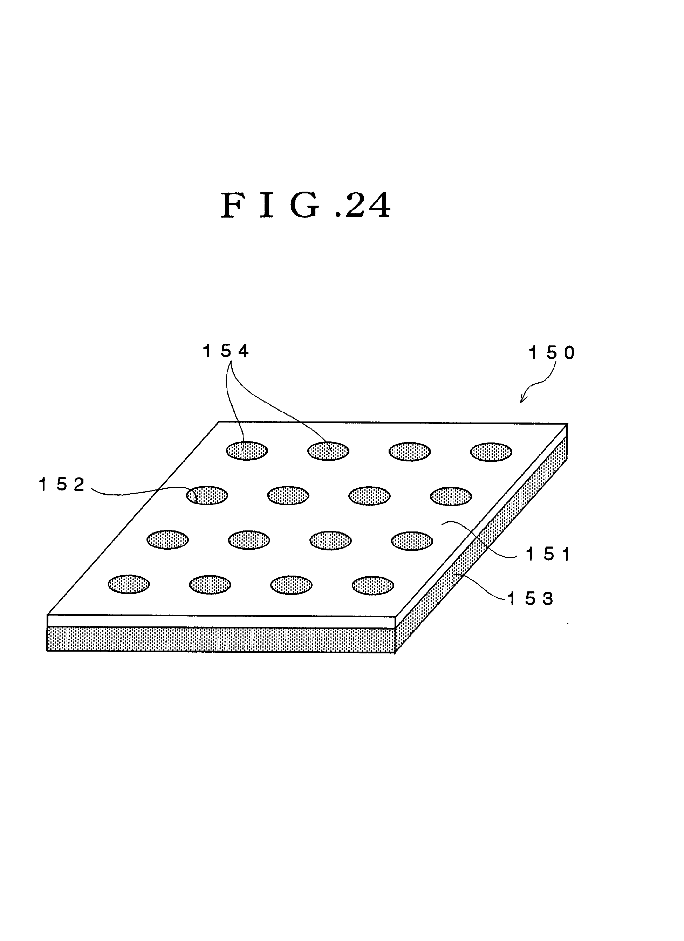

Figure 24 is a schematic perspective view showing another example of a biochemical analysis unit.

As shown in Figure 24, the biochemical analysis unit 150 includes a substrate 151 made of stainless steel and formed with a number of through-holes 152 and a number of absorptive regions 154 are dot-like formed in a regular pattern by pressing an absorptive membrane 153 containing nylon-6 into a number of the through-holes 152 using a calender processing apparatus (not shown).

Although not accurately shown in Figure 24, in this embodiment, about 10,000 absorptive regions 154 having a size of about 0.01 mm² are regularly formed at a density of about 5,000 per cm² in the biochemical analysis unit 1.

The biochemical analysis unit 1 is formed by pressing the absorptive membrane 153 into a number of the through-holes 152 in such a manner that the surfaces of the absorptive regions 154 are located at the same height level as that of the substrate 152.

In this embodiment, similarly to the biochemical analysis unit 1 shown in Figure 1, a solution containing specific binding substances such as cDNAs is spotted using the spotting device shown in Figure 2 onto a number of the absorptive regions 154 formed in the biochemical analysis unit 150 and the specific binding substances are absorbed in a number of the absorptive regions 154.

Since a number of the absorptive regions 104 of the biochemical analysis unit 150 shown in Figure 24 are formed by pressing the absorptive membrane 153 into a number of the through-holes 152 formed in the substrate 151, cavities in the absorptive membrane 153 have been eliminated by the pressing operation in regions between neighboring absorptive regions 154. Therefore, a solution of specific binding substances spotted in the absorptive regions 154 can be effectively prevented from permeating the absorptive membrane 153 and the specific binding substances are absorbed only in the absorptive regions 154.

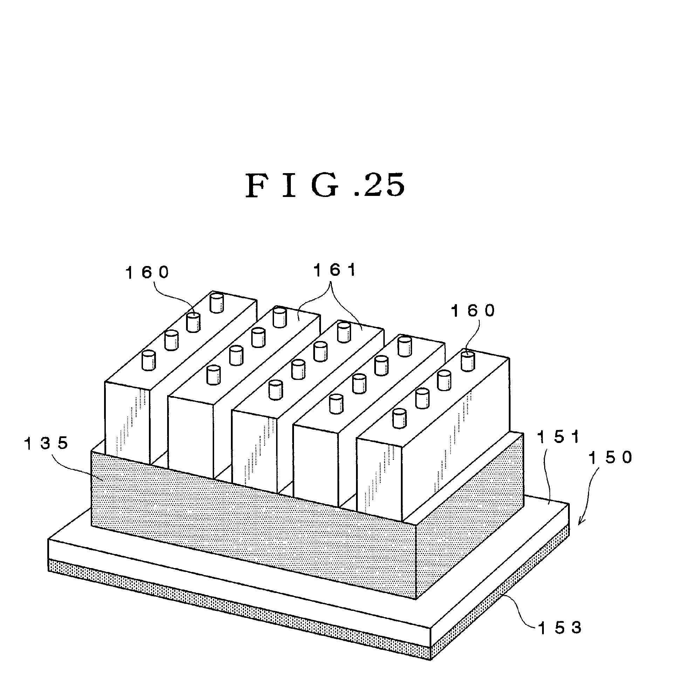

Figure 25 is a schematic perspective view showing an apparatus for conducting a receptor-ligand association reaction which is another preferred embodiment of the present invention.

As shown in Figure 25, in the apparatus for conducting a receptor-ligand association reaction according to this embodiment, a separate temperature control unit 161 is provided for the plurality of capillaries 160 constituting each line of the capillaries 160.

In this embodiment, a solution containing the same kind of specific binding substance is spotted onto the absorptive regions 154 constituting one line of the absorptive regions 154 and the temperatures of a hybridization buffer, a mixed solution of a hybridization buffer and a probe solution and a cleaning solution flowing through the capillaries 160 are controlled for the plurality of capillaries 160 constituting each line of the capillaries 160.

Therefore, according to this embodiment, since the temperature of the mixed solution of the hybridization buffer and the probe solution fed via each line of the capillaries 160 can be controlled by the corresponding temperature control unit 161 in accordance with the kind of specific binding substance absorbed in the absorptive regions 154 constituting each line of the absorptive regions 154, it is possible to effectively prevent a substance derived from a living organism and once hybridized with the specific binding substance from being peeled off from the specific binding substance and, therefore, the accuracy of biochemical analysis can be improved.

Furthermore, according to this embodiment, since the temperature of the cleaning solution to be fed via each line of the capillaries 160 can be controlled by the corresponding temperature control unit 161 in accordance with the kind of specific binding substance absorbed in the absorptive regions 154 constituting each line of the absorptive regions 154, it is possible to effectively prevent a substance derived from a living organism and hybridized with a specific binding substance absorbed in each of the absorptive regions 154 constituting each line of the absorptive regions 154 of the biochemical analysis unit 150, which is to be hybridized with the specific binding substance, from being peeled off from the specific binding substance and, on the other hand, even if a substance derived from a living organism which should not be hybridized with a specific binding substance absorbed in each of the absorptive regions 154 constituting each line of the absorptive regions 154 of the biochemical analysis unit 150 has been bonded with one or more absorptive regions 154 among the absorptive regions 154 constituting each line of the absorptive regions 154 during the process of hybridization, it is possible to effectively peel off and remove the substance derived from a living organism and bonded with the specific binding substance therefrom by cleaning the absorptive regions 154 constituting each line of the absorptive regions 154 of the biochemical analysis unit 150 with the cleaning solution whose temperature is controlled to the temperature optimum for each line of the capillaries 160. Therefore, it is possible to hybridize a substance derived from a living organism with a specific binding substances absorbed in the individual absorptive regions of the biochemical analysis unit 1 with which the substance derived from a living organism is to be hybridized in a desired manner.

Moreover, according to this embodiment, since the temperatures of the antibody solution to be fed via each line of the capillaries 160 can be controlled by the corresponding temperature control unit 161 in accordance with the kind of hapten labeling a substance derived from a living organism and selectively hybridized with a specific binding substance absorbed in the absorptive regions 154 constituting each line of the absorptive regions 154, it is possible to effectively prevent an antibody for the hapten labeled with an enzyme which generates chemiluminescence emission when it contacts a chemiluminescent substrate and once bonded with the hapten labeling a substance derived from a living organism and selectively hybridized with a specific binding substance absorbed in each of the absorptive regions 154 constituting each line of the absorptive regions 154 of the biochemical analysis unit 150 by an antigen-antibody reaction from being peeled off from the hapten and, therefore, the accuracy of biochemical analysis can be improved.

Furthermore, according to this embodiment, since the temperatures of the cleaning solution to be fed via each of the capillaries 160 can be controlled by the corresponding temperature control unit 161 in accordance with the kind of hapten labeling a substance derived from a living organism and selectively hybridized with a specific binding substance absorbed in the absorptive regions 154 constituting each line of the absorptive regions 154, it is possible to effectively prevent an antibody for the hapten labeled with an enzyme which generates chemiluminescence emission when it contacts a chemiluminescent substrate and once bonded with the hapten labeling a substance derived from a living organism and selectively hybridized with a specific binding substance absorbed in each of the absorptive regions 154 constituting each line of the absorptive regions 154 of the biochemical analysis unit 150 by an antigen-antibody reaction from being peeled off from the hapten and, on the other hand, even if an antibody which should not be bonded with the hapten labeling a substance derived from a living body and selectively hybridized with a specific binding substance absorbed in each of the absorptive regions 154 constituting each line of the absorptive regions 154 of the biochemical analysis unit 150 has been bonded with one or more the absorptive regions among the absorptive regions 154 constituting each line of the absorptive regions 154 of the biochemical analysis unit 150, it is possible to effectively peel off and remove an antibody for the hapten from the absorptive region 154 of the biochemical analysis unit 150 by cleaning the absorptive regions 154 constituting each line of the absorptive regions 154 of the biochemical analysis unit 150 with the cleaning solution whose temperature is controlled to the temperature optimum for each line of the capillaries 160. Therefore, it is possible to bind an antibody for the hapten to be bonded with the hapten labeling a substance derived from a living body and selectively hybridized with a specific binding substance absorbed in each of the absorptive regions 4 of the biochemical analysis unit 1 with the hapten by an antigen-antibody reaction in a desired manner.

The present invention has thus been shown and described with reference to specific embodiments. However, it should be noted that the present invention is in no way limited to the details of the described arrangements but changes and modifications may be made without departing from the scope of the appended claims.

For example, in the above described embodiments, radiation data, fluorescence data and chemiluminescence data are selectively recorded in a number of the absorptive regions 4, 154 of the biochemical analysis unit 1, 150 by selectively hybridizing a substance derived from a living organism and labeled with a radioactive labeling substance and a fluorescent substance with specific labeling substances fixed in a number of the absorptive regions 4, 154 of the biochemical analysis unit 1, 150, selectively hybridizing a substance derived from a living organism and labeled with a hapten such as digoxigenin with specific labeling substances fixed in a number of the absorptive regions 4, 154 of the biochemical analysis unit 1, 150 and further binding an antibody for the hapten labeled with an enzyme which generates chemiluminescence emission when it contacts a chemiluminescent substrate with the hapten labeling a substance derived from a living organism selectively hybridized with the specific binding substances by an antigen-antibody reaction. However, the application of the present invention is not limited to such reaction and the present invention can be applied to various kinds of a receptor-ligand association reactions.

Further, in the above described embodiments, chemiluminescence data are selectively recorded in a number of the absorptive regions 4, 154 of the biochemical analysis unit 1, 150 by selectively hybridizing a substance derived from a living organism and labeled with a hapten such as digoxigenin with specific labeling substances fixed in a number of the absorptive regions 4, 154 of the biochemical analysis unit 1, 150 and further binding an antibody for the hapten labeled with an enzyme which generates chemiluminescence emission when it contacts a chemiluminescent substrate with the hapten labeling a substance derived from a living organism and selectively hybridized with the specific binding substances fixed in number of the absorptive regions 4, 154 of the biochemical analysis unit 1, 150 by an antigen-antibody reaction. However, chemiluminescence data may be selectively recorded in a number of the absorptive regions 4, 154 of the biochemical analysis unit 1, 150 by selectively hybridizing a substance derived from a living body and laleled with a labeling substance which generates chemiluminescence emission when it contacts a chemiluminescent substrate with specific binding substances fixed in a number of the absorptive regions 4, 151 of the biochemical analysis unit 1, 150.

Furthermore, in the above described embodiments, fluorescence data are selectively recorded in a number of the absorptive regions 4, 154 of the biochemical analysis unit 1, 150 by selectively hybridizing a substance derived from a living organism and labeled with a fluorescent substance with specific labeling substances fixed in a number of the absorptive regions 4, 154 of the biochemical analysis unit 1, 150. However, fluorescence data may be selectively recorded in a number of the absorptive regions 4, 154 of the biochemical analysis unit 1, 150 by selectively hybridizing a substance derived from a living organism and labeled with a hapten such as digoxigenin with specific labeling substances fixed in a number of the absorptive regions 4, 154 of the biochemical analysis unit 1, 150 and further binding an antibody for the hapten labeled with an enzyme which generates a fluorescence substance when it contacts a fluorescent substrate with the hapten labeling a substance derived from a living organism and selectively hybridized with the specific binding substances fixed in number of the absorptive regions 4, 154 of the biochemical analysis unit 1, 150 by an antigen-antibody reaction.

Further, in the above described embodiments, the probe solution containing a substance derived from a living organism and labeled with a radioactive labeling substance, a fluorescent substance and a hapten such as digoxigenin is prepared and the substance derived from a living organism and labeled with a radioactive labeling substance, a fluorescent substance and a hapten such as digoxigenin is selectively hybridized with specific binding substances fixed in number of the absorptive regions 4, 154 of the biochemical analysis unit 1, 150. However, it is not absolutely necessary for the probe solution to contain a substance derived from a living organism and labeled with a radioactive labeling substance, a fluorescent substance and a hapten such as digoxigenin and it is sufficient for probe solution to contain a substance derived from a living organism and labeled with at least one of a radioactive labeling substance, a fluorescent substance and a hapten such as digoxigenin.

Moreover, in the above described embodiments, as specific binding substances, cDNAs each of which has a known base sequence and is different from the others are used. However, specific binding substances usable in the present invention are not limited to cDNAs but all specific binding substances capable of specifically binding with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, can be employed in the present invention as a specific binding substance.

Further, in the above described embodiments, although the hybridization, the antigen-antibody reaction and the cleaning of a number of the absorptive regions 4, 154 of the biochemical analysis unit 1, 150 are performed by the apparatus for conducting receptor-ligand association reaction, it is possible to perform only the hybridization or the antigen-antibody reaction using the apparatus for conducting receptor-ligand association reaction and perform a number of the absorptive regions 4, 154 of the biochemical analysis unit 1, 150 using a separate cleaning apparatus.

Furthermore, the apparatus for conducting receptor-ligand association reaction shown in Figure 3 is constituted so as to drive the pump 10 in both the forward and reverse directions and alternately move the hybridization buffer, the mixed solution of the hybridization buffer and the probe solution, the antibody solution and the cleaning solution in the direction indicated by the arrow A and the direction indicated by the arrow B in Figure 3. However, the apparatus for conducting receptor-ligand association reaction may be constituted so as to drive the pump 10 in only one direction and move the hybridization buffer, the mixed solution of the hybridization buffer and the probe solution, the antibody solution and the cleaning solution only in the direction indicated by the arrow A or the direction indicated by the arrow B in Figure 3.

Moreover, in the apparatus for conducting receptor-ligand association reaction shown in Figure 3, the hybridization buffer, the mixed solution of the hybridization buffer and the probe solution, the antibody solution and the cleaning solution are forcibly circulated in the cartridge 7 via the solution circulation pipe 9 so as to through the number of the absorptive regions 4 of the biochemical analysis unit 1. However, it is not absolutely necessary to circulate the hybridization buffer, the mixed solution of the hybridization buffer and the probe solution, the antibody solution or the cleaning solution and the hybridization buffer, the mixed solution of the hybridization buffer and the probe solution passing through a number of the absorptive regions 4 of the biochemical analysis unit 1 accommodated in the cartridge 7 may be discharged through the solution discharge pipe 13.

Further, in the apparatus for conducting receptor-ligand association reaction shown in Figure 3, the cleaning solution is forcibly circulated in the cartridge 7 via the solution circulation pipe 9 so as to pass through the number of the absorptive regions 4 of the biochemical analysis unit 1. However, it is not absolutely necessary to circulate the cleaning solution and the cleaning solution passing through a number of the absorptive regions 4 of the biochemical analysis unit 1 accommodated in the cartridge 7 may be discharged through the solution discharge pipe 13.

Furthermore, although the apparatus for conducting receptor-ligand association reaction shown in Figure 3 includes the hybridization buffer tank 12a, the probe solution chip 12b, the antibody solution tank 12c and the cleaning solution tank 12d, it is not absolutely necessary for the apparatus for conducting receptor-ligand association reaction to include the hybridization buffer tank 12a, the probe solution chip 12b, the antibody solution tank 12c and the cleaning solution tank 12d.

Further, in the apparatus for conducting receptor-ligand association reaction shown in Figure 3, a number of the absorptive regions 4 of the biochemical analysis unit 1 accommodated in the cartridge 7 are cleaned by moving the cleaning solution filling the inner spaces of the cartridge 7 and the solution circulation pipe 9 athrough a number of the absorptive regions 4 of the biochemical analysis unit 1 in both the obverse direction and reverse direction and the cleaning operation is completed by discharging the cleaning solution through the solution discharge pipe 13. However, it is possible to repeatedly perform the cleaning operations by feeding a new cleaning solution from the cleaning solution tank 12d into the cartridge 7 and the solution circulation pipe 9 after discharging the cleaning solution from the cartridge 7 and the solution circulation pipe 9.

Furthermore, in the apparatus for conducting receptor-ligand association reaction shown in Figure 3, although a number of the absorptive regions 4 of the biochemical analysis unit 1 accommodated in the cartridge 7 are cleaned by repeatedly moving the cleaning solution filling the inner spaces of the cartridge 7 and the solution circulation pipe 9 through a number of the absorptive regions 4 of the biochemical analysis unit 1 in both the obverse direction and reverse direction, it is possible to feed a new cleaning solution from the cleaning solution tank 12d into the cartridge 7 and the solution circulation pipe 9 after moving the cleaning solution through a number of the absorptive regions 4 of the biochemical analysis unit 1 in both the obverse direction and reverse direction and discharging the cleaning solution from the cartridge 7 and the solution circulation pipe 9 and to repeatedly perform the cleaning operations.

Moreover, in the apparatus for conducting receptor-ligand association reaction shown in Figure 22, the pair of ultrasonic oscillators 122a, 122b are provided on facing inner surfaces of the casing 120b so as to face the opposite surfaces of the biochemical analysis unit 1 when it is held in the reaction vessel 120 by the biochemical analysis unit holding member 121a and the biochemical analysis unit holding member 121b and the pre-hybridization, the hybridization, the antigen-antibody reaction or the cleaning is performed by alternately oscillating the pair of ultrasonic oscillators 122a, 122b by the controller 123 and alternately moving the hybridization buffer, the mixed solution of the hybridization buffer and the probe solution, the antibody solution or the cleaning solution in the direction indicated by the arrow A and the direction indicated by the arrow B in Figure 22. However, the pre-hybridization, the hybridization, the antigen-antibody reaction or the cleaning may be performed by oscillating the ultrasonic oscillator 122a or the ultrasonic oscillator 122b provided on the inner surface of the casing 120b facing one of the surfaces of the biochemical analysis unit 1 by the controller 123 and forcibly moving the hybridization buffer, the mixed solution of the hybridization buffer and the probe solution, the antibody solution or the cleaning solution only in the direction indicated by the arrow A or the direction indicated by the arrow B in Figure 22.

Further, the temperature control unit 132 is provided for each of the capillaries 131 for controlling the temperature of a solution flowing in the capillary 131 in the apparatus for conducting receptor-ligand association reaction shown in Figure 23 and the temperature control unit 161 is provided for a plurality of the capillaries 160 constituting each line of the capillaries 161 for controlling the temperature flowing in the plurality of the capillaries 160 constituting each line of the capillaries 161 in the apparatus for conducting receptor-ligand association reaction shown in Figure 25. However, temperature control units 132, 161 may be provided in such a manner that one temperature control unit 132, 161 is provided for a plurality of the capillaries 131, 160 constituting two or more neighboring lines of the capillaries 131, 160.

Furthermore, the temperature control unit 132 is provided for each of the capillaries 131 for controlling the temperature of a solution flowing in the capillary 131 in the apparatus for conducting receptor-ligand association reaction shown in Figure 23 and the temperature control unit 161 is provided for a plurality of the capillaries 160 constituting each line of the capillaries 161 for controlling the temperature flowing in the plurality of the capillaries 160 constituting each line of the capillaries 161 in the apparatus for conducting receptor-ligand association reaction shown in Figure 25. However, it is possible to divide a plurality of the capillaries 131, 160 into two or more groups and provide a temperature control unit 132, 161 for each group of the capillaries 131, 160.

Moreover, the temperature control unit 132 is provided for each of the capillaries 131 for controlling the temperature of a solution flowing in the capillary 131 in the apparatus for conducting receptor-ligand association reaction shown in Figure 23 and the temperature control unit 161 is provided for a plurality of the capillaries 160 constituting each line of the capillaries 161 for controlling the temperature flowing in the plurality of the capillaries 160 constituting each line of the capillaries 161 in the apparatus for conducting receptor-ligand association reaction shown in Figure 25. However, it is not absolutely necessary to provide any temperature control unit 132, 161 for controlling the temperature of a solution flowing through the capillaries in the apparatus for conducting receptor-ligand association reaction.

Further, although the apparatus for conducting receptor-ligand association reaction shown in Figure 23 includes the hybridization buffer tank 141, the antibody solution tank 142 and the cleaning tank 143, it is not absolutely necessary for the apparatus for conducting receptor-ligand association reaction to include the hybridization buffer tank 141, the antibody solution tank 142 and the cleaning tank 143.

Furthermore, the apparatus for conducting receptor-ligand association reaction shown in Figure 23 is constituted so as to collect the hybridization buffer and the mixed solution of the hybridization buffer and the probe solution via the solution collecting capillaries 133 to be recycled into the hybridization buffer tank 141 and collect the antibody solution via the solution collecting capillaries 133 to be recycled into the antibody solution tank 142. However, it is possible to automatically recycle the hybridization buffer and the mixed solution of the hybridization buffer and the probe solution into the hybridization buffer tank 141 and recycle the antibody solution into the antibody solution tank 142 by connecting the solution collecting capillaries 133 to the hybridization buffer tank 141 and the antibody solution tank 142 via a change-over valve so as to selectively communicating with the hybridization buffer tank 141 or the antibody solution tank 142 and providing a pump in the solution collecting capillaries 133.

Moreover, in the above described embodiments, although about 10,000 substantially circular absorptive regions 4, 154 having a size of about 0.01 mm² are formed in the biochemical analysis unit 1, 150 in a regular pattern at a density of about 5,000 per cm², the shape of each of the absorptive regions 4, 154 is not limited to substantially a circular shape but may be formed in an arbitrary shape, for example, a rectangular shape.

Further, in the above described embodiments, although about 10,000 substantially circular absorptive regions 4, 154 having a size of about 0.01 mm² are formed in the biochemical analysis unit 1, 150 in a regular pattern at a density of about 5,000 per cm², the number or size of the absorptive regions 4, 154 may be arbitrarily selected in accordance with the purpose. Preferably, 10 or more of the absorptive regions 4, 154 having a size of 5 mm² or less are formed in the biochemical analysis unit 1, 150 at a density of 10/ cm² or greater.

Furthermore, in the above described embodiments, although about 10,000 substantially circular absorptive regions 4, 154 having a size of about 0.01 mm² are formed in the biochemical analysis unit 1, 150 in a regular pattern at a density of about 5,000 per cm², it is not absolutely necessary to form the absorptive regions 4, 154 in a regular pattern.

Moreover, although a number of the absorptive regions 4 of the biochemical analysis unit 1 are formed by charging nylon-6 in a number of the through-hole 3 formed in the substrate 2 made of stainless steel in the embodiment shown in Figure 1 and a number of the absorptive regions 154 of the biochemical analysis unit 150 are formed by pressing the absorptive membrane 153 containing nylon-6 into a number of the through-holes 152 formed in the substrate 151 made of stainless steel in the embodiment shown in Figure 24, it is not absolutely necessary to make the substrate 2, 151 of the biochemical analysis unit 1, 150 of stainless steel but the substrate 2, 151 of the biochemical analysis unit 1, 150 may be made of other kinds of material. The substrate 2, 151 of the biochemical analysis unit 1, 150 is preferably made of material capable of attenuating radiation energy and light energy but the material for forming the substrate 2, 151 of the biochemical analysis unit 1, 150 is not particularly limited. The substrate 2, 151 of the biochemical analysis unit 1, 150 can be formed of either inorganic compound material or organic compound material and is preferably formed of a metal material, a ceramic material or a plastic material. Illustrative examples of inorganic compound materials usable for forming the substrate 2, 151 of the biochemical analysis unit 1, 150 and capable of attenuating radiation energy and/or light energy include metals such as gold, silver, copper, zinc, aluminum, titanium, tantalum, chromium, steel, nickel, cobalt, lead, tin, selenium and the like; alloys such as brass, stainless, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. High molecular compounds are preferably used as organic compound material for forming the substrate 2, 151 of the biochemical analysis unit 1, 150 and capable of attenuating radiation energy and light energy and illustrative examples thereof include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4, 10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Further, although a number of the absorptive regions 4 of the biochemical analysis unit 1 are formed by charging nylon-6 in a number of the through-hole 3 formed in the substrate 2 made of stainless steel in the embodiment shown in Figure 1 and a number of the absorptive regions 154 of the biochemical analysis unit 150 are formed by pressing the absorptive membrane 153 containing nylon-6 into a number of the through-holes 152 formed in the substrate 151 made of stainless steel in the embodiment shown in Figure 24, it is not absolutely necessary to form a number of the absorptive regions 4, 154 of the biochemical analysis unit 1, 150 of nylon-6 but a number of the absorptive regions 4, 154 of the biochemical analysis unit 1, 150 may be formed of other absorptive material. A porous material or a fiber material may be preferably used as the absorptive material for forming a number of the absorptive regions 4, 154 of the biochemical analysis unit 1, 150 and a number of the absorptive regions 4, 154 of the biochemical analysis unit 1, 150 may be formed by combining a porous material and a fiber material. A porous material for forming a number of the absorptive regions 4, 154 of the biochemical analysis unit 1, 150 may be any type of an organic material or an inorganic material and may be an organic/inorganic composite material. An organic porous material used for forming a number of the absorptive regions 4, 154 of the biochemical analysis unit 1, 150 is not particularly limited but a carbon porous material such as an activated carbon or a porous material capable of forming a membrane filter can be preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof. An inorganic porous material used for forming a number of the absorptive regions 4, 154 of the biochemical analysis unit 1, 150 is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof. A fiber material used for forming a number of the absorptive regions 4, 154 of the biochemical analysis unit 1, 150 is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

Furthermore, although a number of the absorptive regions 154 of the biochemical analysis unit 150 are formed by pressing the absorptive membrane 153 formed of nylon-6 into a number of the through-holes 152 formed in the substrate 151 made of stainless steel using the calender processing apparatus in the embodiment shown in Figure 24, it is possible to press the absorptive membrane 153 into a number of the through-holes 102 formed in the substrate 151 by other means such as a heat press apparatus. Further, a number of the absorptive regions 154 may be formed by charging the absorptive membrane 153 into a number of the through-holes 152 formed in the substrate 151 by an appropriate method other than pressing.

Moreover, although a number of the absorptive regions 154 of the biochemical analysis unit 150 are formed by pressing the absorptive membrane 153 formed of nylon-6 into a number of the through-holes 152 formed in the substrate 151 made of stainless steel in the embodiment shown in Figure 24, the absorptive membrane 153 may be pressed into a number of the through-holes 152 formed in the substrate 151 via an adhesive agent for improving the strength of the biochemical analysis unit 150.

Further, although a number of the absorptive regions 4 of the biochemical analysis unit 1 are formed by charging nylon-6 in a number of the through-hole 3 formed in the substrate 2 made of stainless steel in the embodiment shown in Figure 1 and a number of the absorptive regions 154 of the biochemical analysis unit 150 are formed by pressing the absorptive membrane 153 containing nylon-6 into a number of the through-holes 152 formed in the substrate 151 made of stainless steel in the embodiment shown in Figure 24, a number of absorptive regions of a biochemical analysis unit may be formed to be spaced apart from each other by closely contacting a perforated plate formed with a number of through-holes onto at least one surface of an absorptive substrate.

Furthermore, although a number of the absorptive regions 4 of the biochemical analysis unit 1 are formed by charging nylon-6 in a number of the through-hole 3 formed in the substrate 2 made of stainless steel in the embodiment shown in Figure 1 and a number of the absorptive regions 154 of the biochemical analysis unit 150 are formed by pressing the absorptive membrane 153 containing nylon-6 into a number of the through-holes 152 formed in the substrate 151 made of stainless steel in the embodiment shown in Figure 24, a biochemical analysis unit formed with a number of absorptive regions containing specific binding substances and spaced apart from each other may be formed by spotting a solution containing specific binding substances on regions spaced apart from each other on an absorptive substrate made of an absorptive material.

Furthermore, in the above-described embodiments, a solution containing specific binding substances such as cDNAs are spotted using the spotting device including an injector 5 and a CCD camera 6 so that when the tip end portion of the injector 5 and the center of the absorptive region 4, 154 into which a solution containing specific binding substances is to be spotted are determined to coincide with each other as a result of viewing them using the CCD camera 6, the solution containing the specific binding substances such as cDNA is spotted from the injector 5. However, the solution containing specific binding substances such as cDNAs can be spotted by detecting the positional relationship between a number of the absorptive regions 4, 154 formed in the biochemical analysis unit 1, 150 and the tip end portion of the injector 5 in advance and two-dimensionally moving the biochemical analysis unit 1, 150 or the tip end portion of the injector 5 so that the tip end portion of the injector 5 coincides with each of the absorptive regions 4, 154.

According to the present invention, it is possible to provide to provide a method for conducting a receptor-ligand association reaction and an apparatus used therefor which can efficiently associate a ligand or a receptor with receptors or ligands fixed in a biochemical analysis unit and produce biochemical analysis data having an excellent high quantitative characteristic with excellent repeatability.

## Claims

1. A method for conducting a receptor-ligand association reaction comprising the step of feeding a reaction solution containing a ligand or a receptor labeled with a labeling substance to a plurality of absorptive regions which are formed in a biochemical analysis unit to be spaced from each other and in which receptors or ligands are fixed so as to cut through the plurality of absorptive regions, thereby selectively associating the ligand or the receptor contained in the reaction solution with the receptors or the ligands fixed in the plurality of absorptive regions of the biochemical analysis unit.

2. A method for conducting a receptor-ligand association reaction in accordance with Claim 1 wherein the reaction solution is fed to the plurality of absorptive regions of the biochemical analysis unit in both an obverse direction and a reverse direction so as to cut through the plurality of absorptive regions.

3. A method for conducting a receptor-ligand association reaction in accordance with Claim 1 or 2 which further comprises steps of accommodating the biochemical analysis unit in a reaction vessel and feeding the reaction solution into the reaction vessel.

4. A method for conducting a receptor-ligand association reaction in accordance with Claim 3 which further comprises a step of circulating the reaction solution into the reaction vessel accommodating the biochemical analysis unit, thereby passing the reaction solution through the plurality of absorptive regions of the biochemical analysis unit.

5. A method for conducting a receptor-ligand association reaction in accordance with Claim 1 or 2 which further comprises steps of accommodating the biochemical analysis unit in a reaction vessel and generating ultrasound in a reaction solution containing a ligand or a receptor and accommodated in the reaction vessel in a direction cutting through the plurality of absorptive regions of the biochemical analysis unit, thereby forcibly passing the reaction solution so as to cut through the plurality of absorptive regions of the biochemical analysis unit.

6. A method for conducting a receptor-ligand association reaction in accordance with Claim 5 which comprises steps of alternately generating ultrasound in the reaction solution in an obverse direction and a reverse direction cutting through the plurality of absorptive regions of the biochemical analysis unit accommodated in the reaction vessel, thereby forcibly passing the reaction solution through the plurality of absorptive regions of the biochemical analysis unit in both the obverse direction and the reverse direction.

7. A method for conducting a receptor-ligand association reaction in accordance with Claim 1 which comprises a step of feeding the reaction solutions whose temperature is controlled for each group consisting at least two of the absorptive regions among the plurality of absorptive regions of the biochemical analysis unit to the plurality of absorptive regions of the biochemical analysis unit, thereby selectively associating the ligand or the receptor contained in the reaction solution with the receptors or the ligands fixed in the plurality of absorptive regions of the biochemical analysis unit.

8. A method for conducting a receptor-ligand association reaction in accordance with Claim 7 wherein the plurality of absorptive regions of the biochemical analysis unit are divided into a plurality of blocks each including two or more absorptive regions and the method for conducting a receptor-ligand association reaction comprises the step of feeding the reaction solution whose temperature is controlled for each of the blocks to the plurality of absorptive regions of the biochemical analysis unit, thereby selectively associating the ligand or the receptor contained in the reaction solution with the receptors or the ligands fixed in the plurality of absorptive regions of the biochemical analysis unit.

9. A method for conducting a receptor-ligand association reaction in accordance with Claim 1 which comprises a step of feeding the reaction solution whose temperature is controlled for each of the plurality of absorptive regions to the plurality of absorptive regions of the biochemical analysis unit, thereby selectively associating the ligand or the receptor contained in the reaction solution with the receptors or the ligands fixed in the plurality of absorptive regions of the biochemical analysis unit.

10. A method for conducting a receptor-ligand association reaction in accordance with any one of Claims 7 to 9 wherein the temperature of the reaction solution is controlled in accordance with the kinds of the receptors or the ligands fixed in the plurality of absorptive regions of the biochemical analysis unit.

11. A method for conducting a receptor-ligand association reaction in accordance with any one of Claims 1 to 10 which further comprises a step of feeding a cleaning solution to the plurality of absorptive regions of the biochemical analysis unit in which the receptors or the ligands selectively associated with the ligand or the receptor contained in the reaction solution are fixed so as to pass through the plurality of absorptive regions of the biochemical analysis unit, thereby cleaning the plurality of absorptive regions of the biochemical analysis unit.

12. A method for conducting a receptor-ligand association reaction in accordance with any one of Claims 3 to 6 wherein the cleaning solution is fed into the reaction vessel so as to pass through the plurality of absorptive regions of the biochemical analysis unit accommodated in the reaction vessel in both an obverse direction and a reverse direction.

13. A method for conducting a receptor-ligand association reaction in accordance with Claim 12 which comprises a step of circulating the cleaning solution into the cartridge accommodating the biochemical analysis unit, thereby passing the cleaning solution through the plurality of absorptive regions of the biochemical analysis unit.

14. A method for conducting a receptor-ligand association reaction in accordance with any one of Claims 3 to 6 which comprises a steps of accommodating a cleaning solution in the reaction vessel and generating ultrasound in the cleaning solution in a direction cutting through the plurality of absorptive regions of the biochemical analysis unit in which the receptors or the ligands selectively associated with the ligand or the receptor contained in the reaction solution are fixed, thereby forcibly passing the cleaning solution so as to pass through the plurality of absorptive regions of the biochemical analysis unit and cleaning the plurality of absorptive regions of the biochemical analysis unit.

15. A method for conducting a receptor-ligand association reaction in accordance with Claim 14 which comprises steps of alternately generating ultrasound in the cleaning solution in an obverse direction and a reverse direction cutting through the plurality of absorptive regions of the biochemical analysis unit accommodated in the reaction vessel, thereby forcibly passing the cleaning solution through the plurality of absorptive regions of the biochemical analysis unit in both the obverse direction and the reverse direction and cleaning the plurality of absorptive regions of the biochemical analysis unit.

16. A method for conducting a receptor-ligand association reaction in accordance with any one of Claims 7 to 10 which comprises a step of feeding the cleaning solutions whose temperature is controlled for each group consisting at least two of the absorptive regions among the plurality of absorptive regions of the biochemical analysis unit to the plurality of absorptive regions of the biochemical analysis unit, thereby cleaning the plurality of absorptive regions of the biochemical analysis unit.

17. A method for conducting a receptor-ligand association reaction in accordance with Claim 16 which comprises a step of feeding the cleaning solution whose temperature is controlled for each of the blocks to the plurality of absorptive regions of the biochemical analysis unit, thereby cleaning the plurality of absorptive regions of the biochemical analysis unit.

18. A method for conducting a receptor-ligand association reaction in accordance with any one of Claims 7 to 10 which comprises a step of feeding the cleaning solution whose temperature is controlled for each of the plurality of absorptive regions to the plurality of absorptive regions of the biochemical analysis unit, thereby cleaning the plurality of absorptive regions of the biochemical analysis unit.

19. A method for conducting a receptor-ligand association reaction in accordance with Claim 18 wherein the temperature of the cleaning solution is controlled in accordance with the kinds of the receptors or the ligands fixed in the plurality of absorptive regions of the biochemical analysis unit.

20. A method for conducting a receptor-ligand association reaction in accordance with any one of Claims 1, 7 to 10 and 16 to 19 wherein the reaction solution and/or the cleaning solution is fed to the plurality of absorptive regions of the biochemical analysis unit by independent conduits.

21. A method for conducting a receptor-ligand association reaction in accordance with any one of Claims 1 to 20 wherein the reaction solution contains a ligand or a receptor labeled with at least one labeling substance selected from a group consisting of a radioactive labeling substance, a fluorescent substance and a labeling substance which generates chemiluminescence emission when it contacts a chemiluminescent substrate.

22. A method for conducting a receptor-ligand association reaction in accordance with any one of Claims 1 to 20 wherein specific binding substances whose structure or characteristics are known are fixed in the plurality of absorptive regions of the biochemical analysis unit and the method for conducting a receptor-ligand association reaction comprises the step of feeding the reaction solution containing a substance derived from a living organism and labeled with a labeling substance to the plurality of absorptive regions of the biochemical analysis unit so as to cut through them, thereby selectively hybridizing the substance derived from a living organism, labeled with a labeling substance and contained in the reaction solution with the specific binding substances fixed in the plurality of absorptive regions of the biochemical analysis unit.

23. A method for conducting a receptor-ligand association reaction in accordance with any one of Claims 1 to 20 wherein antigens or antibodies are fixed in the plurality of absorptive regions of the biochemical analysis unit and the method for conducting a receptor-ligand association reaction comprises the step of feeding the reaction solution containing an antibody or an antigen labeled with a labeling substance to the plurality of absorptive regions of the biochemical analysis unit so as to cut through them, thereby selectively binding the an antibody or the antigen labeled with the labeling substance with the antigens or the antibodies fixed in the plurality of absorptive regions of the biochemical analysis unit.

24. A method for conducting a receptor-ligand association reaction in accordance with any one of Claims 1 to 20 wherein specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a labeling enzyme to the plurality of absorptive regions of the biochemical analysis unit so as to cut through them, thereby binding the antibody labeled with the labeling enzyme with the hapten fixed in the plurality of absorptive regions of the biochemical analysis unit by an antigen-antibody reaction.

25. A method for conducting a receptor-ligand association reaction in accordance with any one of Claims 1 to 20 wherein specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a enzyme which generates chemiluminescence emission when it contacts a chemiluminescent substrate to the plurality of absorptive regions of the biochemical analysis unit so as to cut through them, thereby binding the antibody labeled with the enzyme with the hapten fixed in the plurality of absorptive regions of the biochemical analysis unit by an antigen-antibody reaction.

26. A method for conducting a receptor-ligand association reaction in accordance with any one of Claims 1 to 20 wherein specific binding substances whose structure or characteristics are known are fixed in the plurality of absorptive regions of the biochemical analysis unit and the method for conducting a receptor-ligand association reaction comprises the steps of feeding the reaction solution containing a substance derived from a living organism and labeled with hapten to the plurality of absorptive regions of the biochemical analysis unit so as to cut through them, thereby selectively hybridizing the substance derived from a living organism, labeled with the hapten and contained in the reaction solution with the specific binding substances fixed in the plurality of absorptive regions of the biochemical analysis unit, and feeding an antibody solution containing an antibody for the hapten labeled with a enzyme which generates a fluorescence substance when it contacts a fluorescent substrate to the plurality of absorptive regions of the biochemical analysis unit so as to cut through them, thereby binding the antibody labeled with the enzyme with the hapten fixed in the plurality of absorptive regions of the biochemical analysis unit by an antigen-antibody reaction.

27. A method for conducting a receptor-ligand association reaction in accordance with any one of Claims 1 to 26 wherein the biochemical analysis unit includes an absorptive substrate formed of an absorptive material and the plurality of absorptive regions of the biochemical analysis unit are formed by absorbing a receptor or a ligand in regions spaced apart from each other of the absorptive substrate.

28. A method for conducting a receptor-ligand association reaction in accordance with any one of Claims 1 to 26 wherein the biochemical analysis unit includes a substrate formed of a plurality of through-holes to be spaced apart from each other and the plurality of absorptive regions of the biochemical analysis unit are formed by charging an absorptive material in the plurality of through-holes formed in the substrate.

29. A method for conducting a receptor-ligand association reaction in accordance with Claim 28 wherein the plurality of absorptive regions of the biochemical analysis unit are formed by pressing an absorptive membrane containing an absorptive material in the plurality of through-holes formed in the substrate.

30. A method for conducting a receptor-ligand association reaction in accordance with any one of Claims 1 to 29 wherein the substrate of the biochemical analysis unit is formed with 10 or more absorptive regions.

31. A method for conducting a receptor-ligand association reaction in accordance with any one of Claims 1 to 30 wherein each of the plurality of absorptive regions formed in the substrate of the biochemical analysis unit has a size of less than 5 mm².

32. A method for conducting a receptor-ligand association reaction in accordance with any one of Claims 1 to 31 wherein the plurality of absorptive regions are formed in the substrate of the biochemical analysis unit at a density of 10 or more per cm².

33. A method for conducting a receptor-ligand association reaction in accordance with any one of Claims 1 to 32 wherein the substrate of the biochemical analysis unit has a property of attenuating radiation energy.

34. A method for conducting a receptor-ligand association reaction in accordance with Claim 33 wherein the substrate of the biochemical analysis unit has a property of reducing the energy of radiation to 1/5 or less when the radiation travels in the substrate by a distance equal to that between neighboring absorptive regions.

35. A method for conducting a receptor-ligand association reaction in accordance with any one of Claims 1 to 34 wherein the substrate of the biochemical analysis unit has a property of attenuating radiation energy.

36. A method for conducting a receptor-ligand association reaction in accordance with Claim 35 wherein the substrate of the biochemical analysis unit has a property of reducing the energy of light to 1/5 or less when the light travels in the substrate by a distance equal to that between neighboring absorptive regions.

37. A receptor-ligand associating apparatus comprising a biochemical analysis unit holding means for holding a biochemical analysis unit formed with a plurality of absorptive regions which are spaced apart from each other and in which receptors or ligands are fixed, and a solution feeding means for feeding a solution so as to pass through the plurality of absorptive regions of the biochemical analysis unit held by the biochemical analysis unit holding means.

38. A receptor-ligand associating apparatus in accordance with Claim 37 wherein the biochemical analysis unit holding means is constituted by a reaction vessel for accommodating the biochemical analysis unit therein and the solution feeding means includes a solution circulation passage communicating with spaces in the reaction vessel on the opposite sides of the biochemical analysis unit accommodated in the reaction vessel and a pump for circulating a solution in the reaction vessel and the solution circulation passage.

39. A receptor-ligand associating apparatus in accordance with Claim 38 wherein the pump is constituted so as to be driven in both a forward direction and a reverse direction.

40. A receptor-ligand associating apparatus in accordance with Claim 39 wherein a solution discharge passage is further connected via a change-over valve to the solution circulation passage downstream of the reaction vessel with respect a direction in which a solution flows when the pump is driven in the forward direction.

41. A receptor-ligand associating apparatus in accordance with any one of Claims 38 to 40 which further comprises at least one reaction solution sources capable of communicating with the solution circulation passage for accommodating a reaction solution containing a ligand or a receptor.

42. A receptor-ligand associating apparatus in accordance with Claim 41 which further comprises two or more reaction solution sources for accommodating a reaction solution containing a ligand or a receptor and the two or more reaction solution sources are constituted so as to selectively communicate with the solution circulation passage.

43. A receptor-ligand associating apparatus in accordance with any one of Claims 38 to 42 which further comprises at least one cleaning solution source capable of communicating with the solution circulation passage for accommodating a cleaning solution.

44. A receptor-ligand associating apparatus in accordance with Claim 43 which further comprises two or more reaction solution sources for accommodating a reaction solution containing a ligand or a receptor and at least one cleaning solution source for accommodating a cleaning solution and the two or more reaction solution sources and at least one cleaning solution source are constituted so as to selectively communicate with the solution circulation passage.

45. A receptor-ligand associating apparatus in accordance with Claim 37 wherein the biochemical analysis holding means is constituted by a reaction vessel for accommodating the biochemical analysis unit and a solution and the solution feeding means is constituted as ultrasound generating means for generating ultrasound in the solution accommodated in the reaction vessel in a direction cutting through the plurality of absorptive regions of the biochemical analysis unit accommodated in the reaction vessel.

46. A receptor-ligand associating apparatus in accordance with Claim 45 wherein the ultrasound generating means includes a pair of ultrasonic oscillators, one of them being constituted so as to generate ultrasound in a solution cutting through the plurality of absorptive regions of the biochemical analysis unit accommodated in the reaction vessel in an obverse direction and the other being constituted so as to generate ultrasound in a solution cutting through the plurality of absorptive regions of the biochemical analysis unit accommodated in the reaction vessel in a reverse direction.

47. A receptor-ligand associating apparatus in accordance with Claim 37 which further comprises a plurality of tubular members for feeding a solution, each having an opening facing one of the plurality of absorptive regions of the biochemical analysis unit held by the biochemical analysis unit holding means, and temperature control means, each independently provided for a group consisting of two or more tubular members among the plurality of tubular members for controlling the temperature of a solution flowing through the two or more tubular members constituting the group.

48. A receptor-ligand associating apparatus in accordance with Claim 47 wherein the plurality of tubular members are divided into a plurality of blocks each including two or more tubular members and an independent temperature control means is provided for each of the blocks.

49. A receptor-ligand associating apparatus in accordance with Claim 37 which further comprises a plurality of tubular members for feeding a solution, each having an opening facing one of the plurality of absorptive regions of the biochemical analysis unit held by the biochemical analysis unit holding means, the plurality of tubular members being disposed in the manner of a matrix at least in the vicinity of the biochemical analysis unit held by the biochemical analysis unit holding means, and an independent temperature control means is provided for the plurality of tubular members constituting each line of matrix.

50. A receptor-ligand associating apparatus in accordance with Claim 37 which further comprises a plurality of tubular members for feeding a solution, each having an opening facing one of the plurality of absorptive regions of the biochemical analysis unit held by the biochemical analysis unit holding means, and wherein an independent temperature control means is provided for each of the plurality of tubular members.

51. A receptor-ligand associating apparatus in accordance with any one of Claims 47 to 50 which further comprises a plurality of solution collecting tubular members each facing one of the openings of the plurality of tubular members via one of the plurality of absorptive regions of the biochemical analysis unit held by the biochemical analysis unit holding means and being adapted for receiving a solution fed to the absorptive region of the biochemical analysis unit from the tubular member.

52. A receptor-ligand associating apparatus in accordance with any one of Claims 47 to 51 which further comprises a thermal insulating member between the temperature control means and the biochemical analysis unit holding means, and wherein the plurality of tubular members are disposed so as to extend in the thermal insulating member.

53. A receptor-ligand associating apparatus in accordance with any one of Claims 47 to 52 which further comprises at least one reaction solution source capable of communicating with the plurality of tubular members at end portions opposite to the openings of the plurality of tubular members for accommodating a reaction solution containing a ligand or a receptor.

54. A receptor-ligand associating apparatus in accordance with Claim 53 which further comprises two or more reaction solution sources for accommodating a reaction solution containing a ligand or a receptor and the two or more reaction solution sources are constituted so as to selectively communicate with the plurality of tubular members at end portions opposite to the openings of the plurality of tubular members.

55. A receptor-ligand associating apparatus in accordance with any one of Claims 47 to 54 which further comprises at least one cleaning solution source capable of communicating with the plurality of tubular members at end portions opposite to the openings of the plurality of tubular members for accommodating a cleaning solution.

56. A receptor-ligand associating apparatus in accordance with Claim 55 which further comprises two or more reaction solution sources for accommodating a reaction solution containing a ligand or a receptor and at least one cleaning solution source for accommodating a cleaning solution and the two or more reaction solution sources and wherein the at least one cleaning solution source are constituted so as to selectively communicate with the plurality of tubular members at end portions opposite to the openings of the plurality of tubular members.
